# EUROPEAN PATENT SPECIFICATION

(11) **EP 3 326 998 B1**
(45) Date of publication and mention of the grant of the patent: **02.12.2020**
(21) Application number: 17789860.8
(22) Date of filing: 24.04.2017
(51) Int. Cl.: C09K 11/06, C07D 491/048, C07D 495/04, H01L 51/00, H01L 51/50

(54) **COMPOUND FOR ORGANIC ELECTRIC ELEMENT, ORGANIC ELECTRIC ELEMENT COMPRISING THE SAME, AND ELECTRONIC DEVICE THEREOF**
VERBINDUNG FÜR ORGANISCHES ELEKTRISCHES ELEMENT, ORGANISCHES ELEKTRISCHES ELEMENT DAMIT UND ELEKTRONISCHE VORRICHTUNG DAFÜR
COMPOSÉ POUR ÉLÉMENT ÉLECTRIQUE ORGANIQUE, ÉLÉMENT ÉLECTRIQUE ORGANIQUE LE COMPRENANT ET DISPOSITIF ÉLECTRONIQUE ASSOCIÉ

(30) Priority: 26.04.2016 KR 20160050517; 20.04.2017 KR 20170050859
(43) Date of publication of application: 30.05.2018
(73) Proprietor: Duk San Neolux Co., Ltd., Cheonan-si, Chungcheongnam-do 31027 (KR)
(72) Inventor: PARK, Jung Hwan, Hwaseong-si Gyeonggi-do 18443 (KR); KIM, Won Sam, Hwaseong-si Gyeonggi-do 18429 (KR); MUN, Soung Yun, Cheonan-si Chungcheongnam-do 31044 (KR); LEE, Yun Suk, Seongnam-si Gyeonggi-do 13630 (KR); HAHN, Seung Hoon, Cheonan-si Chungcheongnam-do 31044 (KR); CHOI, Seung Won, Yongin-si Gyeonggi-do 16909 (KR); LEE, Jung Wook, Gunsan-si Jeollabuk-do 54044 (KR); KIM, Seul Gi, Daejeon 34421 (KR)
(74) Representative: Vossius & Partner Patentanwälte Rechtsanwälte mbB
(86) International application number: PCT/KR2017/004320
(87) International publication number: WO 2017/188676

(56) References cited:
- WO-A1-2015/037965
- WO-A1-2015/142036
- KR-A- 20130 083 817
- KR-A- 20130 083 817
- KR-A- 20150 032 447
- KR-A- 20150 135 109
- KR-A- 20150 141 147
- KR-A- 20160 022 784

## Description

### BACKGROUND

### Technical Field

The present invention relates to compounds for organic electric elements, organic electric elements comprising the same, and electronic devices thereof.

### Background Art

In general, organic light emitting phenomenon refers to a phenomenon that converts electric energy into light energy by using an organic material. An organic electric element using an organic light emitting phenomenon usually has a structure including an anode, a cathode, and an organic material layer interposed there between. Here, in order to increase the efficiency and stability of the organic electronic element, the organic material layer is often composed of a multi-layered structure composed of different materials, and for example, may include a hole injection layer, a hole transport layer, an emitting layer, an electron transport layer, an electron injection layer and the like.

A material used as an organic material layer in an organic electric element may be classified into a light emitting material and a charge transport material, such as a hole injection material, a hole transport material, an electron transport material, an electron injection material and the like depending on its function.

In the case of a polycyclic compound containing a heteroatom, the difference in properties according to the material structure is so large that it is applied to various layers as a material of an organic electric element. In particular, it has characteristics of different band gaps (HOMO, LUMO), electrical characteristics, chemical properties, and physical properties depending on the number of rings, fused positions and the type and arrangement of heteroatoms, therefore application development for layers of various organic electric elements using the same has been progressed.

As a representative example thereof, in the following Patent Documents 1 to 4, the performance of the 5-membered cyclic compound in the polycyclic compound has been reported depending on the hetero type, arrangement, substituent type, fused position, and the like.
Patent Document 1: U.S. Patent. 5843607
Patent Document 2: Japanese Laid-Open Patent Publication . 1999-162650
Patent Document 3: Korean Published Patent Application 2008-0085000
Patent Document 4: US Patent Publication 2010-0187977
Patent Document 5: Korean Published Patent Application No. 2011-0018340
Patent Document 6: Korean Published Patent 2009-0057711
Patent Document 7: WO 2015/037965

Patent Documents 1 and 2 disclose an embodiment in which the indolecarbazole core in which the hetero atom in the 5-membered cyclic compound is composed only of nitrogen is used, and an aryl group substituted or unsubstituted in N of indolocarbazole is used. However, in the prior invention 1, there exists only a simple aryl group substituted or unsubstituted with an alkyl group, an amino group, an alkoxy group, or the like as a substituent. so that the effect of the substituents of the polycyclic compounds was very poor to prove, and only the use as a hole transport material is described, and the use thereof as a phosphorescent host material is not described.

Patent Documents 3 and 4 disclose a compound in which pyridine, pyrimidine, triazine or the like containing an aryl group and N is substituted for an indolecarbazole core having a hetero atom N in the same 5-membered cyclic compound as in the above Patent Documents 1 and 2, however only the use examples for phosphorescent green host materials are described, and the performance for other heterocyclic compounds substituted for indolecarbazole core is not described.

In Patent Documents 5, Nitrogen, oxygen (0), sulfur (S), carbon and the like are described as heteroatom in the 5-membered cyclic compound, however there are only examples using the same heteroatom in the performance measurement data, the performance characteristics of a 5-membered cyclic compound containing a different heteroatom could not be confirmed.

Therefore, the patent document does not disclose solutions to low charge carrier mobility and low oxidation stability of a 5-membered cyclic compound containing same heteroatom. When the 5-membered cyclic compound molecules are generally laminated, as the adjacent π- electrons increase, they have a strong electrical interaction, and this is closely related to the charge carrier mobility, particularly, the same 5-membered cyclic compound of type has an edge-to-face morphology as an order of arrangement of molecules when molecules are laminated, otherwise a different 5-membered cyclic compound with different heteroatoms has an antiparallel cofacial π-stacking structure in which the packing structure of the molecules is opposite to each other, so that the arrangement order of the molecules becomes face-to-face morphology. It is reported that the steric effect of the substituent substituted on the asymmetrically arranged hetero atom N as the cause of this laminated structure causes relatively high carrier mobility and high oxidation stability (Org. Lett.2008, 10, 1199).

In Patent Document 6, an example of using as a fluorescent host material for various polycyclic compounds having seven or more membered cyclic compounds has been reported. A host compound useful in organic electronic elements is also described in Patent Document 7.

As described above, the fused positions, the number of rings, the arrangement of heteroatoms, and characteristic change by type of the polycyclic compounds have not yet been sufficiently developed.

Particularly, in a phosphorescent organic electric element using a phosphorescent dopant material, the LUMO and HOMO levels of the host material have a great influence on the efficiency and life span of the organic electric element, this is because the charge balance control in the emitting layer, the quenching of the dopant, and the reduction in efficiency and life span due to light emission at the interface of the hole transport layer can be prevented, depending on whether electron and hole injection in the emitting layer can be efficiently controlled.

For fluorescent and phosphorescent host materials, recently we have been studying the increase of efficiency and life span of organic electric elements using TADF (thermal activated delayed fluorescent), exciplex, etc., particularly, and many studies have been carried out to identify the energy transfer method from the host material to the dopant material.

Although there are various methods for identifying the energy transfer in the emitting layer for TADF Cthermally activated delayed fluorescent) and exciplex, it can be easily confirmed by the PL lifetime (TRTP) measurement method.

The TRTP (Time Resolved Transient PL) measurement method is a method of observing a decay time over time after irradiating the host thin film with a pulsed light source, and therefore it is possible to identify the energy transfer method by observing the energy transfer and the lag time. The TRTP measurement can distinguish between fluorescence and phosphorescence, an energy transfer method in a mixed host material, an exciplex energy transfer method, and a TADF energy transfer method.

There are various factors affecting the efficiency and life span depending on the manner in which the energy is transferred from the host material to the dopant material, and the energy transfer method differs depending on the material, so that the development of stable and efficient host material for organic electric element has not yet been sufficiently developed. Therefore, development of new materials is continuously required, and especially development of a host material for an emitting layer is urgently required.

### SUMMARY

Currently, OLED devices are being developed in the direction of lowering the power consumption and increasing the color purity. In order to solve one or more of the above-mentioned problems in prior art, an aspect of the present invention is to provide a compound into which a Sub having excellent electron characteristics is introduced, and capable of lowering driving voltage, increasing luminous efficiency, improving color purity and lifetime of device, an organic electric element comprising the same, and an electronic device thereof.

In accordance with an aspect of the present invention, the compound represented by the following formula 1, as defined in claim 1, is provided.

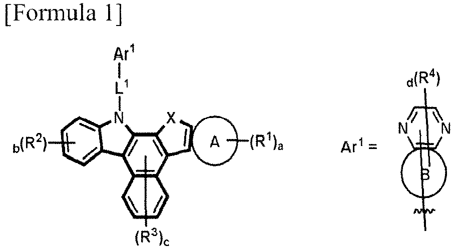

In another aspect of the present invention, organic electric elements comprising the compound represented by the formula 1 above and electronic devices including the organic electric element are provided.

According to the embodiments of the present invention, luminous efficiency, heat-resistance, and lifetime of the organic electric elements can be improved and a driving voltage of the organic electric elements can be lowered because the electron transfer ability and the thermal stability are improved, and electron injection from the ETL is facilitated, resulting in a LUMO energy level that is easy to balance charge in the a light emitting layer by using a specific compound as a material of the organic electric device, wherein the specific compound has an aromatic ring additionally fused to the existing core and a sub-substituent having a strong ET characteristic.

### BRIEF DESCRIPTION OF THE DRAWINGS

Fig.1 illustrates an example of an organic light emitting diode according to an embodiment of the present invention.

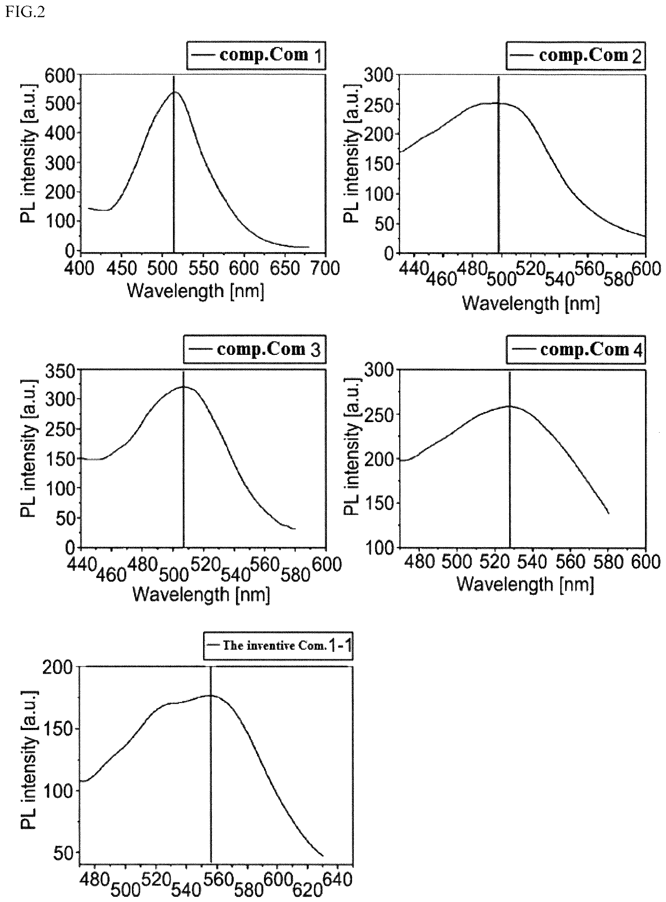

Fig.2 shows the PL results of comparative compounds 1 to 4 and compound 1-1 of the present invention.
Fig.3 shows the 1H NMR results of compound 1-1 of the present invention.
Fig.4 shows the 13C NMR results of compound 1-1 of the present invention.

### DETAILED DESCRIPTION

Hereinafter, some embodiments of the present invention will be described in detail. In the following description of the present invention, a detailed description of known functions and configurations incorporated herein will be omitted when it may make the subject matter of the present invention rather unclear.

In addition, terms, such as first, second, A, B, (a), (b) or the like may be used herein when describing components of the present invention. Each of these terminologies is not used for defining an essence, order or sequence of a corresponding component but used merely to distinguish the corresponding component from other component(s). It should be noted that if it is described in the specification that one component is "connected," "coupled" or "joined" to another component, a third component may be "connected," "coupled," and "joined" between the first and second components, although the first component may be directly connected, coupled or joined to the second component.

As used in the specification and the accompanying claims, unless otherwise stated, the following is the meaning of the term as follows.

Unless otherwise stated, the term "halo" or "halogen" as used herein includes fluorine(F), chlorine(Cl), bromine(Br), or iodine(I).

Unless otherwise stated, the term "alkyl" or "alkyl group" as used herein has a single bond of 1 to 60 carbon atoms, and means aliphatic functional radicals including a linear alkyl group, a branched chain alkyl group, a cycloalkyl group (alicyclic), or an alkyl group substituted with a cycloalkyl.

Unless otherwise stated, the term "halo alkyl" or "halogen alkyl" as used herein includes an alkyl group substituted with a halogen.

Unless otherwise stated, the term "heteroalkyl", as used herein, means alkyl substituted one or more of carbon atoms consisting of an alkyl with hetero atom.

Unless otherwise stated, the term "alkenyl" or "alkynyl" as used herein has, but not limited to, double or triple bonds of 2 to 60 carbon atoms, and includes a linear alkyl group, or a branched chain alkyl group.

Unless otherwise stated, the term "cycloalkyl" as used herein means, but not limited to, alkyl forming a ring having 3 to 60 carbon atoms.

The term "alkoxyl group", "alkoxy group" or "alkyloxy group" as used herein means an oxygen radical attached to an alkyl group, but not limited to, and has 1 to 60 carbon atoms.

Unless otherwise stated, the term "alkenoxyl group", "alkenoxy group", "alkenyloxy group" or "alkenyloxy group", as used herein, means anoxygen radical attached to an alkenyl group, but is not limited thereto, and has 2 to 60 carbon atoms.

Unless otherwise stated, the term "aryloxyl group" or "aryloxy group", as used herein, means an oxygen radical attached to an aryl group, but is not limited thereto, and has 6 to 60 carbon atoms.

Unless otherwise stated, the term "aryl group" or "arylene group", as used herein, has 6 to 60 carbon atoms, but is not limited thereto. Herein, the aryl group or arylene group means a monocyclic and polycyclic aromatic group, and may also be formed in conjunction with an adjacent group. Examples of "aryl group" may include a phenyl group, a biphenyl group, a fluorene group, or a spirofluorene group.

The prefix "aryl" or "ar" means a radical substituted with an arylgroup. For example, an arylalkyl may be an alkyl substituted with an aryl, and an arylalkenyl may be an alkenyl substituted with aryl, and a radical substituted with an aryl has a number of carbon atoms as defined herein.

Also, when prefixes are named subsequently, it means that substituents are listed in the order described first. For example, an arylalkoxy means an alkoxy substituted with an aryl, an alkoxylcarbonyl means a carbonyl substituted with an alkoxyl, and an arylcarbonylalkenyl also means an alkenyl substituted with an arylcarbonyl, wherein the arylcarbonyl may be a carbonyl substituted with an aryl.

Unless otherwise stated, the term "heteroalkyl", as used herein, means alkyl containing one or more of heteroatoms. Unless otherwise stated, the term "heteroaryl group" or "heteroarylene group", as used herein, means a C₂ to C₆₀ aryl containing one or more of heteroatoms or arylene group, but is not limited thereto, and includes at least one of monocyclic and polycyclic rings, and may also be formed in conjunction with an adjacent group.

Unless otherwise stated, the term "heterocyclic group", as used herein, contains one or more heteroatoms, but is not limited thereto, has 2 to 60 carbon atoms, includes any one of monocyclic and polycyclic rings, and may include heteroaliphatic ring and/or heteroaromatic ring. Also, the heterocyclic group may also be formed in conjunction with an adjacent group.

Unless otherwise stated, the term "heteroatom", as used herein, represents at least one of N, O, S, P, or Si.

Also, the term "heterocyclic group" may include SO₂ instead of carbon consisting of cycle. For example, "heterocyclic group" includes compound below.

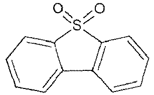

Unless otherwise stated, the term "aliphatic", as used herein, means an aliphatic hydrocarbon having 1 to 60 carbon atoms, and the term "aliphatic ring", as used herein, means an aliphatic hydrocarbon ring having 3 to 60 carbon atoms.

Unless otherwise stated, the term "ring", as used herein, means an aliphatic ring having 3 to 60 carbon atoms, or an aromatic ring having 6 to 60 carbon atoms, or a hetero ring having 2 to 60 carbon atoms, or a fused ring formed by the combination of them, and includes a saturated or unsaturated ring.

Other hetero compounds or hetero radicals other than the above-mentioned hetero compounds include, but are not limited thereto, one or more heteroatoms.

Unless otherwise stated, the term "carbonyl", as used herein, is represented by -COR', wherein R' may be hydrogen, an alkyl having 1 to 20 carbon atoms, an aryl having 6 to 30 carbon atoms, a cycloalkyl having 3 to 30 carbon atoms, an alkenyl having 2 to 20 carbon atoms, an alkynyl having 2 to 20 carbon atoms, or the combination of these.

Unless otherwise stated, the term "ether", as used herein, is represented by -R-O-R', wherein R or R' may be independently hydrogen, an alkyl having 1 to 20 carbon atoms, an aryl having 6 to 30 carbon atoms, a cycloalkyl having 3 to 30 carbon atoms, an alkenyl having 2 to 20 carbon atoms, an alkynyl having 2 to 20 carbon atoms, or the combination of these.

Unless otherwise stated, the term "substituted or unsubstituted" as used herein means that substitution is carried out by at least one substituent selected from the group consisting of, but not limited to, deuterium, halogen, an amino group, a nitrile group, a nitro group, a C₁-C₂₀ alkyl group, a C₁-C₂₀ alkoxy group, a C₁-C₂₀ alkylamine group, a C₁-C₂₀ alkylthiophene group, a C₆-C₂₀ arylthiophene group, a C₂-C₂₀ alkenyl group, a C₂-C₂₀ alkynyl group, a C₃-C₂₀ cycloalkyl group, a C₆-C₂₀ aryl group, a C₆-C₂₀ aryl group substituted by deuterium, a C₈-C₂₀ arylalkenyl group, a silane group, a boron group, a germanium group, and a C₂-C₂₀ heterocyclic group.

Otherwise specified, the formulas used in the present invention are as defined in the index definition of the substituent of the following formula.

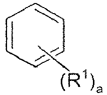

Wherein, when a is an integer of zero, the substituent R¹ is absent, that is, hydrogen atoms are bonded to all the carbon constituting the benzene ring, and chemical formulas or compounds may be written without explicitly describing the hydrogen. In addition, one substituent R¹ is bonded to any carbon of the carbons forming the benzene ring when "a" is an integer of 1, substituents R¹ are bonded, for example, as followings when "a" is an integer of 2 or 3, substituents R¹ are bonded to the carbon of the benzene ring in a similar manner when "a" is an integer of 4 to 6, and R's may be the same or different from each other when "a" is an integer of 2 or more.

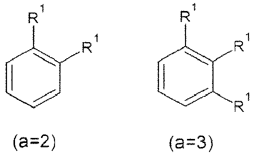

Unless otherwise expressly stated, the terms "ortho", "meta", and "para" used in the present invention refer to the substitution positions of all substituents, and the ortho position indicates the position of the substituent immediately adjacent to the compound, for example, when benzene is used, it means 1, 2 position, and the meta position is the next substitution position of the neighbor substitution position, when benzene as an example stands for 1, 3 position, and the para position is the next substitution position of the meta position, which means 1, 4 position when benzene is taken as an example. A more detailed example of the substitution position is as follows, and it can be confirmed that the ortho-, and meta- position are substituted by non-linear type and para positions are substituted by linear type.

### [Example of ortho-position]

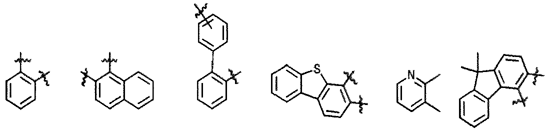

### [Example of meta-position]

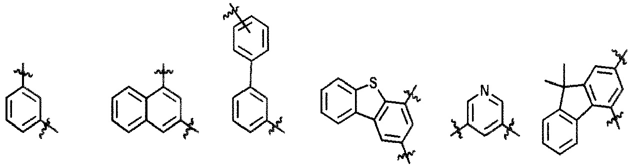

### [Example of para-position]

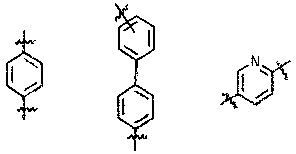

Hereinafter, a compound according to an aspect of the present invention and an organic electric element comprising the same will be described.

The present invention provides a compound represented by the following Formula 1. 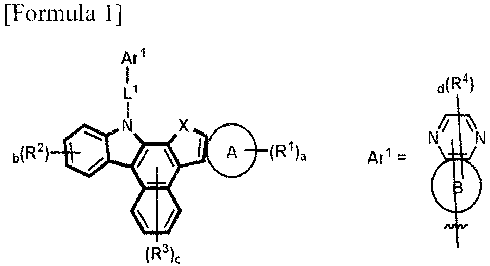 wherein,
1) A ring is C₁₀ aryl group,
2) B ring is selected from the group consisting of the following formulas B-1 to B-16: 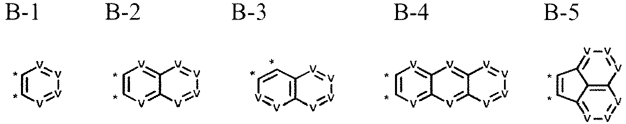 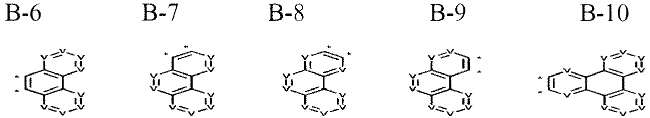 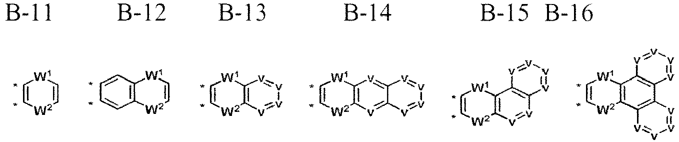 in formulas B-1 to B-16, "*" indicates the position to be condensed with pyrazine comprising two Ns,
3) W¹ and W² are each independently a single bond, S or O,
4) V is N or C,
5) X is O or S,
6) a is an integer of 0 to 6, b and c are each an integer of 0 to 4, d is an integer of 0 to 11, and
7) R¹, R² and R³ are the same or different from each other, and are each independently selected from the group consisting of hydrogen, deuterium, halogen, a cyano group, a nitro group, a C₆-C₆₀ aryl group, a fluorenyl group, a C₂-C₆₀ heterocyclic group containing at least one heteroatom selected from the group consisting of O, N, S, Si, and P, a fused ring group of a C₃-C₆₀ aliphatic ring and a C₆-C₆₀ aromatic ring, a C₁-C₅₀ alkyl group, a C₂-C₂₀ alkenyl group, a C₂-C₂₀ alkynyl group, a C₁-C₃₀ alkoxyl group, a C₆-C₃₀ aryloxyl group and -L'-N(R^{a})(R^{b}).
   Where R¹ to R³ are an aryl group, preferably R¹ to R³ may be each a C₆-C₃₀ aryl group, more preferably a C₆-C₁₈ aryl group. Where R¹ to R³ are a heterocyclic group, preferably R¹ to R³ may be each a C₂-C₄₀ heterocyclic group, more preferably a C₂-C₃₀ heterocyclic group, more preferably a C₂-C₂₀ heterocyclic group.
   In case a, b and c are 2 or more, R¹, R² and R³ are each in plural and are the same or different, and a plurity of R¹, a plurity of R², or a plurity of R³ may be bonded to each other to form a ring. R⁴ is represented by any one of the formulas R-1 to R-10 as defined below.
8) L' is selected from the group consisting of a single bond, a C₆-C₆₀ arylene group, a fluorenylene group, a fused ring group of a C₃-C₆₀ aliphatic ring and a C₆-C₆₀ aromatic ring, and a C₂-C₆₀ heterocyclic group, R^{a} and R^{b} are each independently selected from the group consisting of a C₆-C₆₀ aryl group, a fluorenyl group, a fused ring group of a C₃-C₆₀ aliphatic ring and a C₆-C₆₀ aromatic ring, and a C₂-C₆₀ heterocyclic group containing at least one heteroatom selected from the group consisting of O, N, S, Si, and P.
9) L¹ is each independently selected from the group consisting of a single bond, a C₆-C₆₀ arylene group, a fluorenylene group, a fused ring group of a C₃-C₆₀ aliphatic ring and a C₆-C₆₀ aromatic ring, and a C₂-C₆₀ heterocyclic group.

The aryl group, fluorenyl group, arylene group, heterocyclic group, fused ring group, alkyl group, alkenyl group, alkoxyl group, and aryloxy group may be each optionally further substituted with one or more substituents selected from the group consisting of deuterium, halogen, a silane group substituted or unsubstituted with a C₁-C₂₀ alkyl group or a C₆-C₂₀ aryl group, a siloxane group, a boron group, a germanium group, a cyano group, a nitro group,-L'-N(R^{a})(R^{b}), a C₁-C₂₀ alkylthio group, a C₁-C₂₀ alkoxyl group, a C₁-C₂₀ alkyl group, a C₂-C₂₀ alkenyl group, a C₂-C₂₀ alkynyl group, a C₆-C₂₀ aryl group, a C₆-C₂₀ aryl group substituted with deuterium, a fluorenyl group, a C₂-C₂₀ heterocyclic grou, a C₃-C₂₀ cycloalkyl group, a C₇-C₂₀ arylalkyl group, and a C₈-C₂₀ arylalkenyl group, and these substituents may be linked each other to form a ring, wherein 'ring' comprises a C₃-C₆₀ aliphatic ring, a C₆-C₆₀ aromatic ring, a C₂-C₆₀ heterocyclic group or a fused ring formed by the combination of them, and comprises a saturated or unsaturated ring.

Formula 1 above may be represented by any one of Formulas 2 to 4 below:

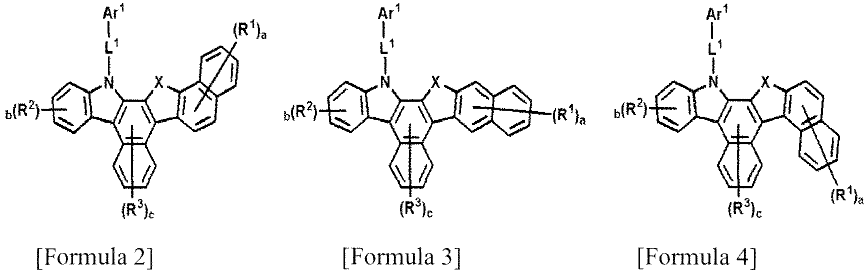

In Formulas 2 to 4, X, L¹, Ar¹, R¹, R², R³, a, b and c are the same as defined above. Further, Formula 1 above may be represented by any one of Formulas 5 to 7 below:

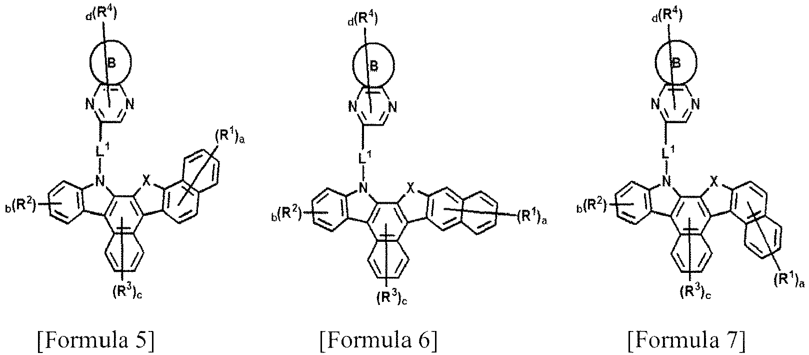

In Formulas 5 to 7, X, L¹, Ar¹, R¹, R², R³, R⁴, a, b, c, d, B ring are the same as defined above.

One embodiment of the present invention provides the compound of which the chemical structure Ar¹ of the formula 1 comprising the pyrazine is represented by any one of the following Formulas C-1 to C-22.

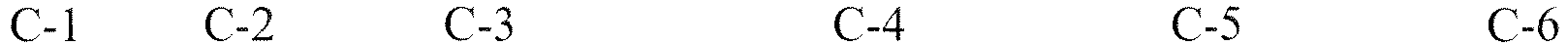

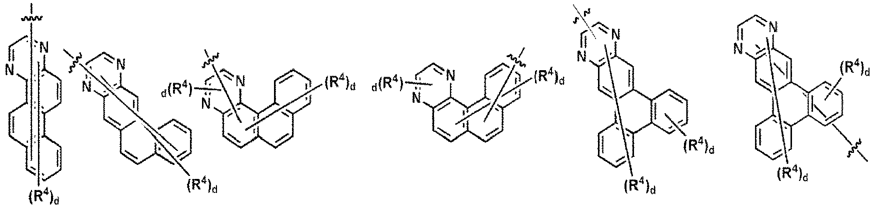

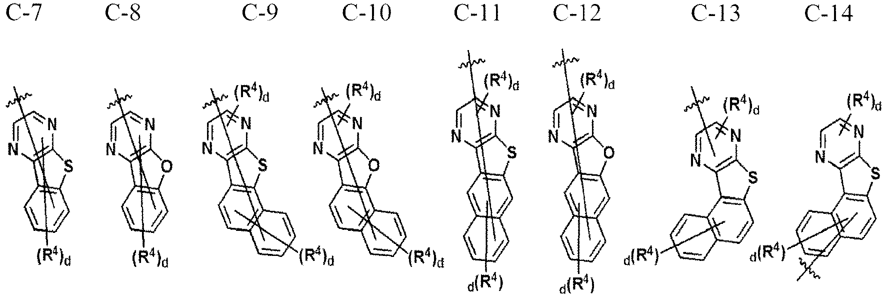

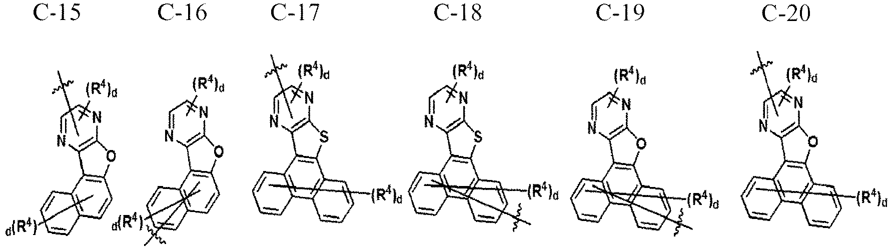

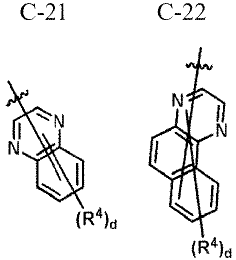

In Formulas C-1 to C-22, R⁴ is the same as defined above, and d is an integer of 0 to 11.

The present invention comprises the compound wherein R⁴ in the above formula 1 is represented by any one of the following formulas R-1 to R-10.

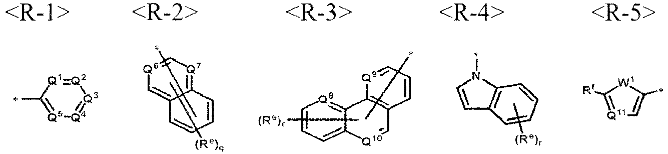

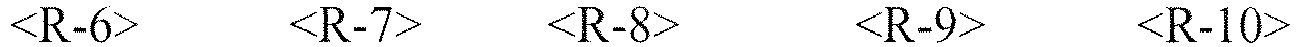

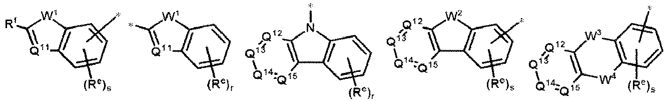

In Formulas R-1 to R-10,
1) Q¹ to Q¹⁵ are each independently CR^{g} or N,
2) W¹ is S, O or NR^{h},
3) W² to W⁴ are each independently S, O, NR^{h} or CRⁱR^{j},
4) R^{e} is selected from the group consisting of hydrogen, deuterium, halogen, a silane group substituted or unsubstituted with a C₁-C₂₀ alkyl group or a C₆-C₂₀ aryl group, a siloxane group, a boron group, a germanium group, a cyano group, a nitro group, a C₁-C₂₀ alkylthio group, a C₁-C₂₀ alkoxyl group, a C₁-C₂₀ alkyl group, a C₂-C₂₀ alkenyl group, a C₂-C₂₀ alkynyl group, a C₆-C₂₀ aryl group, a C₆-C₂₀ aryl group substituted with deuterium, a fluorenyl group, a C₂-C₂₀ heterocyclic group containing at least one heteroatom selected from the group consisting of O, N, S, Si, and P, a C₃-C₂₀ cycloalkyl group, a C₇-C₂₀ arylalkyl group, and a C₈-C₂₀ arylalkenyl group, and when these substituents are adjacent, they may be linked each other to form a ring,
5) R^{f} and R^{g} are each independently selected from the group consisting of hydrogen, deuterium, a C₆-C₂₀ aryl group, a fluorenyl group, a fused ring group of a C₃-C₂₀ aliphatic ring and a C₆-C₂₀ aromatic ring, a C₂-C₂₀ heterocyclic group containing at least one heteroatom selected from O, N, S, Si, and P, a C₁-C₂₀ alkyl group, a C₂-C₂₀ alkenyl group, a C₂-C₂₀ alkynyl group, and a C₁-C₃₀ alkoxyl group,
6) R^{h}, Rⁱ and R^{j} are each independently selected from the group consisting of a C₆-C₂₀ aryl group, a C₂-C₂₀ heterocyclic group containing at least one heteroatom selected from O, N, S, Si, and P, a C₁-C₂₀ alkyl group, a C₂-C₂₀ alkenyl group, a C₁-C₂₀ alkoxyl group and a fluorenyl group, Rⁱ and R^{j} may be linked each other to form a spiro compound together with C to which they are bonded,
7) q is each independently an integer of 0 to 5,
8) r is each independently an integer of 0 to 4,
9) s is each independently an integer of 0 to 3,
when q, r and s are each 2 or more, R^{e} is each the same or different,
"*" indicates the position to be bonded,
When R^{e} to R^{j} are an aryl group, preferably R^{e} to R^{j} may be each a C₆-C₃₀ aryl group, more preferably a C₆-C₁₈ aryl group. When R^{e} to R^{j} are a heterocyclic group, preferably R^{e} to R^{j} may be each a C₂-C₄₀ heterocyclic group, more preferably a C₂-C₃₀ heterocyclic group, more preferably a C₂-C₂₀ heterocyclic group.

Specifically, the compound represented by Formula 1 may be any one of the following compounds.

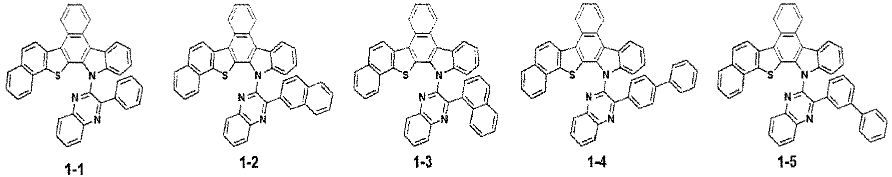

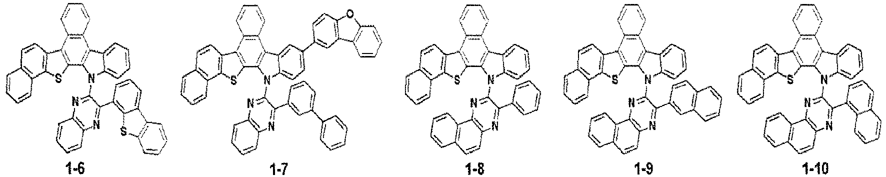

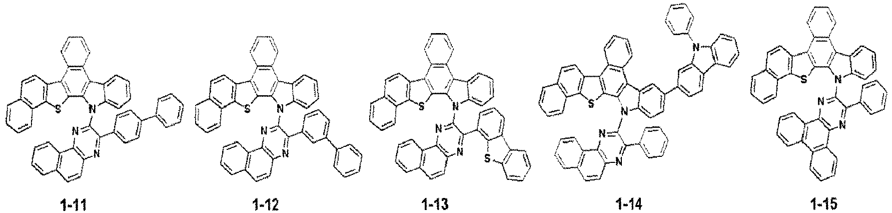

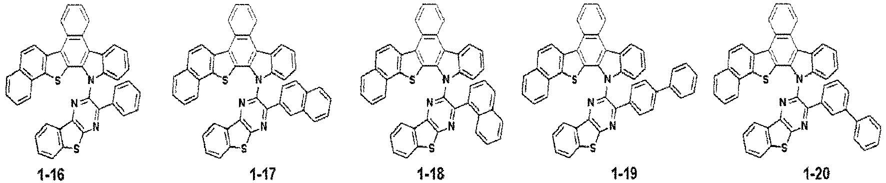

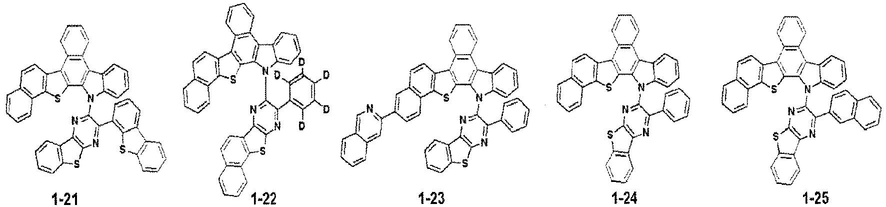

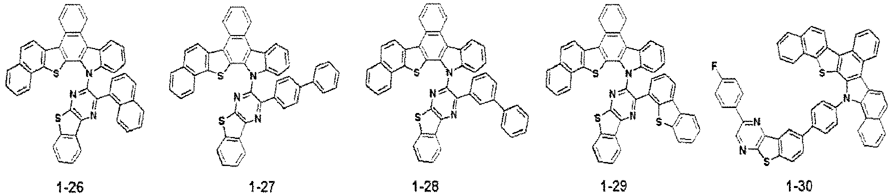

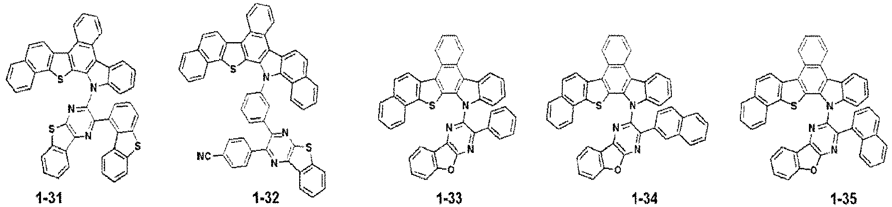

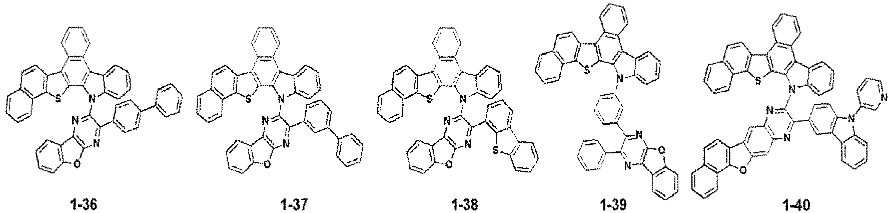

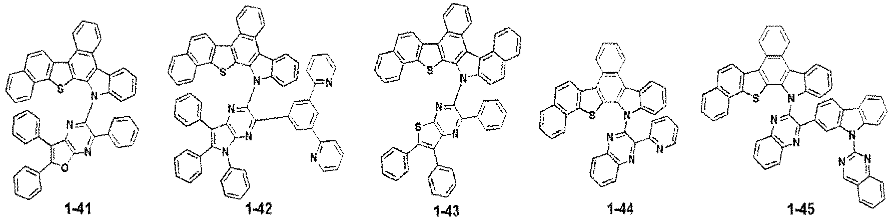

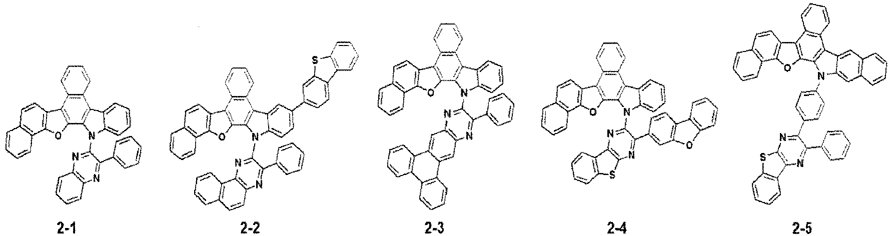

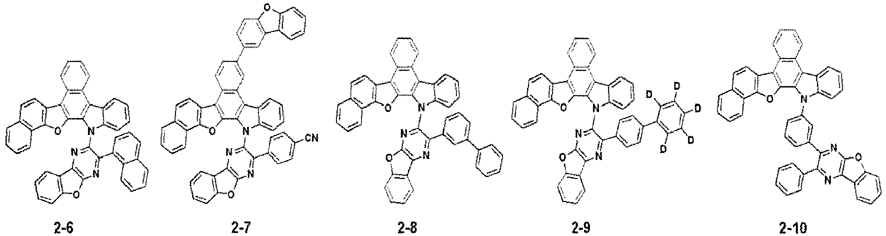

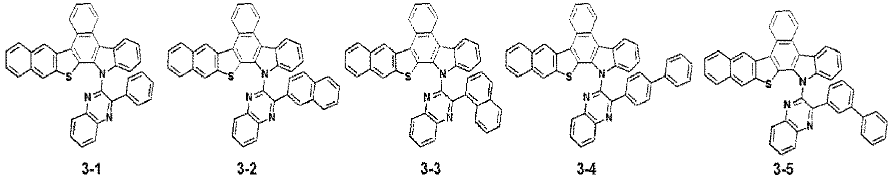

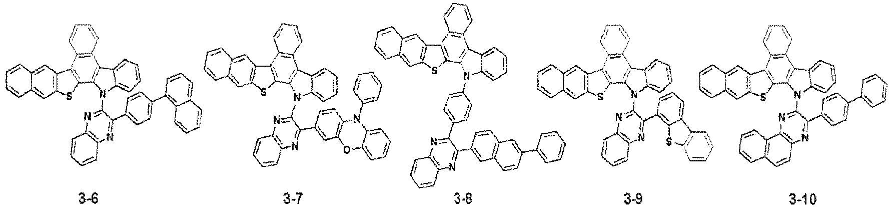

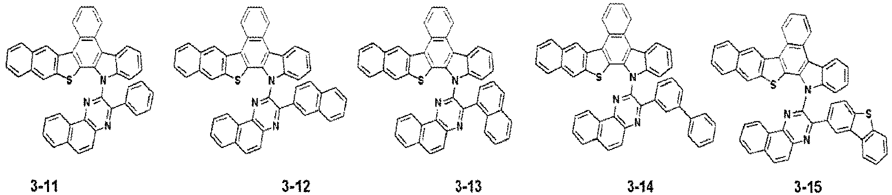

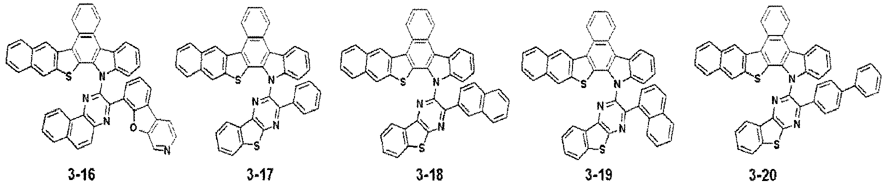

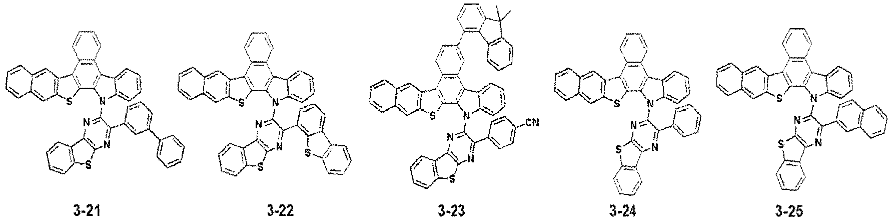

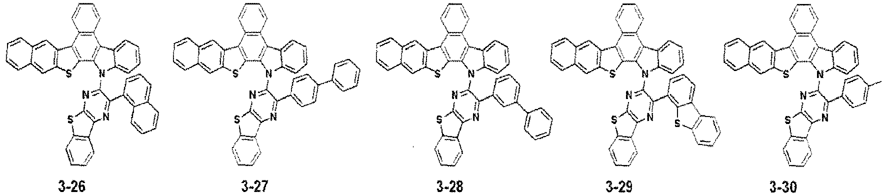

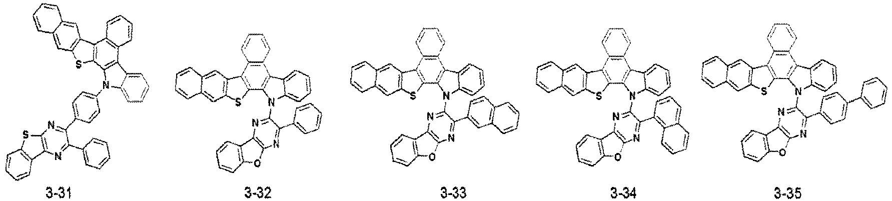

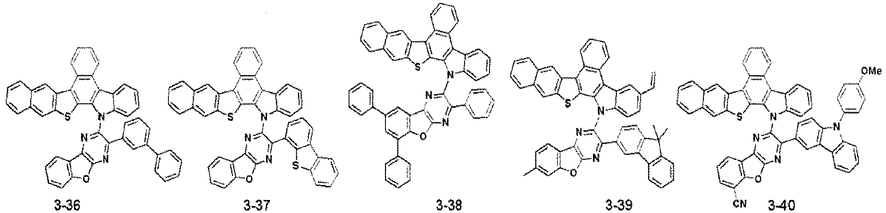

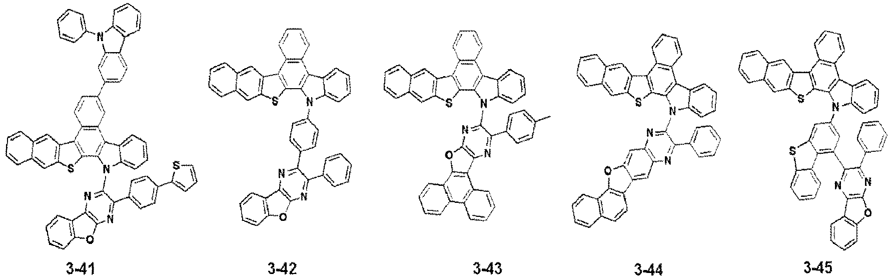

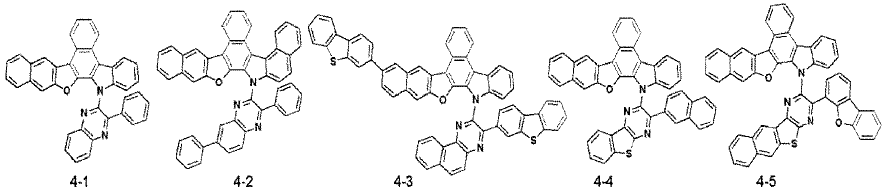

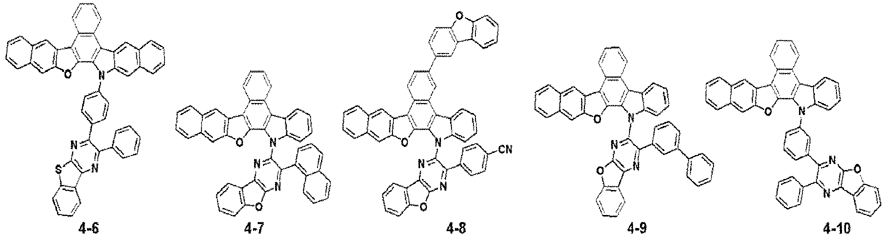

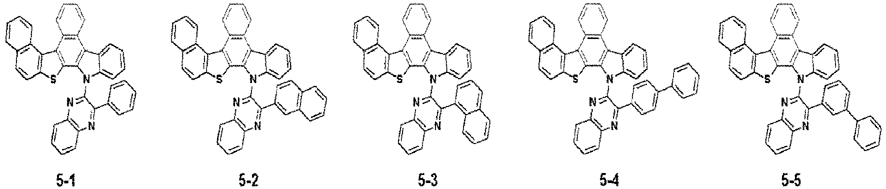

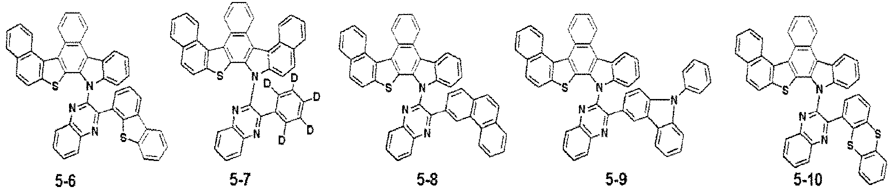

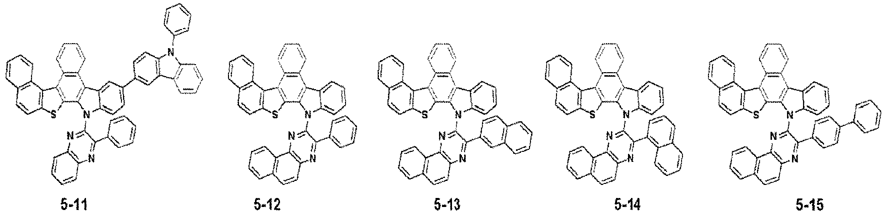

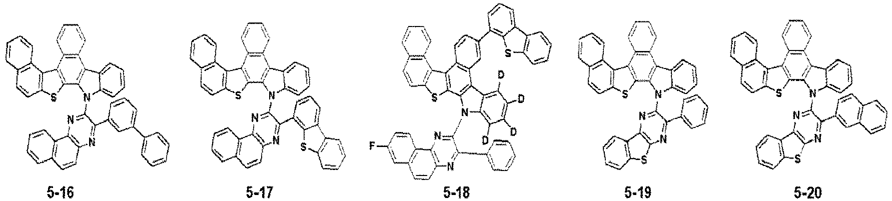

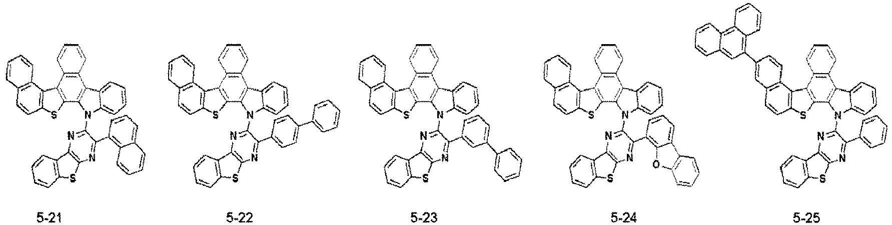

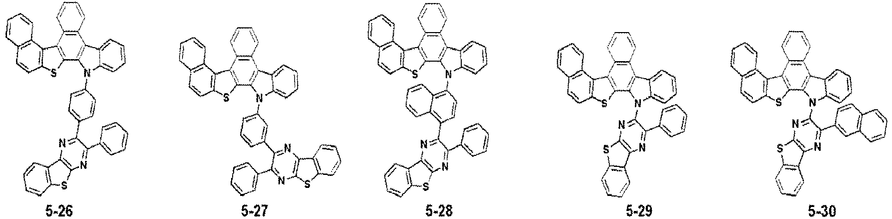

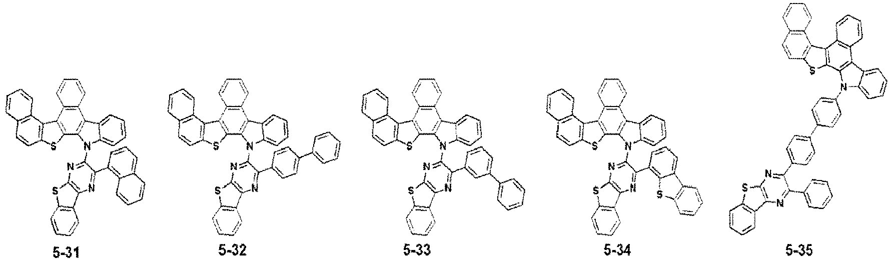

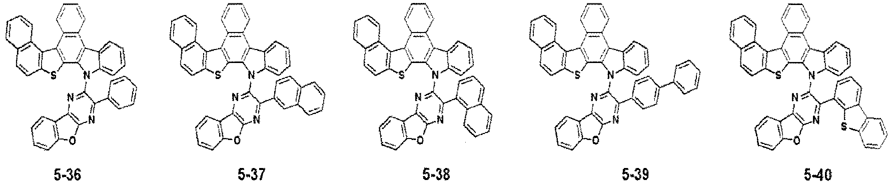

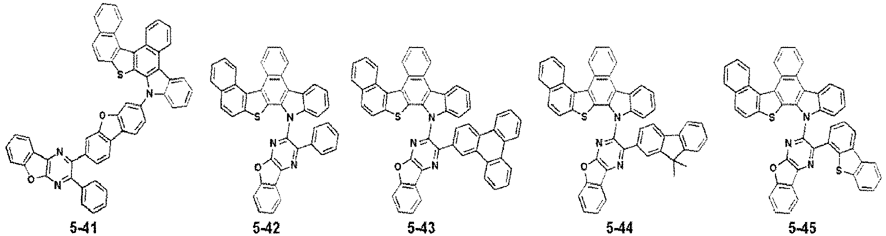

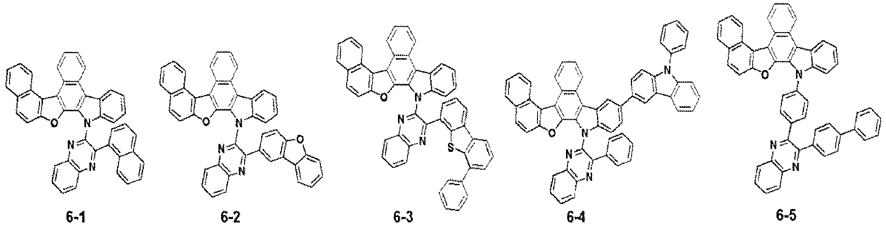

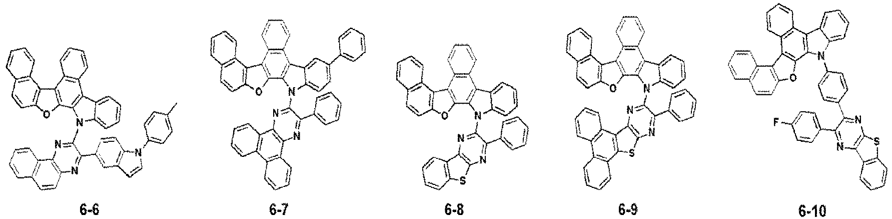

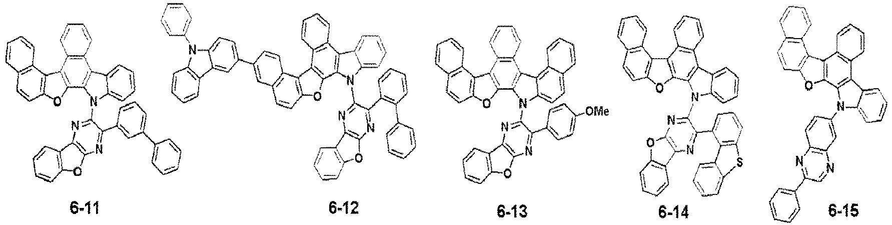

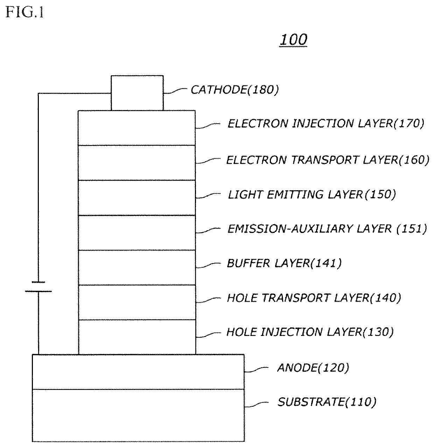

Referring to Fig.1, an organic electric element 100 according to an embodiment of the present invention includes a first electrode 120 formed on a substrate 110, a second electrode 180, and an organic material layer between the first electrode 110 and the second electrode 180, wherein the organic material layer contains compound represented by Formula 1. Here, the first electrode 120 may be an anode (positive electrode), and the second electrode 180 may be a cathode (negative electrode). In the case of an inverted organic electric element, the first electrode may be a cathode, and the second electrode may be an anode.

The organic material layer may include a hole injection layer 130, a hole transport layer 140, a light emitting layer 150, an electron transport layer 160, and an electron injection layer 170 formed in sequence on the first electrode 120. Here, the remaing layers except the light emitting layer 150 may not be formed. The organic material layer may further include a hole blocking layer, an electron blocking layer, an emission-auxiliary layer 151, an electron transport auxiliary layer, a buffer layer 141, etc., and the electron transport layer 160 and the like may serve as a hole blocking layer.

Although not shown, the organic electric element according to an embodiment of the present invention may further include at least one protective layer formed on at least one of the sides the first and second electrodes, which is a side opposite to the organic material layer.

Otherwise, even if the same core is used, the band gap, the electrical characteristics, the interface characteristics, and the like may vary depending on which substituent is bonded at which position, therefore the choice of core and the combination of sub-substituents associated therewith is also very important, and in particular, when the optimal combination of energy levels and T1 values and unique properties of materials (mobility, interfacial characteristics, etc.) of each organic material layer is achieved, a long life span and high efficiency can be achieved at the same time.

The organic electroluminescent device according to an embodiment of the present invention may be manufactured using a PVD (physical vapor deposition) method. For example, a metal or a metal oxide having conductivity or an alloy thereof is deposited on a substrate to form a cathode, and the organic material layer including the hole injection layer (130), the hole transport layer (140), the emitting layer (150), the electron transport layer (160), and the electron injection layer (170) is formed thereon, and then depositing a material usable as a cathode thereon can manufacture an organic electroluminescent device according to an embodiment of the present invention.

In addition, an emission auxiliary layer (151) may be further formed between the hole transport layer(140) and the emitting layer(150), and an electron transport auxiliary layer may be further formed between the emitting layer (150) and the electron transport layer (160).

The present invention may further include a light efficiency enhancing layer formed on at least one of the opposite side to the organic material layer among one side of the first electrode, or one of the opposite side to the organic material layer among one side of the second electrode.

Also, the present invention provides the organic electric element wherein the organic material layer is formed by one of a spin coating process, a nozzle printing process, an inkjet printing process, a slot coating process, a dip coating process or a roll-to-roll process, and since the organic material layer according to the present invention can be formed by various methods, the scope of the present invention is not limited by the method of forming the organic material layer.

The compound represented by Formula 1 may be comprised in the organic material layer, and in at least one layer of a hole injection layer, a hole transport layer, an emission auxiliary layer and the emitting layer, and the compound may be included as a single compound or a mixture of two or more different kinds.

As another specific example, the present invention provides an organicelectric element wherein the emitting layer of the organic material layer is a phosphorescent light emitting layer. For example, compound of the present invention may be used as material of a light emitting layer 150, a hole transport layer 140 and/or an emission-auxiliary layer 151, preferably, as host material of a light emitting layer 150, more preferably, as phosphorescent red host material.

The organic electric element according to an embodiment of the present invention may be of a top emission type, a bottom emission type, or a dual emission type depending on the material used.

WOLED (White Organic Light Emitting Device) has advantages of high resolution realization, an excellent processability, and being produced by using conventional color filter technologies for LCDs. Various structures for WOLED which mainly used as back light units have been suggested and patented. WOLED may employ various arrangement methods, representatively, a parallel side-by-side arrangement method of R(Red), G(Green), B(Blue) light-emitting units, a vertical stack arrangement method of RGB light-emitting units, and a CCM (color conversion material) method which uses electroluminescence from a blue (B) organic light emitting layer and photo-luminescence of inorganic phosphors using lighting of electroluminescence from a blue (B) organic light emitting layer, and the present invention may be applied to such WOLED.

Also, the present invention provides an electronic device including a display device which includes the above described organic electric element, and a control unit for controlling the display device.

Another embodiment of the present invention provides an electronic device including the organic electric element, wherein the organic electric element may be any one of an organic light emitting diode, an organic solar cell, an organic photo conductor, an organic transistor, and an element for monochromatic or white illumination. Here, the electronic device may be a wired/wireless communication terminal which is currently used or will be used in the future, and covers all kinds of electronic devices including a mobile communication terminal such as a cellular phone, a personal digital assistant (PDA), an electronic dictionary, a point-to-multipoint (PMP), a remote controller, a navigation unit, a game player, various kinds of TVs, and various kinds of computers.

Hereinafter, Synthesis Examples of the compound represented by Formula 1 and 2 according to the present invention and preparation examples of the organic electric element will be described in detail by way of example , but are not limited to the following examples of the invent ion.

### SYNTHESIS EXAMPLE

The compound (final products) according to the present invention can be synthesized by reacting according to the following method, but are not limited thereto. 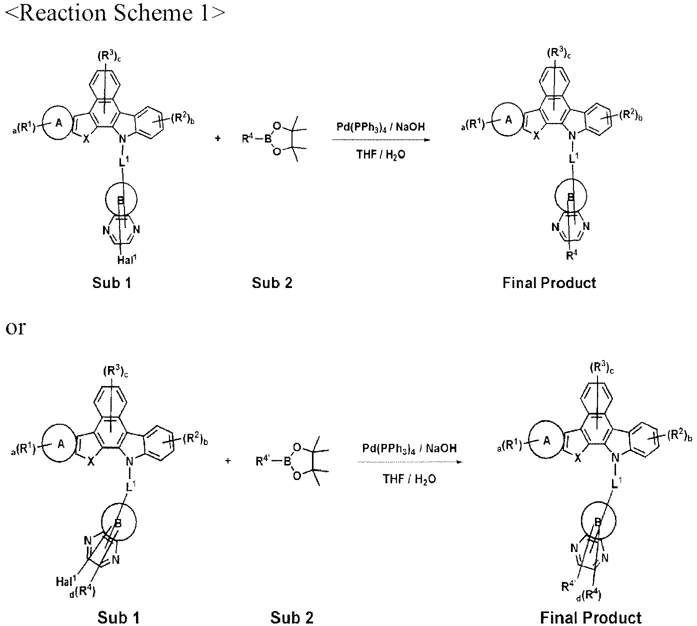
Hal¹= Cl, Br
R^{4'} can be the same as the definition of R⁴, and means a substituent which is the same as R⁴ or different from R⁴.

### I. Synthesis of Sub 1

Sub 1 of the Reaction Scheme 1 can be synthesized according to, but not limited to, the reaction route of the following Reaction Scheme 2 and Reaction Scheme 3. 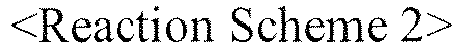 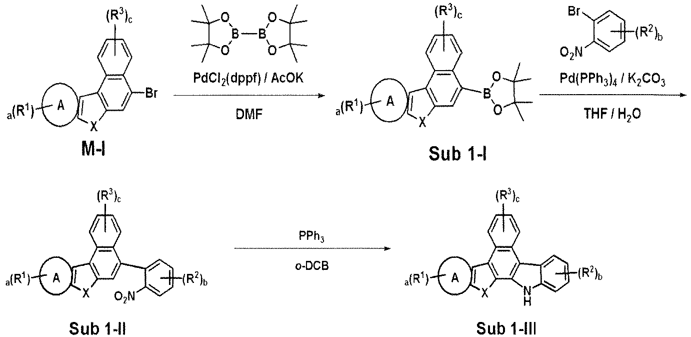 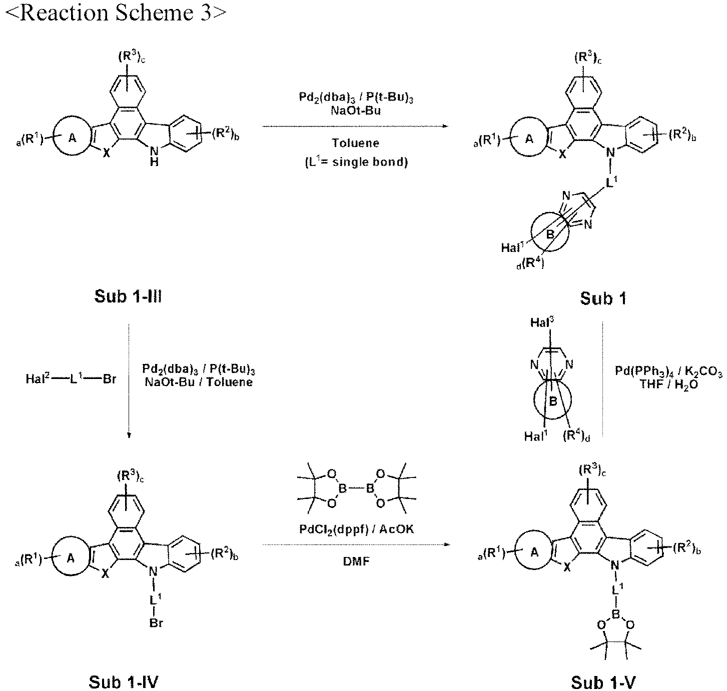
Hal²= I, Br
Hal¹, Hal³= Cl, Br

In the above reaction scheme 1, the reactants of 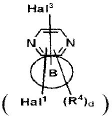 were synthesized by referring to four literatures as follows.
1) The synthesis method disclosed in Korean Patent No. 10-1488560 (Registered on February 3, 2013) filed by Doosan Corporation was used. (See Reaction Scheme A) 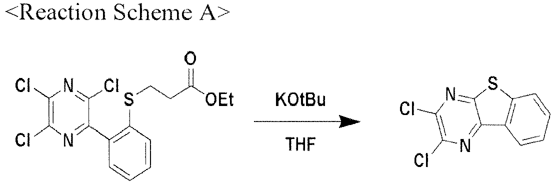
2) The synthesis method disclosed in international published patent PCT/EP2015/068240 (First priority Filing date: August 8, 2014) filed by BASF was used. (See Reaction Scheme B) 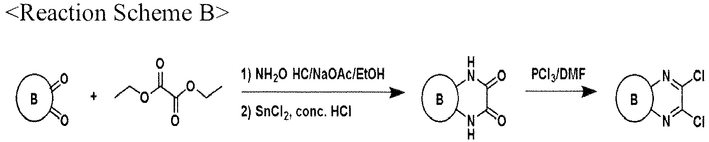
3) The synthesis method disclosed in Chinese Publishe Patent No. 2016-10316704 filed by Soochow University (filed on May 13, 2016) was used. <See Reaction Scheme C> 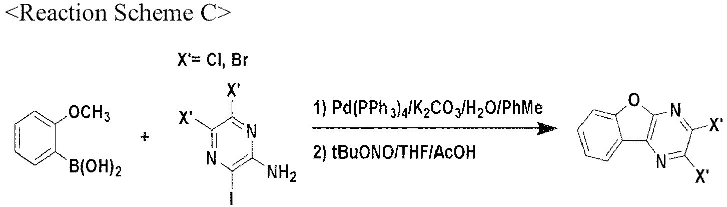
4) The synthesis method disclosed in Korean Laid-Open Patent Publication No. 2015-0130953 (First priority Filing date: December 5, 2014) filed by LG Display Co., Ltd. was used. <See Synthesis of compound 6>

Synthesis Examples of compounds comprised in Sub 1 are as follows.

### Synthesis example of M-I-1

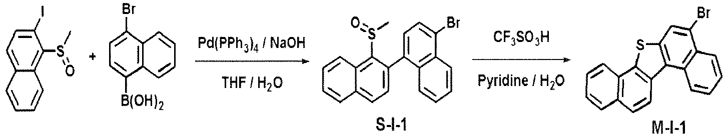

### 1) Synthesis of S-I-1

(4-bromonaphthalen-1-yl)boronic acid (28g, 111.6mmol) was dissolved in THF (491ml) in a round bottom flask, then 2-iodo-1-(methylsulfinyl)naphthalene (35.28g, 111.6mmol), Pd(PPh₃)₄ (1.93g, 1.67mmol), NaOH (6.70g, 167.40mmol) and water (246ml) were added and stirred at 80°C. When the reaction was completed, the reaction product was extracted with CH₂Cl₂ and water, and then, the organic layer was dried with MgSO₄ and concentrated. Then, the concentrate was passed through silica gel column and recrystallized to obtain 30 g (yield: 68%) of the product.

### 2) Synthesis of M-I-1

S-I-1 (30g, 75.89mmol) obtained in the above synthesis was added into a round bottom flask together with triflic acid (100.7ml, 1138.35mmol) and stirred at room temperature for 24 hours. Then, a pyridine aqueous solution (1329ml, pyridine:H₂O = 1:5) was slowly added dropwise, refluxed and stirred for 30 minutes. When the reaction was completed, the reaction product was extracted with CH₂Cl₂ and water, and then, the organic layer was dried with MgSO₄ and concentrated. Then, the concentrate was passed through silica gel column and recrystallized to obtain 21.8g (yield: 79%) of the product.

### Synthesis of M-I-2

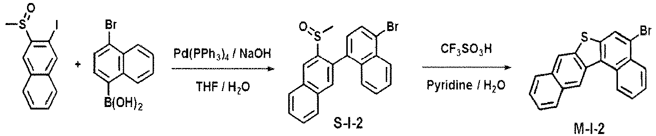

### 1) Synthesis of S-I-2

30.16g (yield: 66%) of the product was obtained by using (4-bromonaphthalen-1-yl)boronic acid (29g, 115.59mmol), THF (509ml), 2-iodo-3-(methylsulfinyl)naphthalene (36.54g, 115.59mmol), Pd(PPh₃)₄ (2g, 1.73mmol), NaOH (6.94g, 173.38mmol), and water (254ml) in the same manner as described above for the synthesis of S-I-1.

### 2) Synthesis of M-I-2

20.1g (yield: 73%) of the product was obtained by using S-I-2 (30g, 75.89mmol) obtained in the above synthesis, triflic acid (100.7ml, 1138.35mmol), pyridine aqueous solution (1329.6ml, pyridine : H₂O = 1 : 5) in the same manner as described above for the synthesis of M-I-1.

### Synthesis of M-I-3

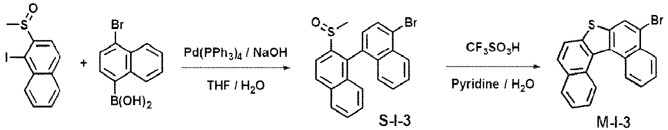

### 1) Synthesis of S-I-3

30.77g (yield: 63%) of the product was obtained by using (4-bromonaphthalen-1-yl)boronic acid (31g, 123.56mmol), THF (543ml), 1-iodo-2-(methylsulfinyl)naphthalene (31g, 123.56mmol), Pd(PPh₃)₄ (2.14g, 1.85mmol), NaOH (7.41g, 185.34mmol), and water (272ml) in the same manner as described above for the synthesis of S-I-1.

### 2) Synthesis of M-I-3

20.7g (yield: 75%) of the product was obtained by using S-I-3 (30g, 75.89mmol) obtained in the above synthesis, triflic acid (100.7ml, 1138.35mmol), pyridine aqueous solution (1329.6ml, pyridine : H₂O = 1 : 5) in the same manner as described above for the synthesis of M-I-1.

### Synthesis of M-I-4

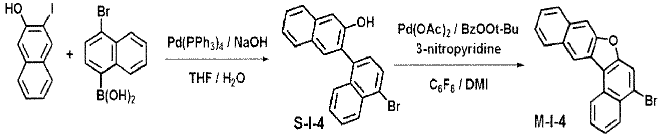

### 1) Synthesis of S-I-4

30.29g (yield: 64%) of the product was obtained by using (4-bromonaphthalen-1-yl)boronic acid (34g, 135.52mmol), THF (596ml), 3-iodonaphthalen-2-ol (36.60g, 135.52mmol), Pd(PPh₃)₄ (2.35g, 2.03mmol), NaOH (8.13g, 203.28mmol), and water (298ml) in the same manner as described above for the synthesis of S-I-1.

### 2) Synthesis of M-I-4

The starting material S-I-4 (30g, 85.90mmol) was added into a round bottom flask together with Pd(OAc)₂ (1.93g, 8.59mmol), 3-nitropyridine (1.07g, 8.59mmol) and dissolved in C₆F₆ (128.9ml) and DMI (85.9ml). Then, *tert-butyl* peroxybenzoate (33.37g, 171.81mmol) was added and stirred at 90°C. When the reaction was completed, the reaction product was extracted with CH₂Cl₂ and water, and then the organic layer was dried with MgSO₄ and concentrated. Then, the concentrate was passed through silica gel column and recrystallized to obtain 21.18g (yield: 71%) of product.

### Synthesis of M-I-5

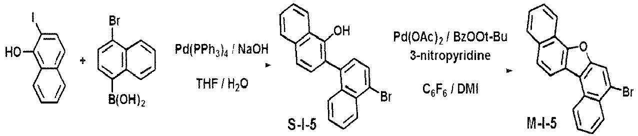

### 1) Synthesis of S-I-5

30.21g (yield: 62%) of the product was obtained by using (4-bromonaphthalen-1-yl)boronic acid (35g, 139.50mmol), THF (614ml), 2-iodonaphthalen-1-ol (37.68g, 139.50mmol), Pd(PPh₃)₄ (2.42g, 2.09mmol), NaOH (8.37g, 209.26mmol), and water (307ml) in the same manner as described above for the synthesis of S-I-1.

### 2) Synthesis of M-I-5

22.07g (yield: 74%) of the product was obtained by using S-I-5 (30g, 85.90mmol) obtained in the above synthesis, Pd(OAc)₂ (1.93g, 8.59mmol), 3-nitropyridine (1.07g, 8.59mmol), C₆F₆ (128.9ml), DMI (85.9ml), *tert-butyl* peroxybenzoate (33.37g, 171.81mmol) in the same manner as described above for the synthesis of M-I-4.

### Synthesis of M-I-6

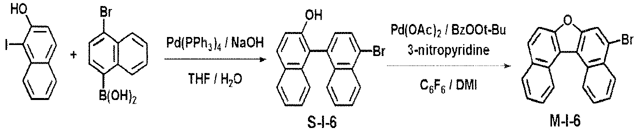

### 1) Synthesis of S-I-6

31.67g (yield: 65%) of the product was obtained by using (4-bromonaphthalen-1-yl)boronic acid (35g, 139.50mmol), THF (614ml), 1-iodonaphthalen-2-ol (37.68g, 139.50mmol), Pd(PPh₃)₄ (2.42g, 2.09mmol), NaOH (8.37g, 209.26mmol), and water (307ml) in the same manner as described above for the synthesis of S-I-1.

### 2) Synthesis of M-I-6

22.67g (yield: 76%) of the product was obtained by using S-I-6 (30g, 85.90mmol) obtained in the above synthesis, Pd(OAc)₂ (1.93g, 8.59mmol), 3-nitropyridine (1.07g, 8.59mmol), C₆F₆ (128.9ml), DMI (85.9ml), *tert*-butyl peroxybenzoate (33.37g, 171.81mmol) in the same manner as described above for the synthesis of M-I-4.

### 1. Synthesis example of Sub 1-1

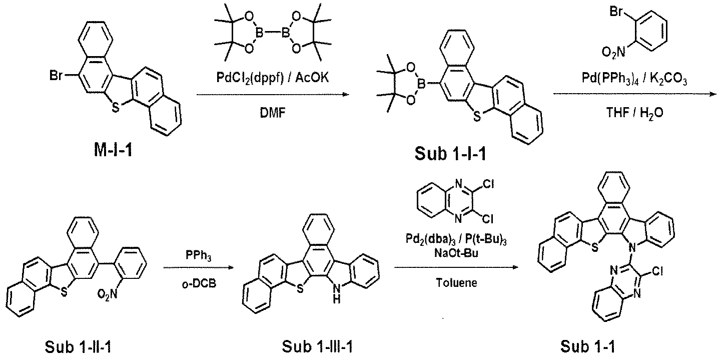

### (1) Synthesis of Sub 1-I-1

The starting material M-I-1 (70g, 192.69mmol) was dissolved in DMF (1214ml) in a round bottom flask, and then Bis(pinacolato)diboron (53.83g, 211.96mmol), Pd(dppf)Cl₂ (4.23g, 5.78mmol), KOAc(56.73g, 578.08mmol) were added and stirred at 90°C. When the reaction was completed, DMF was removed by distillation and the reaction product was extracted with CH₂Cl₂ and water. Then, the organic layer was dried with MgSO₄ and concentrated. Then, the concentrate was passed through silica gel column and recrystallized to obtain 64.05g (yield: 81%) of the product.

### (2) Synthesis of Sub 1-II-1

Sub 1-I-1 (63.2g, 154.02mmol) obtained in the above synthesis was dissolved in THF (216ml) in a round bottom flask, and then 1-bromo-2-nitrobenzene (34.22g, 169.42mmol), Pd(PPh₃)₄ (4.23g, 4.62mmol), K₂CO₃ (44.40g, 462.06mmol), water (108ml) were added and stirred at 80°C. When the reaction was completed, the reaction product was extracted with CH₂Cl₂ and water, and then, the organic layer was dried with MgSO₄ and concentrated. Then, the concentrate was passed through silica gel column and recrystallized to obtain 45.59g (yield: 73%) of the product.

### (3) Synthesis of Sub 1-III-1

Sub 1-II-1 (45.50, 112.22mmol) obtained in the above synthesis was dissolved in *o-*dichlorobenzene (224ml) in a round bottom flask, and then triphenylphosphine (88.30g, 336.65mmol) was added and stirred at 200°C. When the reaction was completed, *o-*dichlorobenzene was removed by distillation and, and then the reaction product was extracted with CH₂Cl₂ and water. Then, the organic layer was dried with MgSO₄ and concentrated. Then, the concentrate was passed through silica gel column and recrystallized to obtain 31.85g (yield: 76%) of the product.

### (4) Synthesis of Sub 1-1

Sub 1-III-1 (30g, 80.33mmol) obtained in the above synthesis was dissolved in toluene (843ml) in a round bottom flask, and then 2,3-dichloroquinoxaline (15.99g, 80.33mmol), Pd₂(dba)₃ (1.1g, 1.2mmol), P(*t*-Bu)₃ (0.81g, 4.02mmol), NaO*t*-Bu (11.58g, 120.49mmol) were added and stirred at 100°C. When the reaction was completed, the reaction product was extracted with CH₂Cl₂ and water, and then, the organic layer was dried with MgSO₄ and concentrated. Then, the concentrate was passed through silica gel column and recrystallized to obtain 17.65g (yield: 41%) of the product.

### 2. Synthesis example of Sub 1-4

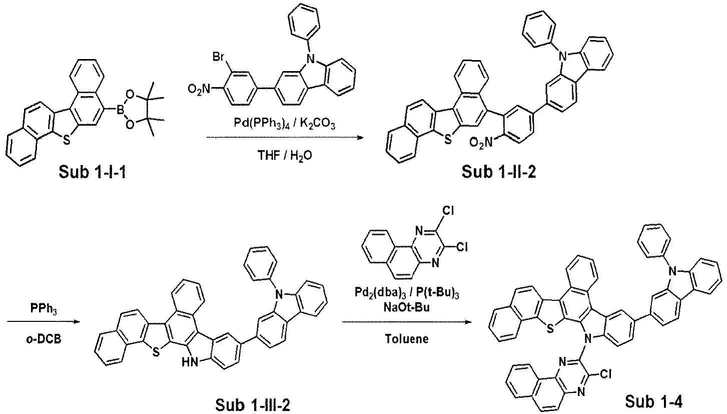

### (1) Synthesis of Sub 1-II-2

2-(3-bromo-4-nitrophenyl)-9-phenyl-9H-carbazole (44.29g, 99.92mmol), Pd(PPh₃)₄ (3.46g, 3.00mmol), K₂CO₃ (41.43g, 299.75mmol), THF (440ml), water (220ml) were added to Sub 1-I-1 (41g, 99.92mmol) obtained in the above synthesis, and then 47.82g (yield: 74%) of the product was obtained by carring out in the same manner as described above for the synthesis of Sub 1-II-1.

### (2) Synthesis of Sub 1-III-2

Triphenylphosphine (57.18g, 218.01mmol), *o*-dichlorobenzene (145ml) were added to Sub 1-II-2 (47g, 72.67mmol) obtained in the above synthesis, and then 28.15g (yield: 63%) of the product was obtained by carring out in the same manner as described above for the synthesis of Sub 1-III-1.

### (3) Synthesis of Sub 1-4

2,3-dichlorobenzo[f]quinoxaline (12.48g, 50.10mmol), Pd₂(dba)₃ (1.25g, 1.37mmol), P(*t*-Bu)₃ (0.74g, 3.64mmol), NaO*t*-Bu (13.13g, 136.64mmol), toluene (228ml) were added to Sub 1-III-2 (28g, 45.55mmol) obtained in the above synthesis, and then 15.07g (yield: 40%) of the product was obtained by carring out in the same manner as described above for the synthesis of Sub 1-1.

### 3. Synthesis example of Sub 1-10

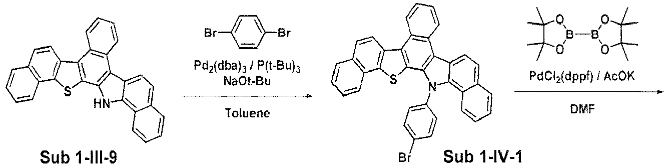

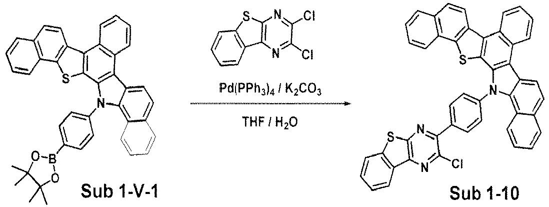

### (1) Synthesis of Sub 1-IV-1

1,4-dibromobenzene (17.16g, 72.72mmol), Pd₂(dba)₃ (0.91g, 0.99mmol), P(*t*-Bu)₃ (0.67g, 3.31mmol), NaO*t*-Bu (9.53g, 99.17mmol), toluene (694ml) were added to Sub 1-III-9 (28g, 66.11mmol) obtained in the above synthesis, and then 27.16g (yield: 71%) of the product was obtained by carring out in the same manner as described above for the synthesis of Sub 1-1.

### (2) Synthesis of Sub 1-V-1

Sub 1-IV-1 (27.16g, 46.95mmol) obtained in the above synthesis was dissolved in DMF (235ml) in a round bottom flask, and Bis(pinacolato)diboron (13.11g, 51.64mmol), Pd(dppf)Cl₂ (1.03g, 1.41mmol), KOAc(13.82g, 140.84mmol) were added, then, stirring at 120°C was followed. When the reaction was completed, DMF was removed by distillation, and the reaction product was extracted with CH₂Cl₂ and water, and then the organic layer was dried with MgSO₄ and concentrated. Then, the concentrate was passed through silica gel column and recrystallized to obtain 22.03g (yield: 75%) of product.

### (3) Synthesis of Sub 1-10

2,3-dichlorobenzo[4,5]thieno[2,3-b]pyrazine (8.98g, 35.21mmol), Pd(PPh₃)₄ (0.61g, 0.53mmol), K₂CO₃ (7.30g, 52.82mmol), THF (155ml), water (77.47ml) were added to Sub 1-V-1 (22.03g, 35.21mmol) obtained in the above synthesis, and then 9.61g (yield: 38%) of the product was obtained by carring out in the same manner as described above for the synthesis of Sub 1-II-1.

### 4. Synthesis example of Sub 1-24

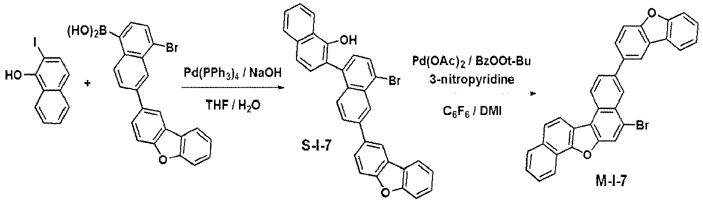

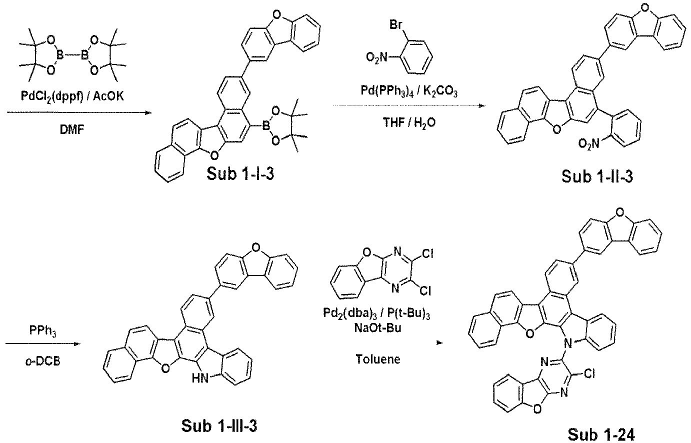

### 1) Synthesis of S-I-7

86.51g (yield: 70%) of the product was obtained by using (4-bromo-6-(dibenzo[b,d]furan-2-yl)naphthalen-1-yl)boronic acid (100g, 239.77mmol), THF (1055ml), 2-iodonaphthalen-1-ol (64.75g, 239.77mmol), Pd(PPh₃)₄ (4.16g, 3.60mmol), NaOH (14.39g, 359.65mmol), and water (527ml) in the same manner as described above for the synthesis of S-I-1.

### 2) Synthesis of M-I-7

The starting material S-I-7 (86g, 166.86mmol) was added into a round bottom flask together with Pd(OAc)₂ (3.75g, 16.69mmol), 3-nitropyridine (2.07g, 16.69mmol) and dissolved in C₆F₆ (250ml) and DMI (167ml). Then, *tert-butyl* peroxybenzoate (64.82g, 333.71mmol) was added and stirred at 90°C. When the reaction was completed, the reaction product was extracted with CH₂Cl₂ and water, and then the organic layer was dried with MgSO₄ and concentrated. Then, the concentrate was passed through silica gel column and recrystallized to obtain 62.53g (yield: 73%) of product.

### 3) Synthesis of Sub 1-I-3

51.86g (yield: 76%) of the product was obtained by using M-I-7 (62.50g, 121.74mmol) obtained in the above synthesis, DMF (609ml), Bis(pinacolato)diboron (34.01g, 133.91mmol), Pd(dppf)Cl₂ (2.67g, 3.65mmol), KOAc (35.84g, 365.22mmol) in the same manner as described above for the synthesis of Sub 1-V-1.

### 4) Synthesis of Sub 1-II-3

1-bromo-2-nitrobenzene (18.67g, 92.42mmol), Pd(PPh₃)₄ (3.2g, 2.77mmol), K₂CO₃ (38.32g, 277.27mmol), THF (407ml), water (203ml) were added to Sub 1-I-3 (51.8g, 92.42mmol) obtained in the above synthesis, and then 43.13g (yield: 84%) of the product was obtained by carring out in the same manner as described above for the synthesis of Sub 1-II-1.

### 5) Synthesis of Sub 1-III-3

Triphenylphosphine (60.9g, 232.19mmol), *o*-dichlorobenzene (155ml) were added to Sub 1-II-3 (43g, 77.4mmol) obtained in the above synthesis, and then 31.61g (yield: 78%) of the product was obtained by carring out in the same manner as described above for the synthesis of Sub 1-III-1.

### 6) Synthesis of Sub 1-24

2,3-dichlorobenzofuro[2,3-b]pyrazine (15.88g, 66.41mmol), Pd₂(dba)₃ (1.66g, 1.81mmol), P(*t*-Bu)₃ (0.98g, 4.83mmol), NaO*t*-Bu (17.41g, 181.11mmol), toluene (302ml) were added Sub 1-III-3 (31.61g, 60.37mmol) obtained in the above synthesis, and then 18.41g (yield: 42%) of the product was obtained by carring out in the same manner as described above for the synthesis of Sub 1-1.

### 5. Synthesis example of Sub 1-41

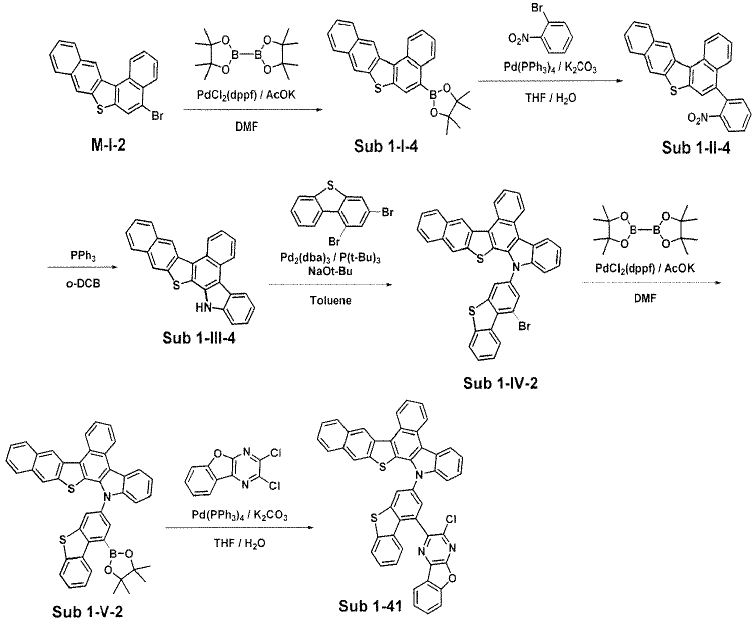

### 1) Synthesis of Sub 1-1-4

93.70g (yield: 79%) of the product was obtained by using M-I-2 (105g, 289.04mmol) obtained in the above synthesis, DMF (1445ml), Bis(pinacolato)diboron (80.74g, 317.95mmol), Pd(dppf)Cl₂ (6.34g, 8.67mmol), KOAc (85.10g, 867.12mmol) in the same manner as described above for the synthesis of Sub 1-V-1.

### 2) Synthesis of Sub 1-II-4

1-bromo-2-nitrobenzene (45.78g, 226.64mmol), Pd(PPh₃)₄ (7.86g, 6.80mmol), K₂CO₃ (93.97g, 679.92mmol), THF (997ml), water (498ml) were added to M-I-4 (93g, 226.64mmol) obtained in the above synthesis, and then 76.27g (yield: 83%) of the product was obtained by carring out in the same manner as described above for the synthesis of Sub 1-II-1.

### 3) Synthesis of Sub 1-III-4

Triphenylphosphine (147.49g, 562.31mmol), *o*-dichlorobenzene (375ml) were added to Sub 1-II-4 (76g, 187.44mmol) obtained in the above synthesis, and then 53.20g (yield: 76%) of the product was obtained by carring out in the same manner as described above for the synthesis of Sub 1-III-1.

### 4) Synthesis of Sub 1-IV-2

1,3-dibromodibenzo[b,d]thiophene (48.54g, 141.91mmol), Pd₂(dba)₃ (1.95g, 2.13mmol), P(*t*-Bu)₃ (1.44g, 7.10mmol), NaO*t*-Bu (20.46g, 212.87mmol), toluene (1490ml) were added to Sub 1-III-4 (53g, 141.91mmol) obtained in the above synthesis, and then 32.42g (yield: 36%) of the product was obtained by carring out in the same manner as described above for the synthesis of Sub 1-1.

### 5) Synthesis of Sub 1-V-2

25.09g (yield: 73%) of the product was obtained by using Sub 1-IV-2 (32g, 50.42mmol) obtained in the above synthesis, DMF (252ml), Bis(pinacolato)diboron (14.09g, 55.47mmol), Pd(dppf)Cl₂ (1.11g, 1.51mmol), KOAc (14.85g, 151.27mmol) in the same manner as described above for the synthesis of Sub 1-V-1.

### 6) Synthesis of Sub 1-41

2,3-dichlorobenzofuro[2,3-b]pyrazine (8.77g, 36.67mmol), Pd(PPh₃)₄ (0.64g, 0.55mmol), K₂CO₃ (7.60g, 55.01mmol), THF (161ml), water (80ml) were added to Sub 1-V-2 (25g, 36.67mmol) obtained in the above synthesis, and then 11.40g (yield: 41%) of the product was obtained by carring out in the same manner as described above for the synthesis of Sub 1-II-1.

### 6. Synthesis example of Sub 1-18

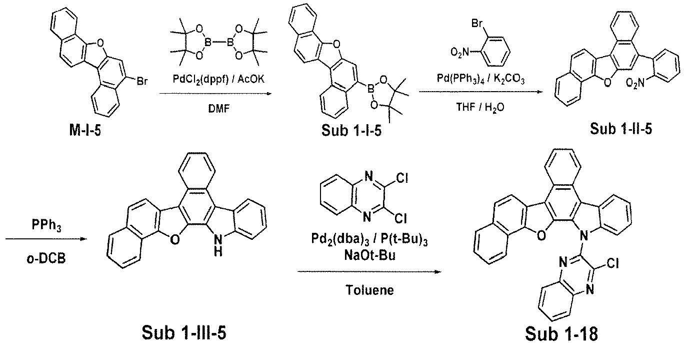

### 1) Synthesis of Sub 1-1-5

17.91g (yield: 83%) of the product was obtained by using M-I-5 (19g, 54.72mmol) obtained in the above synthesis, DMF (274ml), Bis(pinacolato)diboron (15.29g, 60.19mmol), Pd(dppf)Cl₂ (1.20g, 1.64mmol), KOAc (16.11g, 164.17mmol) in the same manner as described above for the synthesis of Sub 1-V-1.

### 2) Synthesis of Sub 1-II-5

1-bromo-2-nitrobenzene (8.71g, 43.12mmol), Pd(PPh₃)₄ (1.49g, 1.29mmol), K₂CO₃ (17.88g, 129.35mmol), THF (189ml), water (95ml) were added to Sub 1-1-5 (17g, 43.12mmol) obtained in the above synthesis, and then 14.61g (yield: 87%) of the product was obtained by earring out in the same manner as described above for the synthesis of Sub 1-II-1.

### 3) Synthesis of Sub 1-III-5

Triphenylphosphine (29.30g, 111.71mmol), o-dichlorobenzene (74ml) were added to Sub 1-II-5 (14.5g, 37.24mmol) obtained in the above synthesis, and then 10.65g (yield: 80%) of the product was obtained by carring out in the same manner as described above for the synthesis of Sub 1-III-1.

### 4) Synthesis of Sub 1-18

2,3-dichloroquinoxaline (5.85g, 29.38mmol), Pd₂(dba)₃ (0.40g, 0.44mmol), P(*t*-Bu)₃ (0.30g, 1.47mmol), NaO*t*-Bu (4.23g, 44.07mmol), toluene (308ml) were added to Sub 1-III-5 (10.5g, 29.38mmol) obtained in the above synthesis, and then 11g (yield: 72%) of the product was obtained by carring out in the same manner as described above for the synthesis of Sub 1-1.

### 7. Synthesis example of Sub 1-44

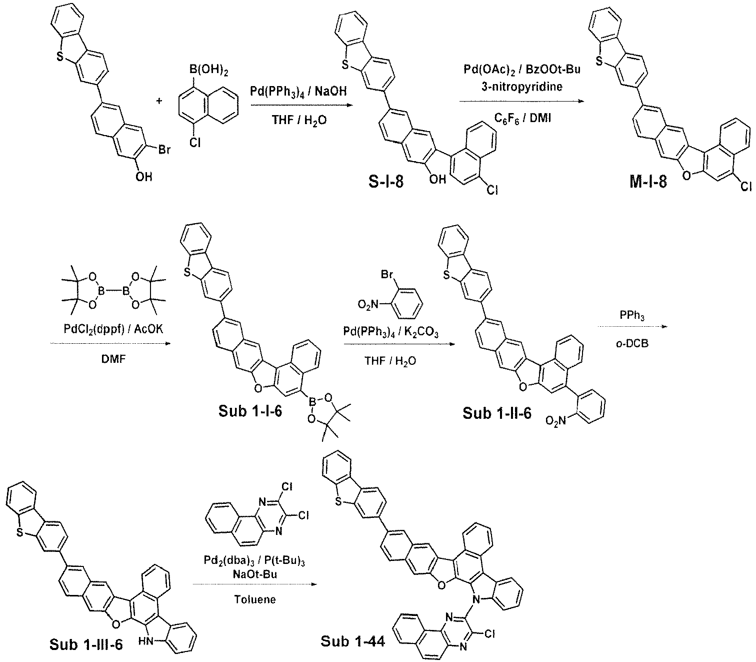

### 1) Synthesis of S-I-8

(4-chloronaphthalen-1-yl)boronic acid (31.07g, 150.5mmol), THF (662ml), 3-bromo-6-(dibenzo[b,d]thiophen-3-yl)naphthalen-2-ol (61g, 150.5mmol), Pd(PPh₃)₄ (2.61g, 2.26mmol), NaOH (9.03g, 225.75mmol), water (331ml) were reacted in the same manner as described above for the synthesis of Sub S-I-1 to obtain 51.31g (yield: 70%) of the product.

### 2) Synthesis of M-I-8

S-I-8 (51g, 104.72mmol), Pd(OAc)₂ (2.35g, 10.47mmol), 3-nitropyridine (1.30g, 10.47mmol) were dissolved in C₆F₆ (157.1ml) and DMI (104ml) and *tert-butyl* peroxybenzoate (40.68g, 209.44mmol) was added, and then, 36.57g (yield: 72%) of the product was obtained by carring out in the same manner as described above for the synthesis of M-I-8.

### 3) Synthesis of Sub 1-I-6

34.23g (yield: 80%) of the product was obtained by using M-I-8 (36g, 74.23mmol) obtained in the above synthesis, DMF (371ml), Bis(pinacolato)diboron (20.73g, 81.65mmol), Pd(dppf)Cl₂ (1.63g, 2.23mmol), KOAc (21.85g, 222.68mmol) in the same manner as described above for the synthesis of Sub 1-V-1.

### 4) Synthesis of Sub 1-II-6

1-bromo-2-nitrobenzene (11.91g, 58.97mmol), Pd(PPh₃)₄ (2.04g, 1.77mmol), K₂CO₃ (24.45g, 176.92mmol), THF (259ml), water (129ml) were added to Sub 1-I-6 (34g, 58.97mmol) obtained in the above synthesis, and then 27.64g (yield: 82%) of the product was obtained by carring out in the same manner as described above for the synthesis of Sub 1-II-1.

### 5) Synthesis of Sub 1-III-6

Triphenylphosphine (37.85g, 144.32mmol), *o*-dichlorobenzene (96ml) were added to Sub 1-II-6 (27.5g, 48.11mmol) obtained in the above synthesis, and then 20.25g (yield: 78%) of the product was obtained by carring out in the same manner as described above for the synthesis of Sub 1-III-1.

### 6) Synthesis of Sub 1-44

2,3-dichlorobenzo[f]quinoxaline (9.23g, 37.06mmol), Pd₂(dba)₃ (0.51g, 0.56mmol), P(*t-*Bu)₃ (0.37g, 1.85mmol), NaO*t*-Bu (5.34g, 55.59mmol), toluene (389ml) were added to Sub 1-III-6 (20g, 37.06mmol) obtained in the above synthesis, and then 11.15g (yield: 40%) of the product was obtained by carring out in the same manner as described above for the synthesis of Sub 1-1.

### 8. Synthesis example of Sub 1-52

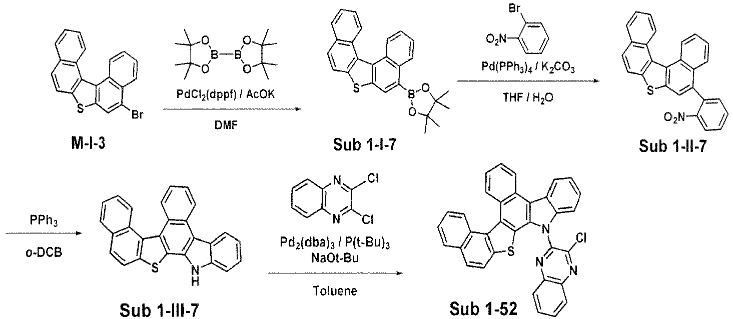

### 1) Synthesis of Sub 1-I-7

20.38g (yield: 82%) of the product was obtained by using M-I-3 (22g, 60.56mmol) obtained in the above synthesis, DMF (302ml), Bis(pinacolato)diboron (16.92g, 66.62mmol), Pd(dppf)Cl₂ (1.33g, 1.82mmol), KOAc (17.83g, 181.68mmol) in the same manner as described above for the synthesis of Sub 1-V-1.

### 2) Synthesis of Sub 1-II-7

1-bromo-2-nitrobenzene (9.6g, 47.52mmol), Pd(PPh₃)₄ (1.65g, 1.43mmol), K₂CO₃ (19.70g, 142.56mmol), THF (209ml), water (105ml) were added Sub 1-I-7 (19.5g, 47.52mmol) obtained in the above synthesis, and then 16.38g (yield: 85%) of the product was obtained by carring out in the same manner as described above for the synthesis of Sub 1-II-1.

### 3) Synthesis of Sub 1-III-7

Triphenylphosphine (31.05g, 118.38mmol), *o*-dichlorobenzene (79ml) were added to Sub 1-II-7 (16g, 39.46mmol) obtained in the above synthesis, and then 12.08g (yield: 82%) of the product was obtained by carring out in the same manner as described above for the synthesis of Sub 1-III-1.

### 4) Synthesis of Sub 1-52

2,3-dichloroquinoxaline (6.40g, 32.13mmol), Pd₂(dba)₃ (0.44g, 0.48mmol), P(*t*-Bu)₃ (0.33g, 1.61mmol), NaO*t*-Bu (4.63g, 48.20mmol), toluene (337ml) were added to Sub 1-III-7 (12g, 32.13mmol) obtained in the above synthesis, and then 11.71g (yield: 68%) of the product was obtained by carring out in the same manner as described above for the synthesis of Sub 1-1.

### 9. Synthesis example of Sub 1-64

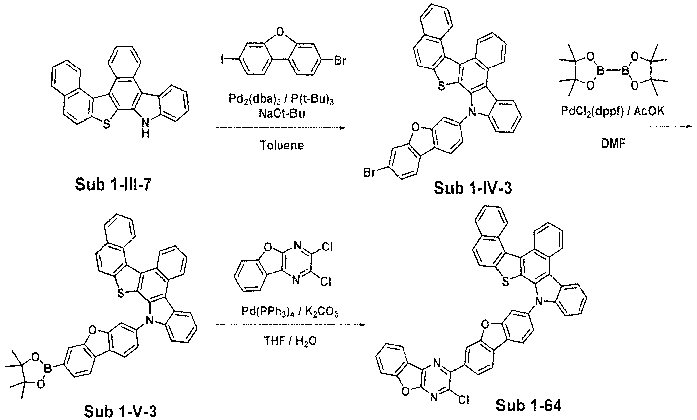

### 1) Synthesis of Sub 1-IV-3

3-bromo-7-iododibenzo[b,d]furan (38.95g, 104.43mmol), Pd₂(dba)₃ (1.43g, 1.57mmol), P(*t*-Bu)₃ (1.06g, 5.22mmol), NaO*t*-Bu (15.05g, 156.64mmol), toluene (1096ml) were added to Sub 1-III-7 (39g, 104.43mmol) obtained in the above synthesis, and then 38.76g (yield: 60%) of the product was obtained by earring out in the same manner as described above for the synthesis of Sub 1-1.

### 2) Synthesis of Sub 1-V-3

31.49g (yield: 76%) of the product was obtained by using Sub 1-IV-3 (38.5g, 62.24mmol) obtained in the above synthesis, DMF (311ml), Bis(pinacolato)diboron (17.39g, 68.47mmol), Pd(dppf)Cl₂ (1.37g, 1.87mmol), KOAc (18.33g, 186.73mmol) in the same manner as described above for the synthesis of Sub I-V-1.

### 3) Synthesis of Sub 1-64

2,3-dichlorobenzofuro[2,3-b]pyrazine (11.13g, 46.57mmol), Pd(PPh₃)₄ (0.81g, 0.70mmol), K₂CO₃ (9.66g, 69.86mmol), THF (205ml), water (102ml) were added to Sub 1-V-3 (31g, 46.57mmol) obtained in the above synthesis, and then 11.06g (yield: 32%) of the product was obtained by carring out in the same manner as described above for the synthesis of Sub 1-11-1.

### 10. Synthesis example of Sub 1-70

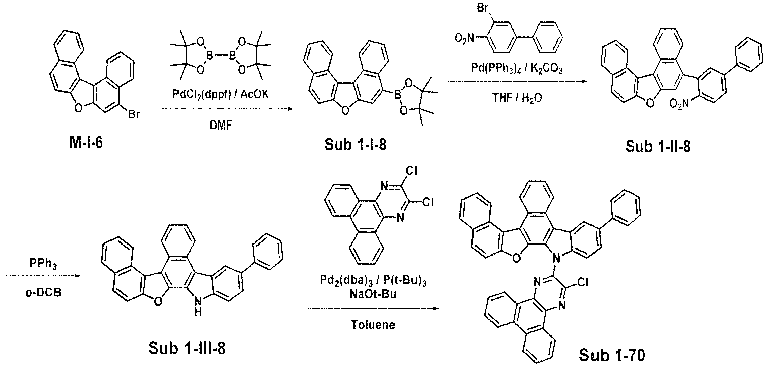

### 1) Synthesis of Sub 1-I-8

17.94g (yield: 79%) of the product was obtained by using M-I-6 (20g, 57.60mmol) obtained in the above synthesis, DMF (288ml), Bis(pinacolato)diboron (16.09g, 63.36mmol), Pd(dppf)Cl₂ (1.26g, 1.73mmol), KOAc (16.96g, 172.81mmol) in the same manner as described above for the synthesis of Sub I-V-1.

### 2) Synthesis of Sub 1-II-8

3-bromo-4-nitro-1,1'-biphenyl (11.99g, 43.12mmol), Pd(PPh₃)₄ (1.49g, 1.29mmol), K₂CO₃ (17.88g, 129.35mmol), THF (189ml), water (94ml) were added to Sub 1-I-8 (17g, 43.12mmol) obtained in the above synthesis, and then 15.45g (yield: 77%) of the product was obtained by carring out in the same manner as described above for the synthesis of Sub 1-II-1.

### 2) Synthesis of Sub 1-III-8

Triphenylphosphine (25.36g, 96.67mmol), *o*-dichlorobenzene (64ml) were added to Sub 1-II-8 (15g, 32.22mmol) obtained in the above synthesis, and then 11.73g (yield: 84%) of the product was obtained by carring out in the same manner as described above for the synthesis of Sub 1-III-1.

### 3) Synthesis of Sub 1-70

2,3-dichlorodibenzo[f,h]quinoxaline (7.59g, 25.37mmol), Pd₂(dba)₃ (0.35g, 0.38mmol), P(*t*-Bu)₃ (0.26g, 1.27mmol), NaO*t*-Bu (3.66g, 38.06mmol), toluene (266ml) were added to Sub 1-III-8 (11g, 25.37mmol) obtained in the above synthesis, and then 11.13g (yield: 63%) of the product was obtained by carring out in the same manner as described above for the synthesis of Sub 1-1.

### Example of Sub 1

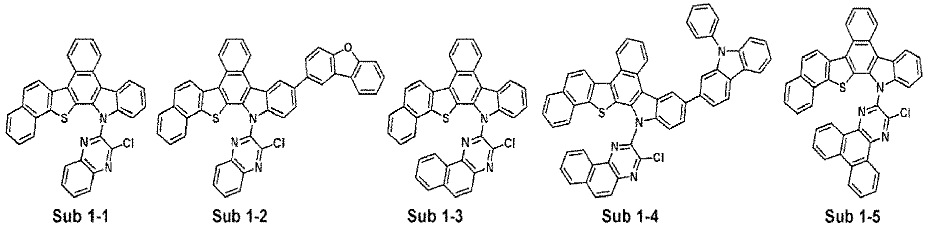

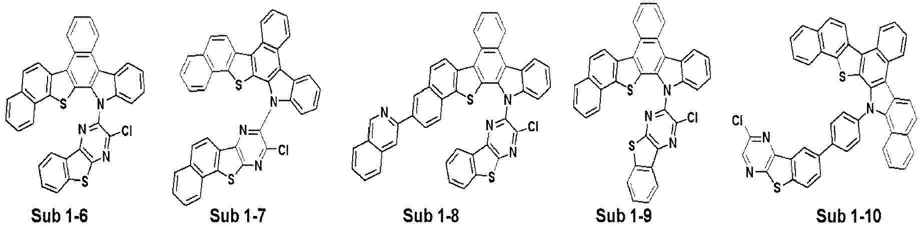

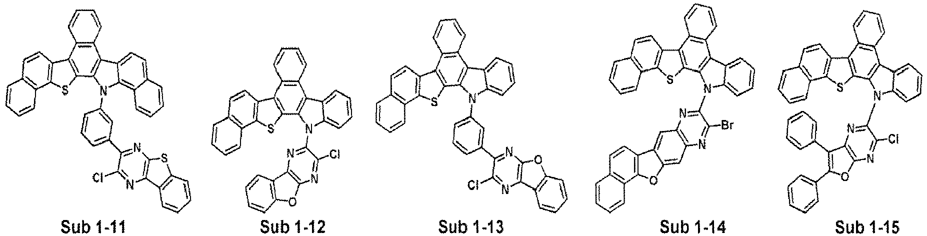

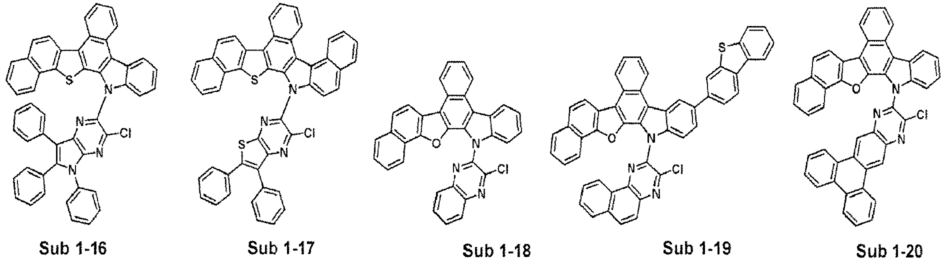

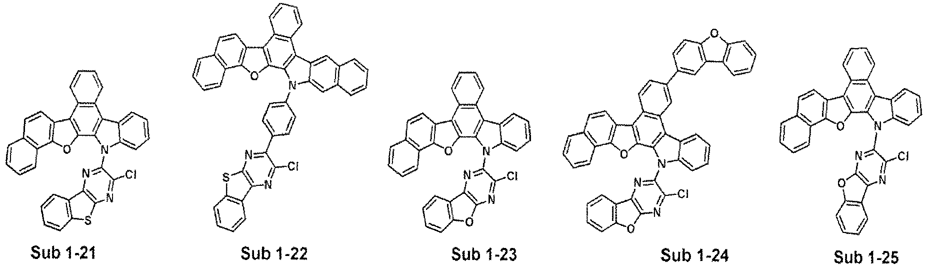

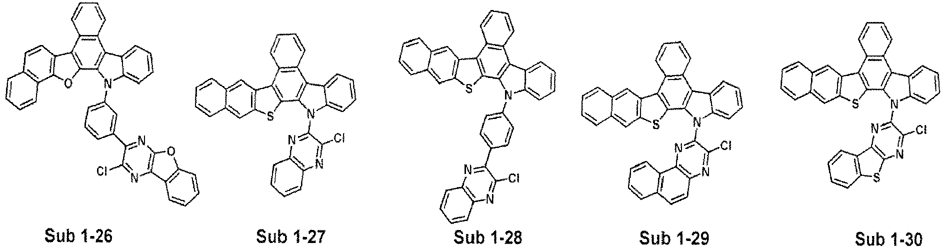

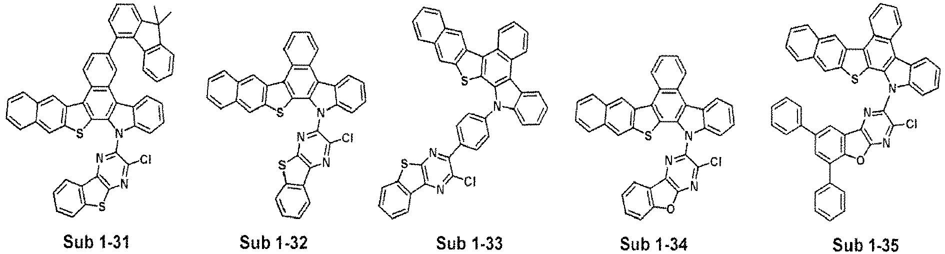

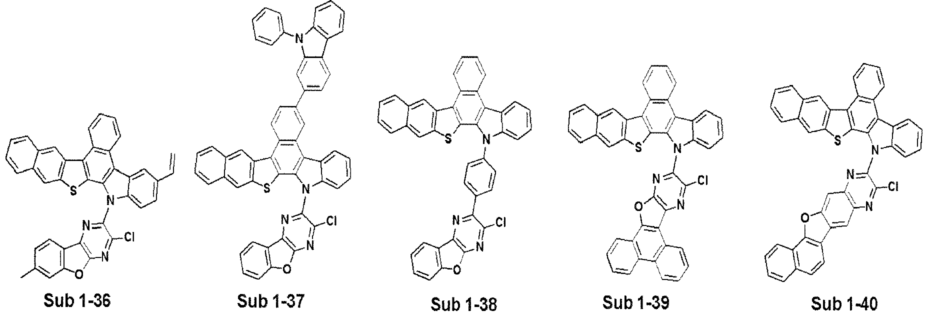

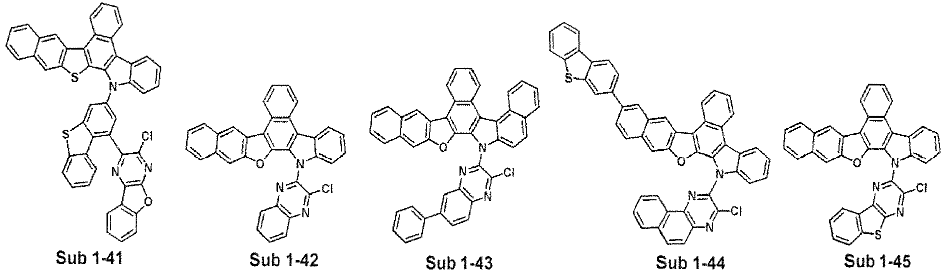

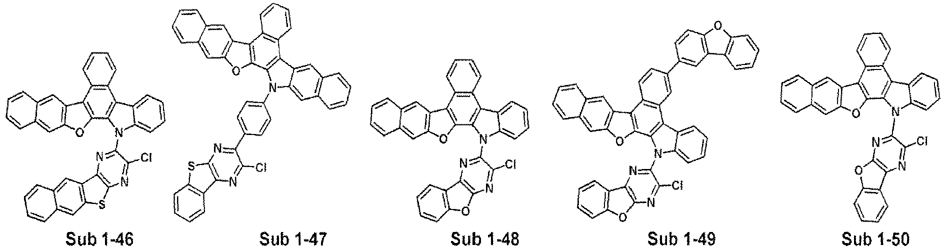

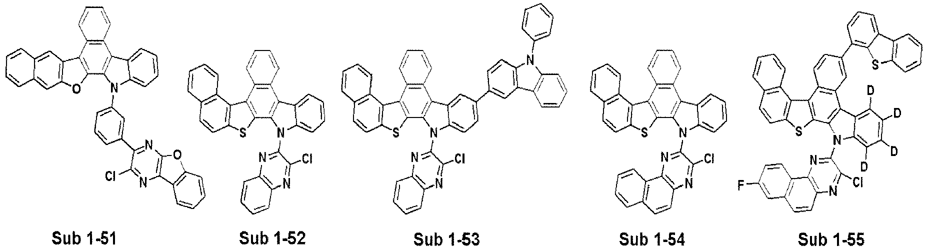

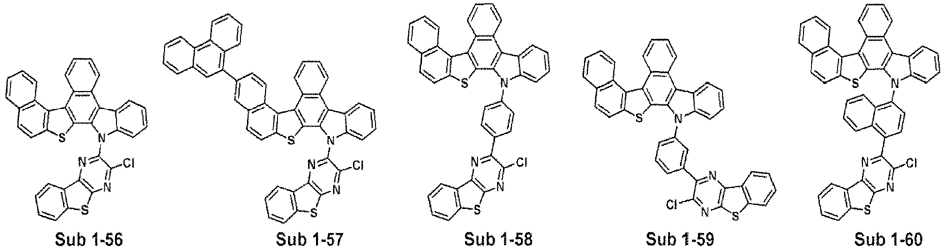

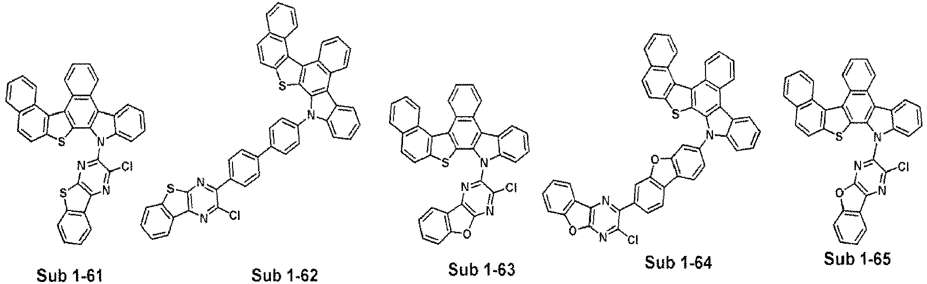

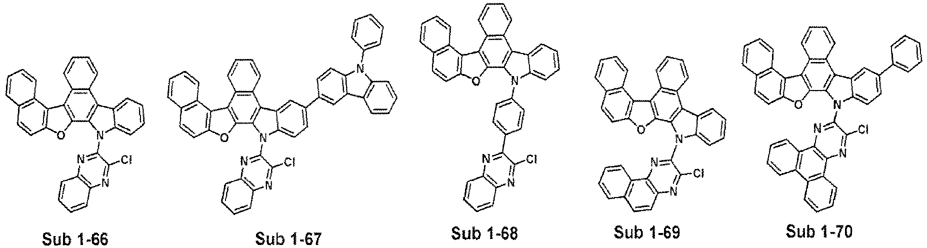

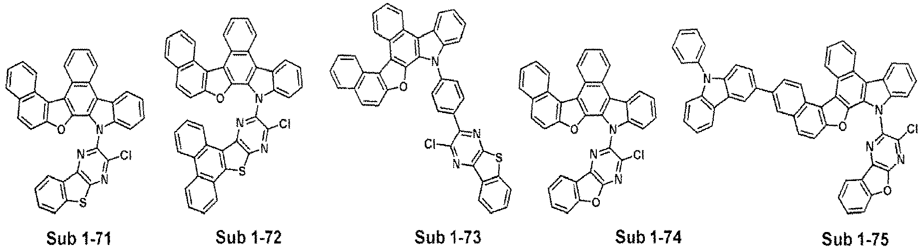

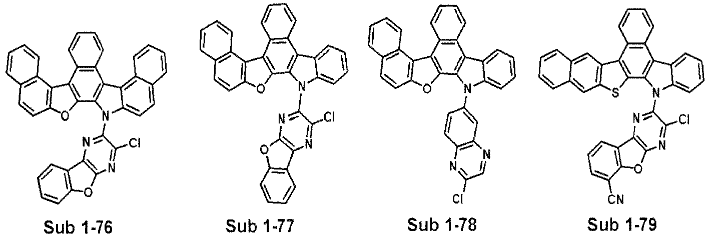

**[Table 1]**

| Compound | FD-MS | Compound | FD-MS |
|---|---|---|---|
| Sub 1-1 | m/z=541.05(C₃₂H₁₆ClN₃S₂=542.07) | Sub 1-4 | m/z=782.14(C₅₀H₂₇ClN₄S₂=783.36) |
| Sub 1-10 | m/z=717.11(C₄₆H₂₄ClN₃S₂=718.29) | Sub 1-18 | m/z=519.11(C₃₄H₁₈ClN₃O=519.99) |
| Sub 1-24 | m/z=725.15(C₄₈H₂₄ClN₃O₃=726.19) | Sub 1-41 | m/z=7.57.10(C₄₈H₂₄ClN₃OS₂=758.31) |
| Sub 1-44 | m/z=751.15(C₅₀H₂₆ClN₃OS=752.29) | Sub 1-52 | m/z=535.09(C₃₄H₁₈ClN₃S=536.05) |
| Sub 1-64 | m/z=741.13(C₄₈H₂₄ClN₃O₂S=742.25) | Sub 1-70 | m/z=695.18(C₄₈H₂₆ClN₃O=696.21) |

### II. Synthesis of Sub 2

Sub 2 of the Reaction Scheme 1 can be synthesized according to, but not limited to, the reaction route of the following Reaction Scheme.

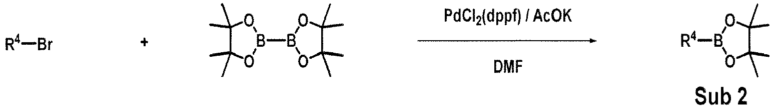

Synthesis Examples of compounds comprised in Sub 2 are as follows.

### 1. Synthesis example of Sub 2-1

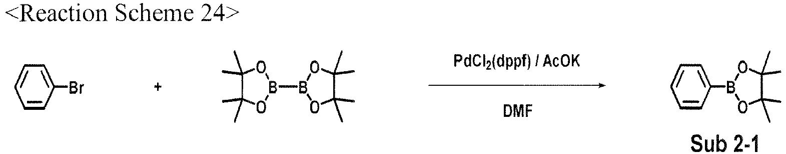

The starting material bromobenzene (29.16 g, 185.72 mmol) was dissolved in DMF (930ml) in a round bottom flask, and then Bis(pinacolato)diboron (51.88 g, 204.29 mmol), Pd(dppf)Cl₂ (4.55 g, 5.57 mmol), KOAc (54.68 g, 557.16 mmol) were added and stirred at 90°C. When the reaction was completed, DMF was removed by distillation and the reaction product was extracted with CH₂Cl₂ and water. Then, the organic layer was dried with MgSO₄ and concentrated. Then, the concentrate was passed through silica gel column and recrystallized to obtain 31.84 g (yield: 84%) of the product.

### 2. Synthesis example of Sub 2-3

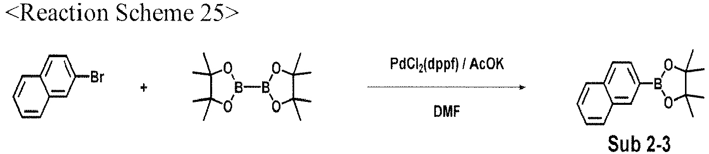

Bis(pinacolato)diboron (29.04 g, 114.37 mmol), Pd(dppf)Cl₂ (2.55 g, 3.12 mmol), KOAc (30.61 g, 311.92 mmol), DMF (520ml) were added to 2-bromonaphthalene (21.53 g, 103.97 mmol) being starting material, and then 21.14 g (yield: 80%) of the product was obtained by carring out in the same manner as described above for the synthesis of Sub 2-1.

### 3. Synthesis example of Sub 2-5

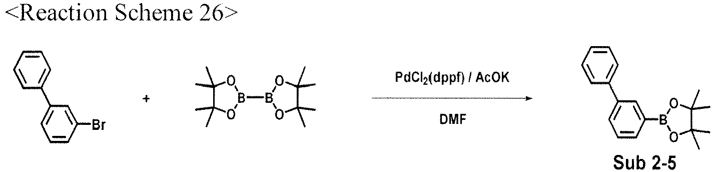

Bis(pinacolato)diboron (19.46 g, 76.63 mmol), Pd(dppf)Cl₂ (1.71 g, 2.09 mmol), KOAc (20.51 g, 209.00 mmol), DMF (350ml) were added to 3-bromo-1,1'-biphenyl (16.24 g, 69.67 mmol) being starting material, and then 15.81 g (yield: 81%) of the product was obtained by carring out in the same manner as described above for the synthesis of Sub 2-1.

### 4. Synthesis example of Sub 2-12

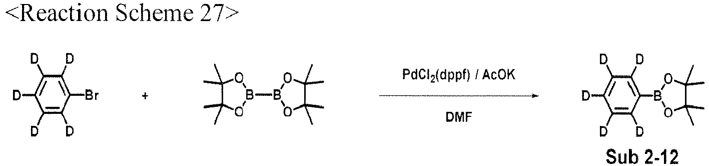

Bis(pinacolato)diboron (18.70 g, 73.65 mmol), Pd(dppf)Cl₂ (1.64 g, 2.01 mmol), KOAc (19.71 g, 200.88 mmol), DMF (335ml) were added to 1-bromobenzene-2,3,4,5,6-d5 (10.85 g, 66.96 mmol) being starting material, and then 10.22 g (yield: 73%) of the product was obtained by earring out in the same manner as described above for the synthesis of Sub 2-1.

### 5. Synthesis example of Sub 2-21

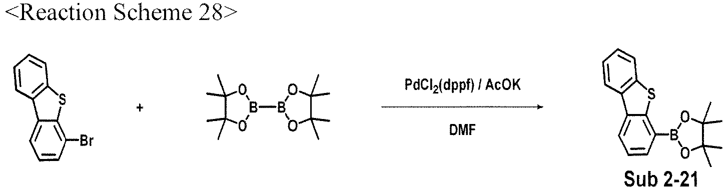

Bis(pinacolato)diboron (15.11 g, 59.48 mmol), Pd(dppf)Cl₂(1.32 g, 1.62 mmol), KOAc (15.92 g, 162.23 mmol), DMF (270ml) were added to 4-bromodibenzo[b,d]thiophene (14.23 g, 54.08 mmol) being starting material, and then 13.76 g (yield: 82%) of the product was obtained by carring out in the same manner as described above for the synthesis of Sub 2-1.

### 6. Synthesis example of Sub 2-28

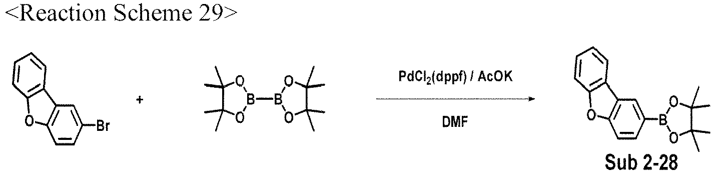

Bis(pinacolato)diboron (18.48 g, 72.79 mmol), Pd(dppf)Cl₂(1.62 g, 1.99 mmol), KOAc (19.48 g, 198.51 mmol), DMF (330ml) were added to 2-bromodibenzo[b,d]furan (16.35 g, 66.17 mmol) being starting material, and then 16.74 g (yield: 86%) of the product was obtained by carring out in the same manner as described above for the synthesis of Sub 2-1.

### 7. Synthesis example of Sub 2-32

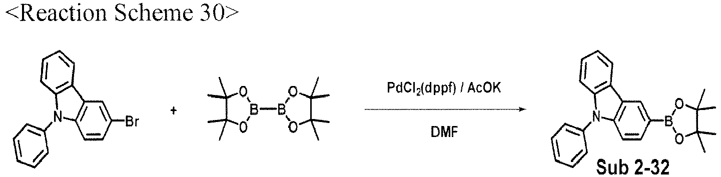

Bis(pinacolato)diboron (10.55 g, 41.55 mmol), Pd(dppf)Cl₂ (0.93 g, 1.13 mmol), KOAc (11.12 g, 113.31 mmol), DMF (190ml) were added to 3-bromo-9-phenyl-9H-carbazole (12.17 g, 37.77 mmol) being starting material, and then 10.46 g (yield: 75%) of the product was obtained by carring out in the same manner as described above for the synthesis of Sub 2-1.

### 8. Synthesis example of Sub 2-27

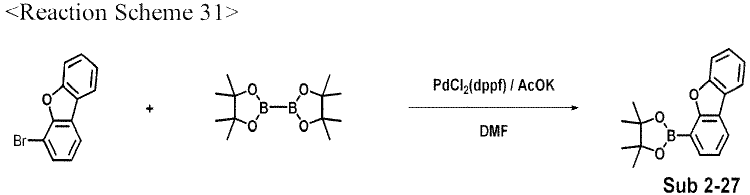

Bis(pinacolato)diboron (18.97 g, 74.70 mmol), Pd(dppf)Cl₂ (1.66 g, 2.04 mmol), KOAc (19.99 g, 203.73 mmol), DMF (340ml) were added to 1-bromodibenzo[b,d]furan (16.78 g, 67.91 mmol) being starting material, and then 15.98 g (yield: 80%) of the product was obtained by carring out in the same manner as described above for the synthesis of Sub 2-1.

### 9. Synthesis example of Sub 2-36

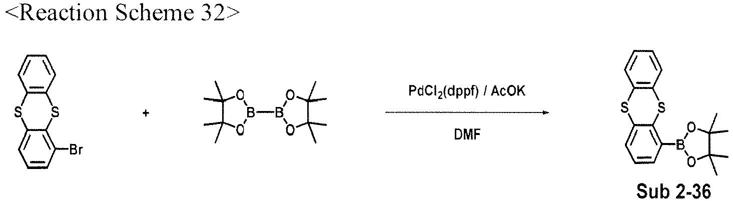

Bis(pinacolato)diboron (12.59 g, 49.60 mmol), Pd(dppf)Cl₂ (1.10 g, 1.35 mmol), KOAc (13.27 g, 135.26 mmol), DMF (225ml) were added to 1-bromothianthrene (13.31 g, 45.09 mmol) being starting material, and then 10.34 g (yield: 67%) of the product was obtained by carring out in the same manner as described above for the synthesis of Sub 2-1.

The compound belonging to Sub 2 may be, but not limited to, the following compounds, and Table 2 shows FD-MS (Field Desorption-Mass Spectrometry) values of compounds belonging to Sub 2.

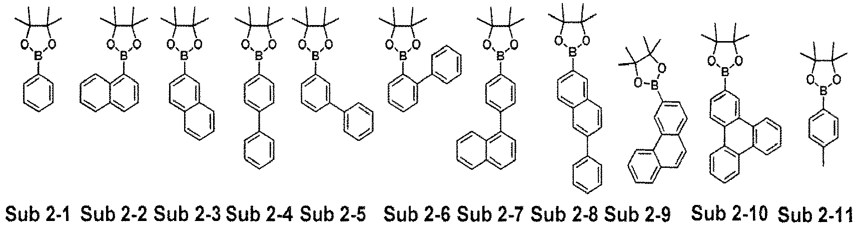

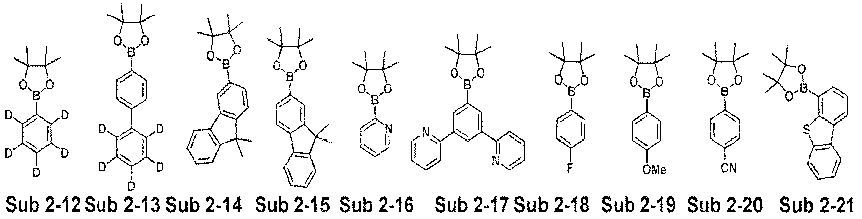

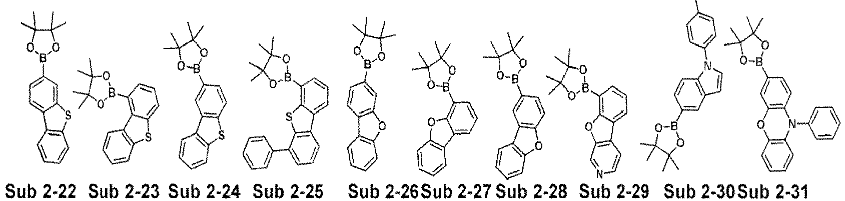

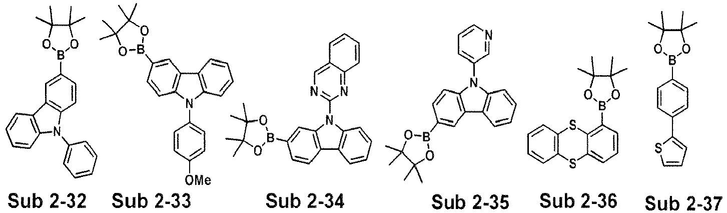

**[Table 2]**

| Compound | FD-MS | Compound | FD-MS |
|---|---|---|---|
| Sub 2-1 | m/z=204.13(C₁₂H₁₇BO₂=204.08) | Sub 2-3 | m/z=254.15(C₁₆H₁₉BO₂= 254.14) |
| Sub 2-5 | m/z=280.16(C₁₈H₂₁BO₂=280.17) | Sub 2-12 | m/z=209.16(C₁₂H₁₂D₅BO₂=209.11) |
| Sub 2-21 | m/z=310.12(C₁₈H₁₉BO₂S=310.22) | Sub 2-28 | m/z=294.14(C₁₈H₁₉BO₃=294.16) |
| Sub 2-32 | m/z=369.19(C₂₄H₂₄BNO₂=369.27) | Sub 2-27 | m/z=294.14(C₁₈H₁₉BO₃=294.16) |
| Sub 2-36 | m/z=342.09(C₁₈H₁₉BO₂S₂=342.28) | | |

### III. Synthesis of Product

Sub 1 (1 eq.) was dissolved in THF in a round bottom flask, and Pd(PPh₃)₄ (0.04 eq.), NaOH (3 eq.) and water were added, then, stirring at 70°C was followed. When the reaction was completed, the reaction product was extracted with CH₂Cl₂ and water, and then the organic layer was dried with MgSO₄ and concentrated. Then, the concentrate was passed through silica gel column and recrystallized to obtain final product.

### Synthesis example of 1-1

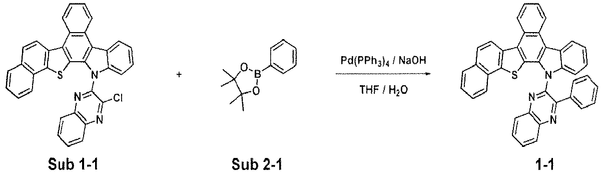

Sub 1-1 (9g, 16.79mmol) was dissolved in THF(74ml) in a round bottom flask, and Sub 2-1 (3.43g, 16.79mmol), Pd(PPh₃)₄(0.78g, 0.67mmol), NaOH (2.01g, 50.37mmol) and water (37ml) were added, then, stirring at 70°C was followed. When the reaction was completed, the reaction product was extracted with CH₂Cl₂ and water, and then the organic layer was dried with MgSO₄ and concentrated. Then, the concentrate was passed through silica gel column and recrystallized to obtain 7.47g (yield: 77%) of product.

### Synthesis example of 1-14

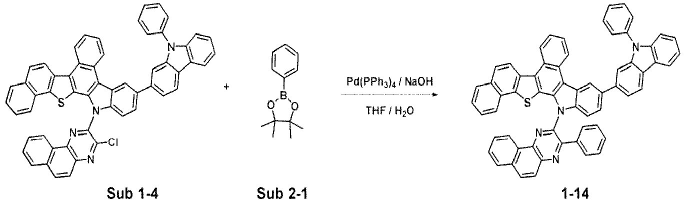

Sub 1-4 (9g, 10.88mmol), THF(48ml), Sub 2-1 (2.22g, 10.88mmol), Pd(PPh₃)₄ (0.5g, 0.44mmol), NaOH (1.31g, 32.63mmol), water (24ml) were carried out in the same manner as described above for the synthesis of the compound 1-1 to obtain 6.71g of the product (yield: 71%).

### Synthesis example of 1-30

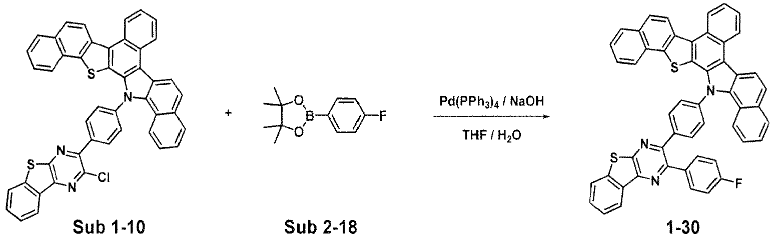

Sub 1-10 (11g, 15.31mmol), THF(67ml), Sub 2-18 (3.4g, 15.31mmol), Pd(PPh₃)₄(0.71g, 0.61mmol), NaOH (1.84g, 45.94mmol), water (34ml) were carried out in the same manner as described above for the synthesis of the compound 1-1 to obtain 7.51g of the product (yield: 63%).

### Synthesis example of 1-44

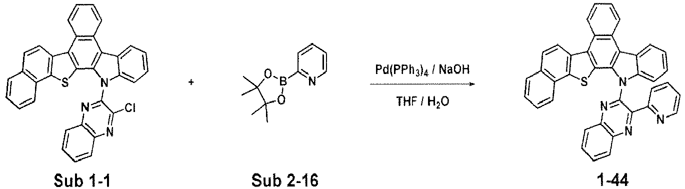

Sub 1-1 (11g, 20.52mmol), THF(90ml), Sub 2-16 (4.21g, 20.52mmol), Pd(PPh₃)₄(0.95g, 0.82mmol), NaOH (2.46g, 61.56mmol), water (45ml) were carried out in the same manner as described above for the synthesis of the compound 1-1 to obtain 7.72g of the product. (yield: 65%).

### Synthesis example of 1-45

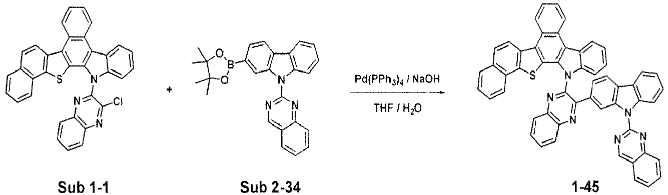

Sub 1-1 (11g, 20.52mmol), THF(90ml), Sub 2-34 (8.65g, 20.52mmol), Pd(PPh₃)₄(0.95g, 0.82mmol), NaOH (2.46g, 61.56mmol), water (45ml) were carried out in the same manner as described above for the synthesis of the compound 1-1 to obtain 9.95g of the product (yield: 61%).

### Synthesis example of 2-7

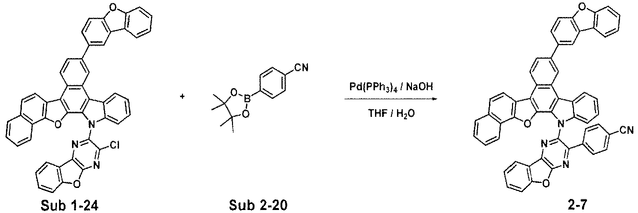

Sub 1-24 (11g, 15.15mmol), THF(66ml), Sub 2-20 (3.47g, 15.15mmol), Pd(PPh₃)₄(0.7g, 0.61mmol), NaOH (1.82g, 45.44mmol), water (33ml) were carried out in the same manner as described above for the synthesis of the compound 1-1 to obtain 7.69g of the product (yield: 64%).

### Synthesis example of 2-9

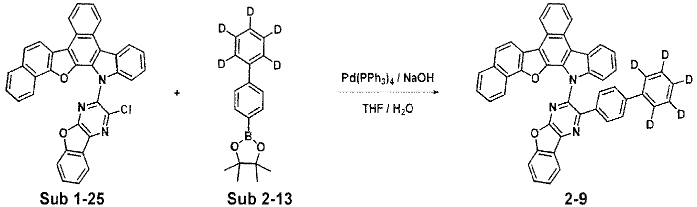

Sub 1-25 (11g, 19.64mmol), THF(86ml), Sub 2-13 (5.6g, 19.64mmol), Pd(PPh₃)₄ (0.91g, 0.79mmol), NaOH (2.36g, 58.93mmol), water (43ml) were carried out in the same manner as described above for the synthesis of the compound 1-1 to obtain 9.92g of the product (yield: 74%).

### Synthesis example of 3-16

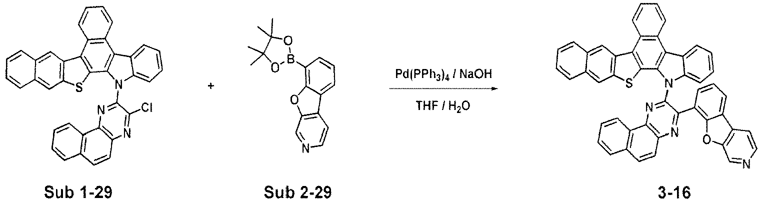

Sub 1-29 (11g, 18.77mmol), THF(82ml), Sub 2-29 (5.54g, 18.77mmol), Pd(PPh₃)₄ (0.87g, 0.75mmol), NaOH (2.25g, 56.30mmol), water (41ml) were carried out in the same manner as described above for the synthesis of the compound 1-1 to obtain 8.5g of the product (yield: 63%).

### Synthesis example of 4-3

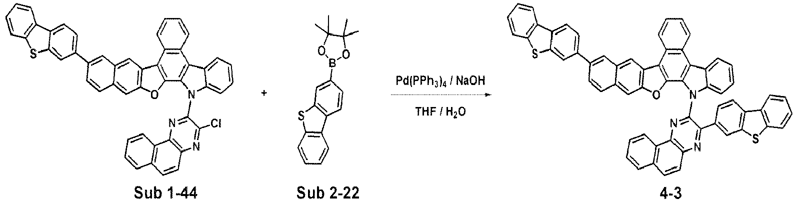

Sub 1-44 (11g, 14.62mmol), THF(64ml), Sub 2-22 (4.54g, 14.62mmol), Pd(PPh₃)₄ (0.68g, 0.58mmol), NaOH (1.75g, 43.87mmol), water (32ml) were carried out in the same manner as described above for the synthesis of the compound 1-1 to obtain 8.03g of the product (yield: 61%).

### Synthesis example of 5-9

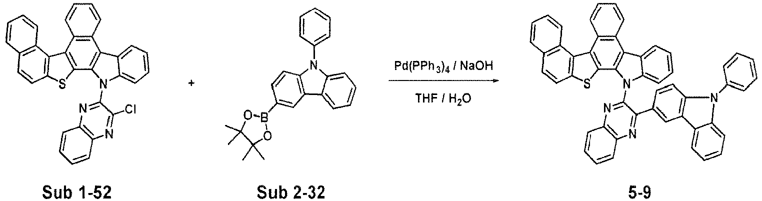

Sub 1-52 (11g, 20.52mmol), THF(90ml), Sub 2-32 (7.58g, 20.52mmol), Pd(PPh₃)₄ (0.95g, 0.82mmol), NaOH (2.46g, 61.56mmol), water (45ml) were carried out in the same manner as described above for the synthesis of the compound 1-1 to obtain 10.98g of the product (yield: 72%).

### Synthesis example of 5-25

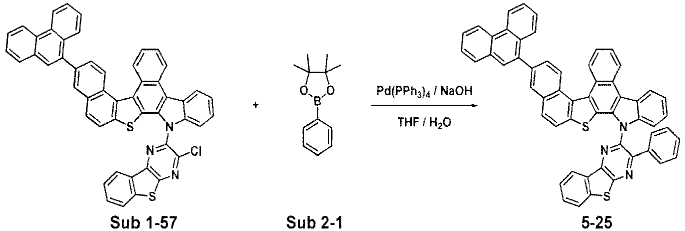

Sub 1-57 (11g, 14.32mmol), THF(63ml), Sub 2-1 (2.92g, 14.32mmol), Pd(PPh₃)₄(0.66g, 0.57mmol), NaOH (1.72g, 42.95mmol), water (31ml) were carried out in the same manner as described above for the synthesis of the compound 1-1 to obtain 8.35g of the product (yield: 72%).

### Synthesis example of 5-41

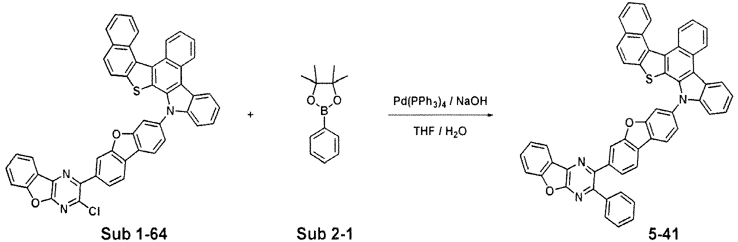

Sub 1-64 (11g, 14.82mmol), THF(65ml), Sub 2-1 (3.02g, 14.82mmol), Pd(PPh₃)₄ (0.69g, 0.59mmol), NaOH (1.78g, 44.46mmol), water (32ml) were carried out in the same manner as described above for the synthesis of the compound 1-1 to obtain 8.83g of the product (yield: 76%).

### Synthesis example of 6-15

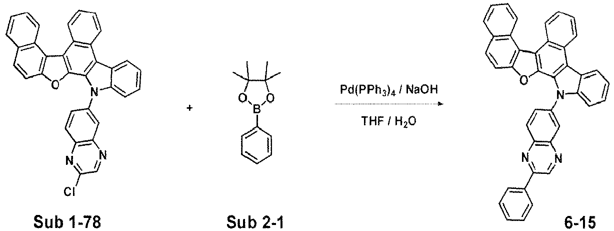

Sub 1-78 (11g, 21.15mmol), THF(93ml), Sub 2-1 (4.32g, 21.15mmol), Pd(PPh₃)₄(0.98g, 0.85mmol), NaOH (2.54g, 63.46mmol), water (46ml) were carried out in the same manner as described above for the synthesis of the compound 1-1 to obtain 9.27g of the product (yield: 78%).

**[Table 3]**

| Compound | FD-MS | Compound | FD-MS |
|---|---|---|---|
| 1-1 | m/z=577.16(C₄₀H₂₃N₃S=577.71) | 1-2 | m/z=627.18(C₄₄H₂₅N₃S=627.77) |
| 1-4 | m/z=868.27(C₆₂H₃₆N₄S=869.06) | 1-6 | m/z=683.15(C₂₆H₂₅N₃S₂=683.85) |
| 1-8 | m/z=627.18(C₄₄H₂₅N₃S=627.77) | 1-14 | m/z=868.27(C₆₂H₃₆N₄S=869.06) |
| 1-16 | m/z=633.13(C₄₂H₂₃N₃S₂=633.79) | 1-26 | m/z=683.15(C₂₆H₂₅N₃S₂=683.85) |
| 1-30 | m/z=777.17(C₅₂H₂₈FN₃S₂=777.94) | 1-34 | m/z=667.17(C₄₆H₂₅N₃OS=667.79) |
| 1-39 | m/z=693.19(C₄₈H₂₇N₃-OS=693.82) | 1-44 | m/z=578.16(C₃₉H₂₂N₄S=578.69) |
| 1-45 | m/z=794.23(C₅₄H₃₀N₆S=794.94) | 2-1 | m/z=561.18(C₄₀H₂₃N₃O=561.64) |
| 2-4 | m/z=707.17(C₄₈H₂₅N₃-O₂S=707.81) | 2-5 | m/z=743.20(C₅₂H₂₉N₃-OS=743.88) |
| 2-7 | m/z=792.22(C₅₅H₂₈N₄-O₃=792.85) | 2-9 | m/z=682.24(C₄₈H₂₂D₅N₃O₂=682.79) |
| 3-1 | m/z=577.16(C₄₀H₂₃N₃S=577.71) | 3-2 | m/z=627.18(C₄₄H₂₅N₃S=627.77) |
| 3-4 | m/z=653.19(C₄₆H₂₇N₃S=653.80) | 3-15 | m/z=733.16(C₅₀H₂₇N₃S₂=733.91) |
| 3-16 | m/z=718.18(C₄₉H₂₆N₄OS=718.83) | 3-19 | m/z=683.15(C₂₆H₂₅N₃S₂=683.85) |
| 3-36 | m/z=693.19(C₄₈H₂₇N₃-OS=693.82) | 3-44 | m/z=717.19(C₅₀H₂₇N₃-OS=717.85) |
| 3-45 | m/z=799.18(C₅₄H₂₉N₃-OS₂=799.97) | 4-1 | m/z=561.18(C₄₀H₂₃N₃O=561.64) |
| 4-2 | m/z=687.23(C₅₀H₂₉N₃O=687.80) | 4-3 | m/z=899.21(C₆₂H₃₃N₃OS₂=900.09) |
| 4-9 | m/z=677.21(C₄₈H₂₇N₃O₂=677.76) | 5-1 | m/z=577.16(C₄₀H₂₃N₃S=577.71) |
| 5-2 | m/z=627.18(C₄₄H₂₅N₃S=627.77) | 5-7 | m/z=632.21(C₄₄H₂₀D₅N₃S=632.80) |
| 5-8 | m/z=677.19(C₄₈H₂₇N₃S=677.83) | 5-9 | m/z=742.22(C₅₂H₃₀N₄S=742.90) |
| 5-11 | m/z=818.25(C₅₈H₃₄N₄S=819.00) | 5-24 | m/z=723.14(C₄₈H₂₅N₃OS₂=723.87) |
| 5-25 | m/z=809.20(C₅₆H₃₁N₃S₂=810.01) | 5-26 | m/z=709.16(C₄₈H₂₇N₃S₂=709.89) |
| 5-31 | m/z=683.15(C₂₆H₂₅N₃S₂=683.85) | 5-41 | m/z=783.20(C₅₄H₂₇₉N₃O₂S=783.91) |
| 5-42 | m/z=617.16(C₄₂H₂₃N₃OS=617.73) | 5-44 | m/z=733.22(C₅₁H₃₁N₃OS=733.89) |
| 6-1 | m/z=611.20(C₄₄H₂₅N₃O=611.70) | 6-2 | m/z=651.19(C₄₆H₂₅N₃O₂=651.73) |
| 6-3 | m/z=743.20(C₅₂H₂₉N₃OS=743.88) | 6-5 | m/z=713.25(C₅₂H₃₁N₃O=713.84) |
| 6-7 | m/z=737.25(C₅₄H₃₁N₃O=737.86) | 6-12 | m/z=918.30(C₆₆H₃₈N₄O₂=919.06) |
| 6-15 | m/z=561.18(C₄₀H₂₃N₃O=561.64) | | |

In the above, even though an exemplary synthesis example of the present invention represented by the Formula 1 are described, all of them are based on Buchwald-Hartwig cross coupling reaction, Miyaura boration reaction, Suzuki cross-coupling reaction, Intramolecular acid-induccd cyclization reaction (J. mater. Chem. 1999, 9, 2095.), Pd(II)-catalyzed oxidative cyclization reaction (Org. Lett. 2011, 13, 5504), Grignard reaction, Cyclic Dehydration reaction and PPh₃-mediated reductive cyclization reaction (J. Org. Chem. 2005, 70, 5014.). Therefore, it will be understood by those skilled in the art that the above reaction proceeds even when other substituents (substituents of R¹, R², R³, R⁴, L¹, L², Ar¹ and the like) defined in Formula 1 are bonded, in addition to the substituents described in the specific synthesis example.

### Fabrication and Evaluation of Organic Electronic Element

### [Example 1] Red OLED (Phosphorescent host)

Organic light emitting diodes (OLEDs) were fabricated according to a conventional method by using a compound obtained by synthesis as host material of a light emitting.

First, an ITO layer (anode) was formed on a glass substrate, and a film of N¹-(naphthalen-2-yl)-N⁴,N⁴-bis(4-(naphthalen-2-yl(phenyl)amino)phenyl)-N¹-phenylbenzene-1,4-diamine (hereinafter abbreviated as "2-TNATA") was vacuum-deposited on the ITO(anode) layer to form a hole injection layer with a thickness of 60nm. Then, 4,4-bis[N-(1-naphthyl)-N-phenylamino]biphenyl (hereinafter abbreviated as "NPD") was vacuum-deposited on the hole injection layer to form a hole transfer layer with a thickness of 60nm.

Subsequently, a light emitting layer with a thickness of 30nm was deposited on the hole transport layer by doping the hole transport layer with the compound 1-1 of the present invention as host material and bis-(1-phenylisoquinolyl)iridium(III)acetylacetonate (hereinafter abbreviated as "(piq)₂Ir(acac)") as a dopant material in a weight ratio of 95:5.

Next, a film of ((1,1'-bisphenyl)-4-olato)bis(2-methyl-8-quinolinolato)aluminum (hereinafter abbreviated as "BAlq") was vacuum-deposited with a thickness of 5nm on the light emitting layer to form a hole blocking layer, and a film of Bis(10-hydroxybenzo[h]quinolinato)beryllium (hereinafter abbreviated as "BeBq₂") was formed with a thickness of 40nm to form an electron transport layer.

Next, LiF as halogenated alkali metal was deposited with a thickness of 0.2nm on the electron transport layer to form an electron injection layer, and then Al was deposited with a thickness of 150nm on the electron injection layer to form a cathode.

### [Example 2] to [Example 44]

OLEDs were manufactured in the same manner as described in Example 1, except that any one of the compounds of the present invention in the Table 4 below was used as host material of the light emitting layer, instead of the inventive compound 1-1.

### [Comparative Example 1] to [Comparative Example 4]

OLEDs were manufactured in the same manner as described in Example 1, except that any one of comparative compounds 1 to 4 was used as host material of the light emitting layer, instead of the inventive compound 1-1.

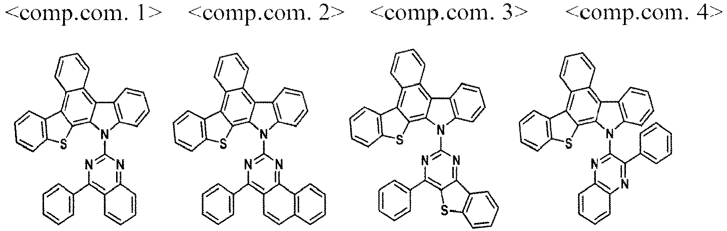

A forward bias DC voltage was applied to each of the OLEDs manufactured through the Examples 1 to 44 and Comparative Examples 1 to 4, and electro-luminescence (EL) characteristics of the OLED were measured by PR-650 (Photo research). T95 life span was measured by life span measuring equipment (Mc science) at reference brightness of 2500cd/m². Table 4 below shows results of fabrication and evaluation of OLED.

**[Table 4]**

| | compound | Voltage (V) | Current Density (mA/cm²) | Brightness (cd/m²) | Efficiency (cd/A) | T(95) | CIE | |
|---|---|---|---|---|---|---|---|---|
| | | | | | | | X | Y |
| comp.Ex(1) | comp.Com 1 | 6.1 | 26.9 | 2500 | 10.3 | 112.1 | 0.65 | 0.32 |
| comp.Ex(2) | comp.Com 2 | 6.0 | 24.0 | 2500 | 13.8 | 110.8 | 0.66 | 0.33 |
| comp.Ex(3) | comp.Com 3 | 5.8 | 22.9 | 2500 | 11.4 | 108.3 | 0.65 | 0.33 |
| comp.Ex(4) | comp.Com 4 | 5.6 | 18.5 | 2500 | 18.2 | 119.7 | 0.66 | 0.32 |
| Ex.(1) | Com. 1-1 | 5.2 | 14.6 | 2500 | 26.1 | 162.7 | 0.68 | 0.33 |
| Ex.(2) | Com. 1-2 | 5.2 | 14.5 | 2500 | 26.2 | 163.1 | 0.68 | 0.33 |
| Ex.(3) | Com. 1-4 | 5.2 | 14.2 | 2500 | 26.6 | 162.2 | 0.68 | 0.33 |
| Ex.(4) | Com. 1-6 | 5.2 | 14.4 | 2500 | 26.3 | 160.2 | 0.68 | 0.32 |
| Ex.(5) | Com. 1-8 | 5.2 | 14.1 | 2500 | 26.7 | 158.1 | 0.68 | 0.33 |
| Ex.(6) | Com. 1-16 | 5.1 | 14.1 | 2500 | 26.7 | 158.4 | 0.68 | 0.33 |
| Ex.(7) | Com. 1-26 | 5.1 | 14.0 | 2500 | 26.9 | 159.0 | 0.68 | 0.33 |
| Ex.(8) | Com. 1-34 | 5.2 | 13.2 | 2500 | 28.0 | 158.8 | 0.68 | 0.32 |
| Ex.(9) | Com. 1-39 | 5.2 | 13.6 | 2500 | 27.4 | 158.4 | 0.68 | 0.32 |
| Ex.(10) | Com. 1-44 | 5.2 | 14.2 | 2500 | 26.6 | 158.2 | 0.68 | 0.33 |
| Ex.(11) | Com. 5-1 | 5.2 | 13.9 | 2500 | 27.0 | 157.7 | 0.68 | 0.32 |
| Ex.(12) | Com. 5-2 | 5.2 | 14.1 | 2500 | 26.7 | 156.5 | 0.68 | 0.32 |
| Ex.(13) | Com. 5-7 | 5.2 | 14.4 | 2500 | 26.3 | 156.3 | 0.68 | 0.32 |
| Ex.(14) | Com. 5-8 | 5.2 | 14.2 | 2500 | 26.6 | 154.1 | 0.68 | 0.33 |
| Ex.(15) | Com. 5-9 | 5.2 | 13.8 | 2500 | 27.1 | 153.4 | 0.68 | 0.33 |
| Ex.(16) | Com. 5-11 | 5.2 | 14.5 | 2500 | 26.2 | 152.8 | 0.68 | 0.33 |
| Ex.(17) | Com. 5-24 | 5.1 | 14.7 | 2500 | 26.0 | 152.7 | 0.68 | 0.33 |
| Ex.(18) | Com. 5-26 | 5.1 | 14.4 | 2500 | 26.4 | 151.9 | 0.68 | 0.33 |
| Ex.(19) | Com. 5-31 | 5.1 | 14.2 | 2500 | 26.7 | 151.5 | 0.68 | 0.33 |
| Ex.(20) | Com. 5-42 | 5.2 | 13.7 | 2500 | 27.3 | 151.4 | 0.68 | 0.33 |
| Ex.(21) | Com. 5-44 | 5.2 | 14.0 | 2500 | 26.8 | 149.2 | 0.68 | 0.33 |
| Ex.(22) | Com. 3-1 | 5.3 | 14.2 | 2500 | 26.6 | 153.0 | 0.68 | 0.33 |
| Ex.(23) | Com. 3-2 | 5.3 | 14.6 | 2500 | 26.1 | 150.8 | 0.68 | 0.33 |
| Ex.(24) | Com. 3-4 | 5.3 | 14.4 | 2500 | 26.4 | 151.4 | 0.68 | 0.33 |
| Ex.(25) | Com. 3-15 | 5.3 | 14.5 | 2500 | 26.3 | 149.5 | 0.68 | 0.32 |
| Ex.(26) | Com. 3-19 | 5.2 | 14.2 | 2500 | 26.6 | 143.8 | 0.68 | 0.32 |
| Ex.(27) | Com. 3-36 | 5.3 | 14.0 | 2500 | 26.9 | 144.2 | 0.68 | 0.32 |
| Ex.(28) | Com. 3-44 | 5.3 | 14.1 | 2500 | 26.7 | 139.2 | 0.68 | 0.33 |
| Ex.(29) | Com. 3-45 | 5.3 | 14.0 | 2500 | 26.9 | 141.7 | 0.68 | 0.33 |
| Ex.(30) | Com. 2-1 | 5.3 | 14.6 | 2500 | 26.1 | 145.9 | 0.68 | 0.33 |
| Ex.(31) | Com. 2-4 | 5.2 | 14.4 | 2500 | 26.4 | 140.2 | 0.68 | 0.32 |
| Ex.(32) | Com. 2-5 | 5.2 | 14.3 | 2500 | 26.5 | 139.4 | 0.68 | 0.33 |
| Ex.(33) | Com. 2-9 | 5.3 | 14.0 | 2500 | 26.9 | 139.9 | 0.68 | 0.33 |
| Ex.(34) | Com. 6-1 | 5.4 | 14.5 | 2500 | 26.2 | 138.8 | 0.68 | 0.33 |
| Ex.(35) | Com. 6-2 | 5.4 | 14.5 | 2500 | 26.3 | 133.1 | 0.68 | 0.32 |
| Ex.(36) | Com. 6-3 | 5.4 | 14.6 | 2500 | 26.1 | 134.2 | 0.68 | 0.32 |
| Ex.(37) | Com. 6-5 | 5.4 | 14.4 | 2500 | 26.4 | 131.6 | 0.68 | 41.33 |
| Ex.(38) | Com. 6-7 | 5.3 | 14.7 | 2500 | 26.0 | 135.3 | 0.68 | 0.33 |
| Ex.(39) | Com. 6-9 | 5.2 | 14.8 | 2500 | 25.9 | 133.5 | 0.68 | 0.33 |
| Ex.(40) | Com. 6-12 | 5.4 | 14.5 | 2500 | 26.3 | 131.4 | 0.68 | 0.32 |
| Ex.(41) | Com. 6-15 | 5.6 | 15.0 | 2500 | 25.7 | 129.2 | 0.68 | 0.33 |
| Ex.(42) | Com. 4-1 | 5.5 | 15.5 | 2500 | 25.1 | 133.1 | 0.68 | 0.33 |
| Ex.(43) | Com. 4-2 | 5.5 | 16.4 | 2500 | 24.2 | 130.5 | 0.68 | 0.33 |
| Ex.(44) | Com. 4-9 | 5.5 | 14.8 | 2500 | 25.9 | 130.2 | 0.68 | 0.32 |

As can be seen from the results of Table 4, it was confirmed that in case of Examples 1 to 44 using compound according to one embodiment of the present invention as a phosphorescent host, the driving voltage, luminescent efficiency, life span and color purity were significantly improved as compared to Comparative Examples 1 to 4.

At present, the present inventors are studying to lower power consumption, increase efficiency and color purity, and a sub-substituent having excellent electron mobility is required. Therefore, the fused pyrazine type substituent which has better electron transfer properties than the substituents of the fused pyrimidine type used in conventional phosphorescent red host.was introduced. In fact, it was confirmed that the introduction of a specific substituent (fused pyrazine type) such as comparative compound 4 is excellent in the electron mobility and thus the efficiency is increased and the driving voltage is lowered.

**[Table 5]**

| **Fused pyrazine type (The present invention)** | **Fused pyrimidine** |
|---|---|
| 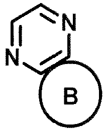 | 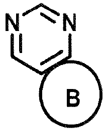 |
| Quinoxaline, benzoquinoxaline, dibenzoquinoxaline, benzothienopyrazine, benzofuropyrazine, etc. | Quinazoline, benzoquinazoline, dibenzoquinazoline, benzothienopyrimidine, benzofuropyrimidine, etc. |
| (The definition of the B ring is the same as the B ring of claim 1) | |

Comparing Comparative Examples 1 to 3 with Comparative Example 4, it was confirmed that Comparative Example 4 having quinoxaline(a fused pyrazine) as a sub substituent showed the improved efficiency and remarkably improved driving voltage, compared with Comparative Compounds 1 to 3 in which fused pyrimidine was bonded as a sub substituent having a different N substitution position. It can be explained that the energy band gap is changed and electron mobility becomes high due to the binding of specific substituents even if the core is the same.

That is, in the case of the compound of the present invention in which fused pyrazine is introduced as a sub-substituent instead of fused pyrimidine, electron injection from the ETL into a light emitting layer becomes easier as the LUMO level becomes lower and the charge balance in a light emitting layer is improved, and thus it is considered that the driving voltage and the lifetime are improved.

In addition, it seems that efficiency is improved because the conjugation length becomes longer and the charge transfer to the dopant becomes easy as benzene being more fused at a specific position of the 6-ring heterocyclic core. It can be confirmed that the PL wavelength is red shifted when a benzene ring is formed at a specific position of the core (Comparison of PL data of Comparative Compound 4 and the inventive Compound 1-1: long wavelength from 527 nm to 555 nm). It can be confirmed that the efficiency and color purity are improved because the charge transfer to the dopant is facilitated by making the wavelength of the host longer wavelength.

It is considered that as a result, HOMO / LUMO, T1 value and energy band gap are optimized so that the charge transfer from the host to the dopant can be smoothly performed as the effect of the fused pyrazine type substituent(high charge carrier mobility, improved driving voltage) and the formation of additional benzene rings at specific positions of the core (7 ring), thereby improving the performance of the device as a whole.

**[Table 6]**

| <PL data of comparative compounds 1 to 4 and compound 1-1 of the present invention> | | | | | |
|---|---|---|---|---|---|
| | **comp.Com 1** | **comp.Com 2** | **comp.Com 3** | **comp.Com 4** | **The inventive Com. 1-1** |
| **PL (nm)** | **515** | **497** | **507** | **527** | **555** |

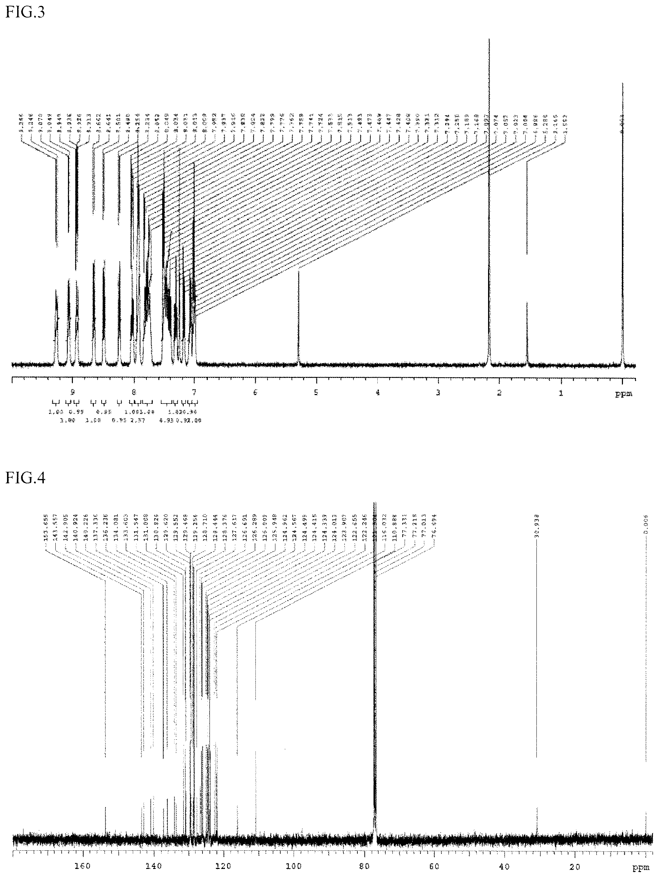

### Refer to Fig.4.

Although exemplary embodiments of the present invention have been described for illustrative purposes, those skilled in the art will appreciate that various modifications, additions and substitutions are possible, without departing from the scope of the invention as disclosed in the accompanying claims. Therefore, the embodiment disclosed in the present invention is intended to illustrate the scope of the technical idea of the present invention, and the scope of the present invention is not limited by the embodiment. The scope of the present invention shall be construed on the basis of the accompanying claims.

## Claims

1. A compound of Formula 1 below: 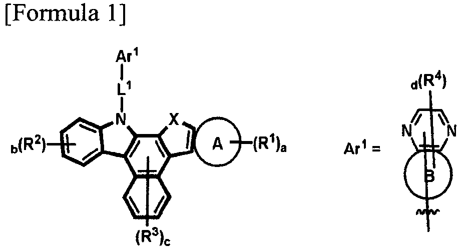 wherein,
1) A ring is C₁₀ aryl group,
2) B ring is selected from the group consisting of the following formulas B-1 to B-16: 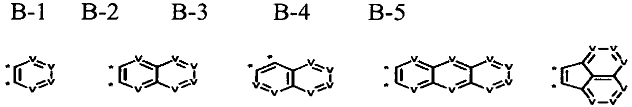 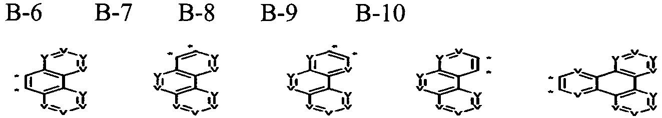 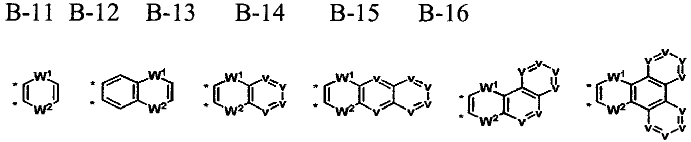 in formulas B-1 to B-16, "*" indicates the position to be condensed with pyrazine comprising two Ns,
3) W¹ and W² are each independently a single bond, S or O,
4) V is N or C,
5) X is O or S,
6) a is an integer of 0 to 6, b and c are each an integer of 0 to 4, d is an integer of 0 to 11,
7) R¹, R² and R³ are the same or different from each other, and are each independently selected from the group consisting of hydrogen, deuterium, halogen, a cyano group, a nitro group, a C₆-C₆₀ aryl group, a fluorenyl group, a C₂-C₆₀ heterocyclic group containing at least one heteroatom selected from the group consisting of O, N, S, Si, and P, a fused ring group of a C₃-C₆₀ aliphatic ring and a C₆-C₆₀ aromatic ring, a C₁-C₅₀ alkyl group, a C₂-C₂₀ alkenyl group, a C₂-C₂₀ alkynyl group, a C₁-C₃₀ alkoxyl group, a C₆-C₃₀ aryloxyl group and -L'-N(R^{a})(R^{b}),
or when a, b and c are 2 or more, R¹, R² and R³ are each in plural and are the same or different, and a plurity of R¹, a plurity of R², or a plurity of R³ may be bonded to each other to form a ring, and
R⁴ is represented by any one of the following formulas R-1 to R-10: 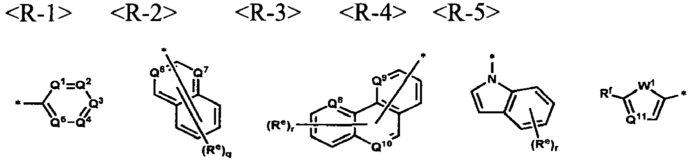 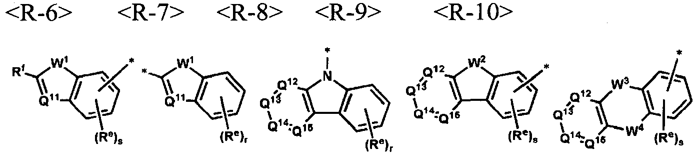 in Formulas R-1 to R-10,
1) Q¹ to Q¹⁵ are each independently CR^{g} or N,
2) W¹ is S, O or NR^{h},
3) W² to W⁴ are each independently S, O, NR^{h} or CRⁱR^{j},
4) R^{e} is selected from the group consisting of hydrogen, deuterium, halogen, a silane group substituted or unsubstituted with a C₁-C₂₀ alkyl group or a C₆-C₂₀ aryl group, a siloxane group, a boron group, a germanium group, a cyano group, a nitro group, a C₁-C₂₀ alkylthio group, a C₁-C₂₀ alkoxyl group, a C₁-C₂₀ alkyl group, a C₂-C₂₀ alkenyl group, a C₂-C₂₀ alkynyl group, a C₆-C₂₀ aryl group, a C₆-C₂₀ aryl group substituted with deuterium, a fluorenyl group, a C₂-C₂₀ heterocyclic group containing at least one heteroatom selected from the group consisting of O, N, S, Si, and P, a C₃-C₂₀ cycloalkyl group, a C₇-C₂₀ arylalkyl group, and a C₈-C₂₀ arylalkenyl group, and when these substituents are adjacent, they may be linked each other to form a ring,
5) R^{f} and R^{g} are each independently selected from the group consisting of hydrogen, deuterium, a C₆-C₂₀ aryl group, a fluorenyl group, a fused ring group of a C₃-C₂₀ aliphatic ring and a C₆-C₂₀ aromatic ring, a C₂-C₂₀ heterocyclic group containing at least one heteroatom selected from O, N, S, Si, and P, a C₁-C₂₀ alkyl group, a C₂-C₂₀ alkenyl group, a C₂-C₂₀ alkynyl group, and a C₁-C₃₀ alkoxyl group,
6) R^{h}, Rⁱ and R^{j} are each independently selected from the group consisting of a C₆-C₂₀ aryl group, a C₂-C₂₀ heterocyclic group containing at least one heteroatom selected from O, N, S, Si, and P, a C₁-C₂₀ alkyl group, a C₂-C₂₀ alkenyl group, a C₁-C₂₀ alkoxyl group and a fluorenyl group, Rⁱ and R^{j} may be linked each other to form a spiro compound together with C to which they are bonded,
7) q is each independently an integer of 0 to 5,
8) r is each independently an integer of 0 to 4,
9) s is each independently an integer of 0 to 3,
when q, r and s are each 2 or more, R^{e} is each the same or different, and * indicates the position to be bonded,
8) L' is selected from the group consisting of a single bond, a C₆-C₆₀ arylene group, a fluorenylene group, a fused ring group of a C₃-C₆₀ aliphatic ring and a C₆-C₆₀ aromatic ring, and a C₂-C₆₀ heterocyclic group, and R^{a} and R^{b} are each independently selected from the group consisting of a C₆-C₆₀ aryl group, a fluorenyl group, a fused ring group of a C₃-C₆₀ aliphatic ring and a C₆-C₆₀ aromatic ring, and a C₂-C₆₀ heterocyclic group containing at least one heteroatom selected from the group consisting of O, N, S, Si, and P,
9) L¹ is each independently selected from the group consisting of a single bond, a C₆-C₆₀ arylene group, a fluorenylene group, a fused ring group of a C₃-C₆₀ aliphatic ring and a C₆-C₆₀ aromatic ring, and a C₂-C₆₀ heterocyclic group, and
the aryl group, fluorenyl group, arylene group, heterocyclic group, fused ring group, alkyl group, alkenyl group, alkoxyl group, and aryloxy group may be each optionally further substituted with one or more substituents selected from the group consisting of deuterium, halogen, a silane group substituted or unsubstituted with a C₁-C₂₀ alkyl group or a C₆-C₂₀ aryl group, a siloxane group, a boron group, a germanium group, a cyano group, a nitro group, -L'-N(R^{a})(R^{b}), a C₁-C₂₀ alkylthio group, a C₁-C₂₀ alkoxyl group, a C₁-C₂₀ alkyl group, a C₂-C₂₀ alkenyl group, a C₂-C₂₀ alkynyl group, a C₆-C₂₀ aryl group, a C₆-C₂₀ aryl group substituted with deuterium, a fluorenyl group, a C₂-C₂₀ heterocyclic group, a C₃-C₂₀ cycloalkyl group, a C₇-C₂₀ arylalkyl group, and a C₈-C₂₀ arylalkenyl group, and these substituents may be linked each other to form a ring, wherein 'ring' is a C₃-C₆₀ aliphatic ring, a C₆-C₆₀ aromatic ring, a C₂-C₆₀ heterocyclic group or a fused ring formed by the combination of them, which includes a saturated or unsaturated ring.

2. The compound of claim 1, wherein Formula 1 above is represented by any one of Formulas 2 to 4 below: 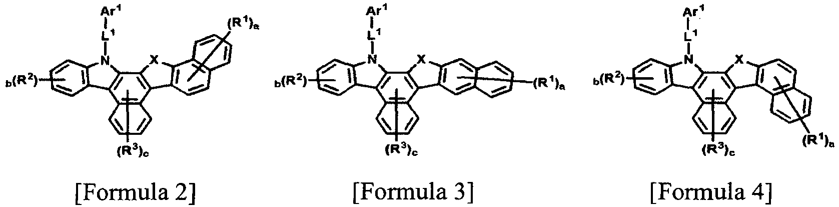 in Formulas 2 to 4, X, L¹, Ar¹, R¹, R², R³, a, b and c are the same as defined in claim 1.

3. The compound of claim 1, wherein Formula 1 above is represented by any one of Formulas 5 to 7 below: 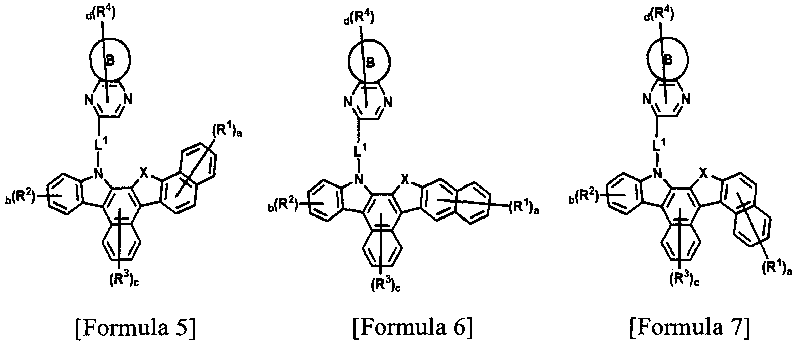 in Formulas 5 to 7, X, L¹, Ar¹, R¹, R², R³, R⁴, a, b, c, d and B ring are the same as defined in claim 1.

4. The compound of claim 1, wherein the chemical structure Ar¹ of the formula 1 comprising the pyrazine is represented by any one of the following Formulas C-1 to C-22: 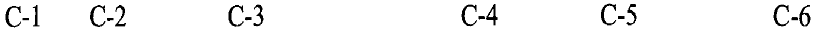 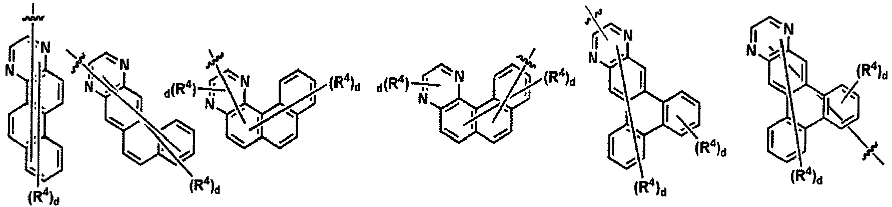 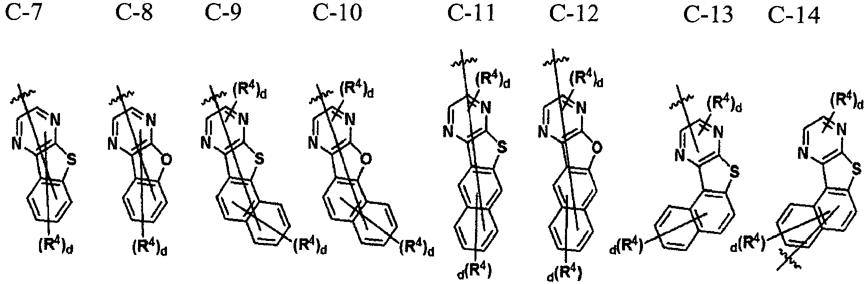 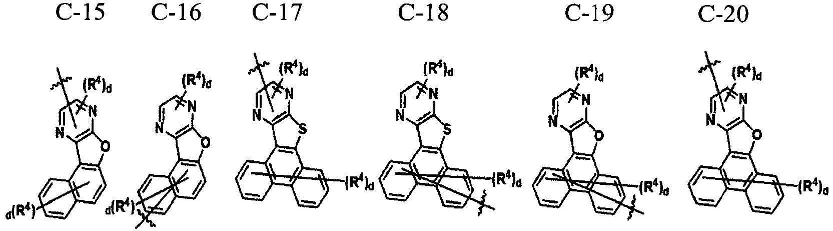 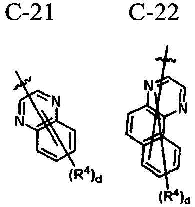 in Formulas C-1 to C-22, R⁴ is the same as defined in claim 1, and d is an integer of 0 to 11.

5. The compound of claim 1, wherein Formula 1 is any one of the compounds below:

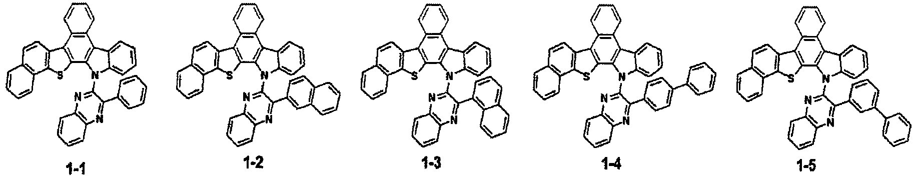

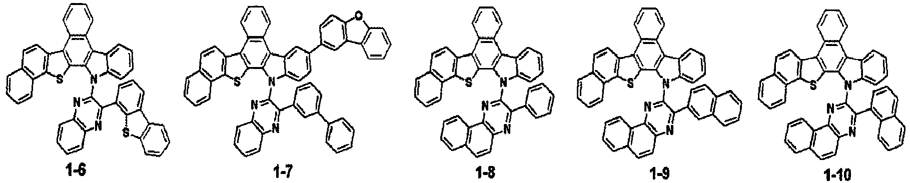

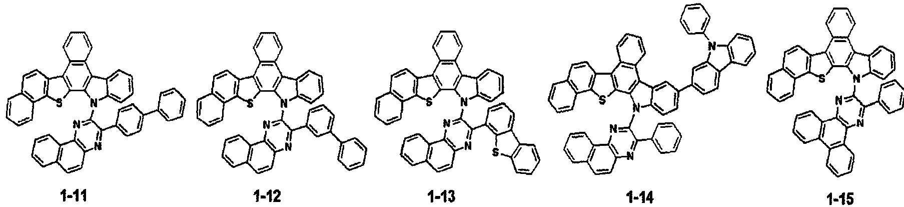

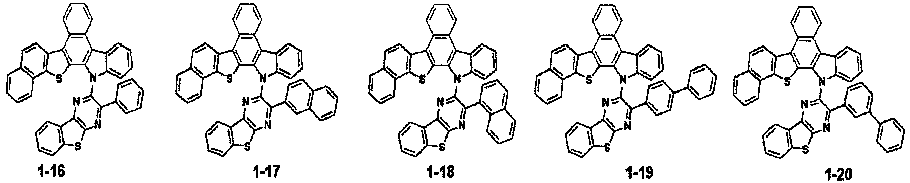

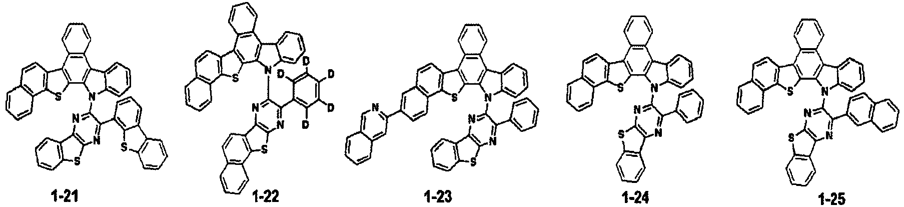

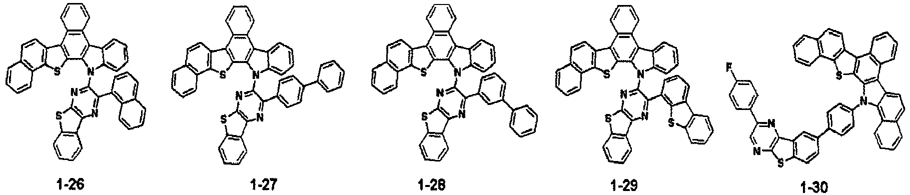

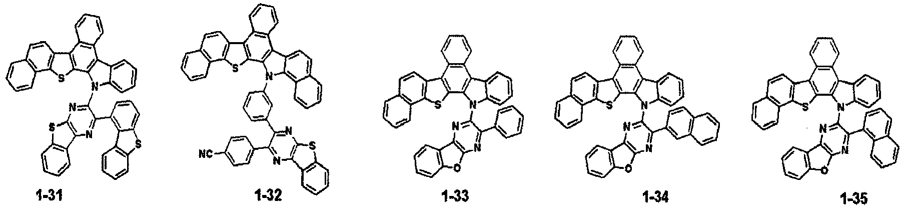

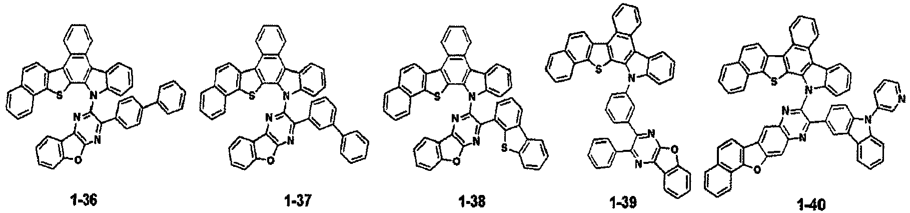

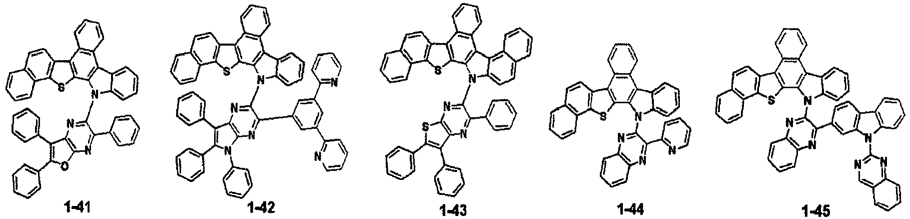

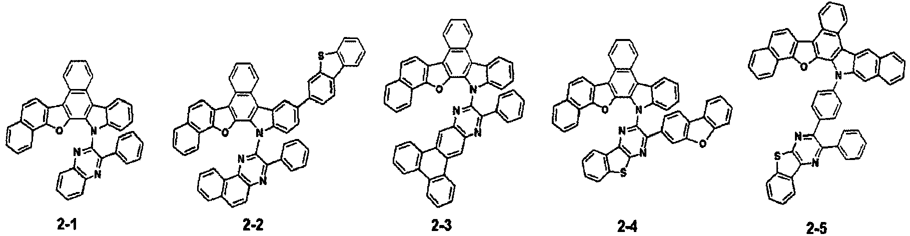

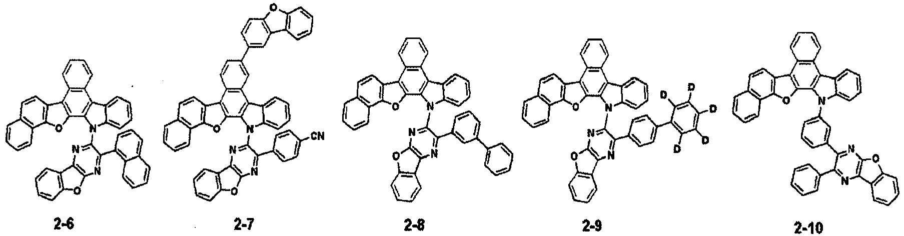

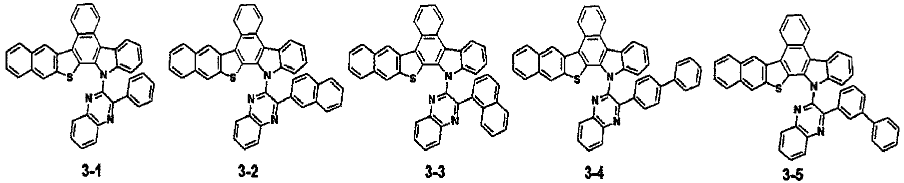

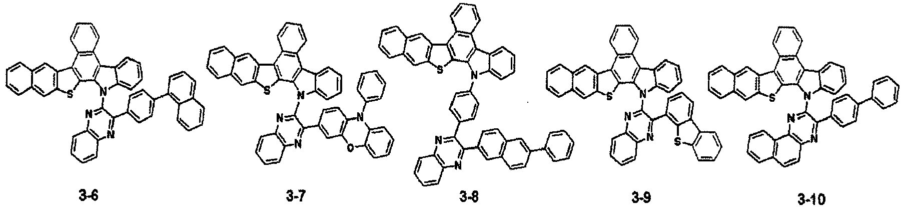

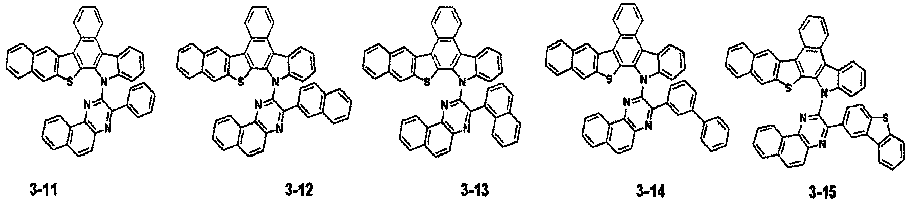

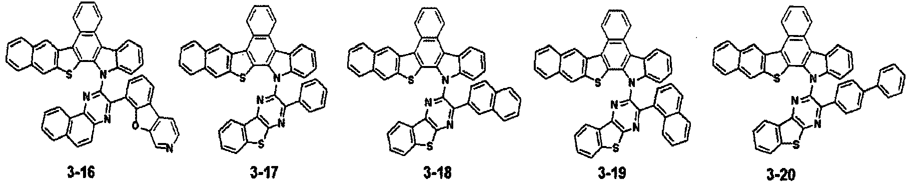

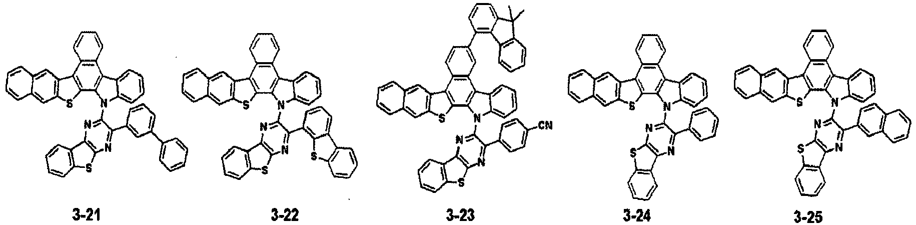

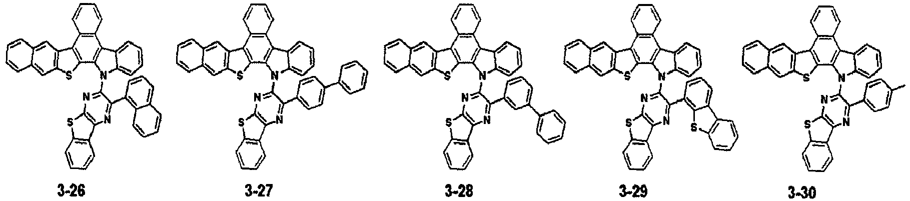

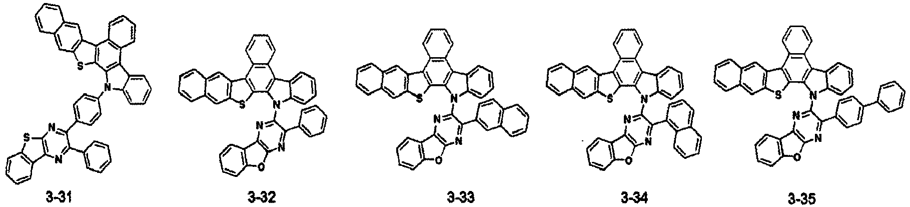

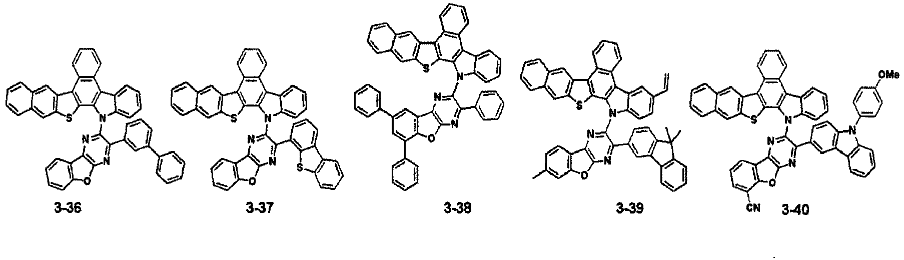

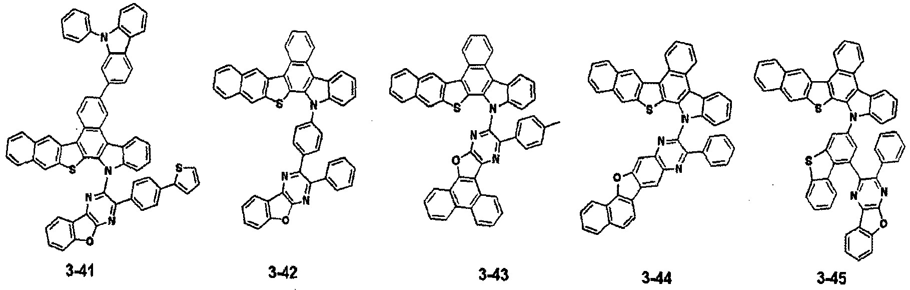

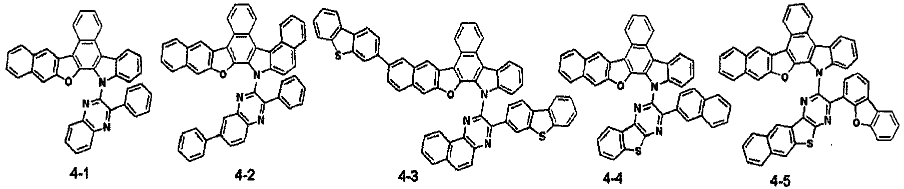

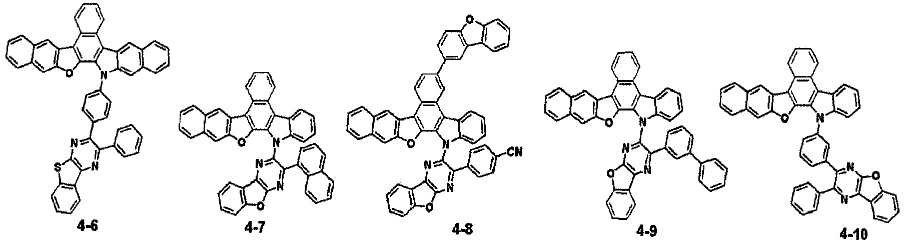

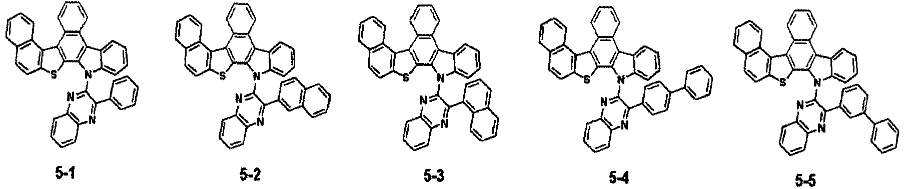

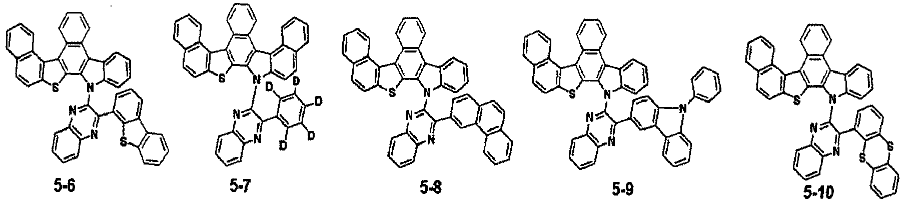

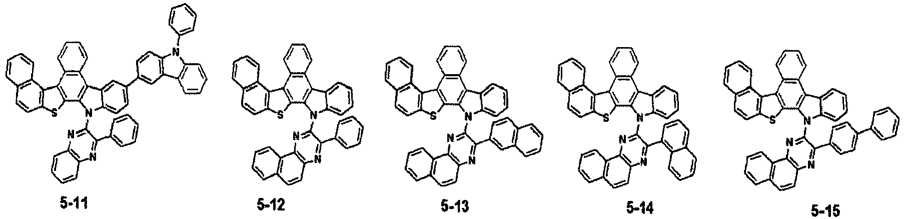

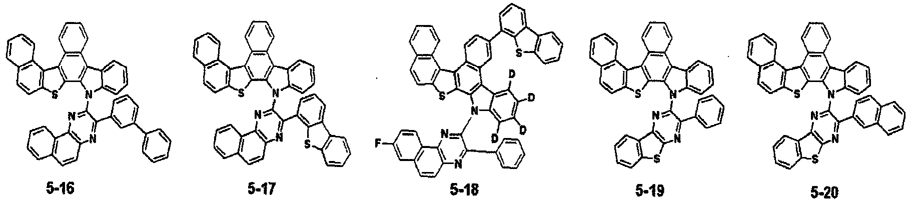

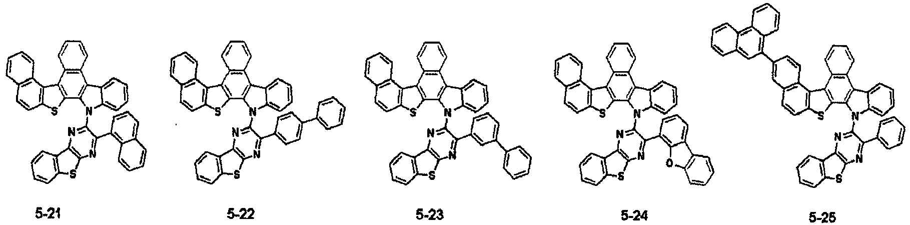

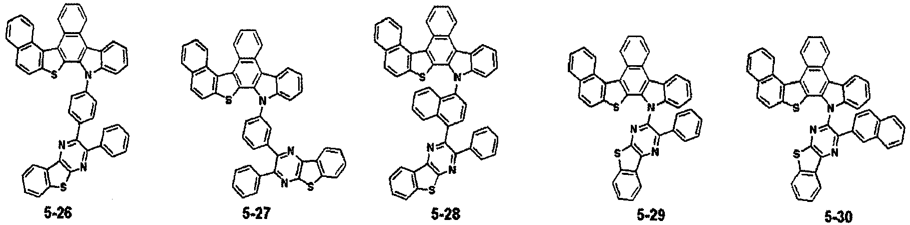

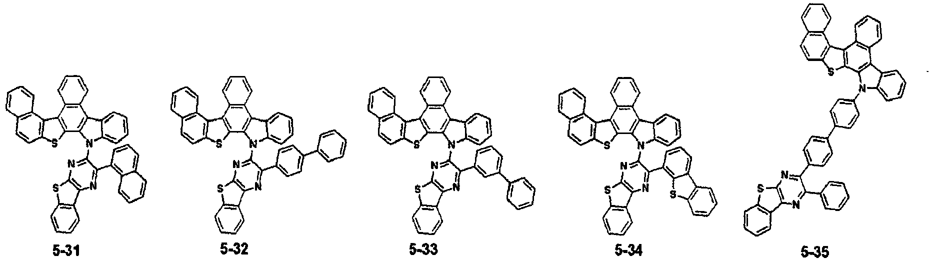

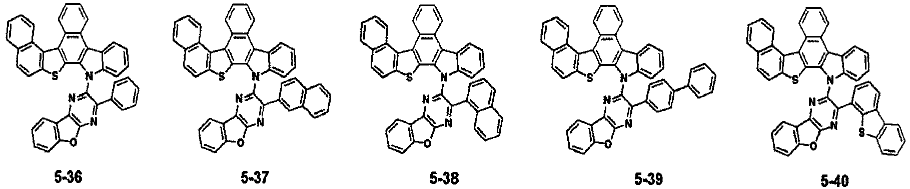

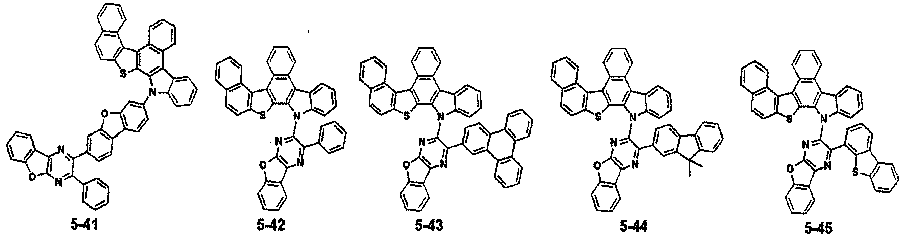

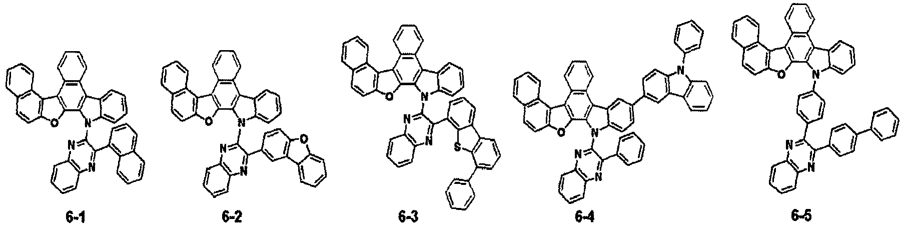

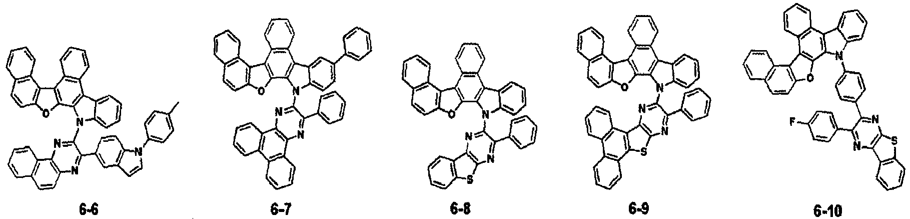

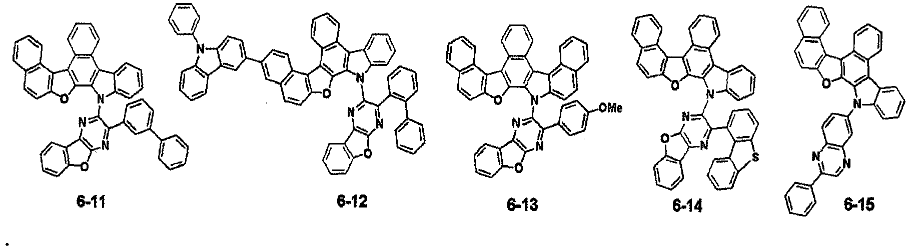

6. An organic electric element comprising a first electrode, a second electrode, and an organic material layer formed between the first electrode and the second electrode, wherein the organic material layer comprises the compound according to any of claims 1 to 5.

7. The organic electric element of claim 6, wherein the compound is comprised in at least one layer of a hole injection layer, a hole transport layer, an emission-auxiliary layer, an electron transport-auxiliary layer, an electron transport layer and an light emitting layer, and the compound is comprised as a single compound or a mixture of two or more different kinds.

8. The organic electric element of claim 6, wherein the compound is used as a phosphorescent host material of the light emitting layer.

9. The organic electric element of claim 6, wherein the organic material layer is formed by any one of the processes of spin coating, nozzle printing, inkjet printing, slot coating, dip coating or roll-to-roll.

10. An electronic device comprising a display device and a control unit for driving the display device, wherein the display device comprises the organic electric element of claim 6.

11. The electronic device of claim 10, wherein the organic electric element is selected from the group consisting of an organic light emitting diode, an organic solar cell, an organic photo conductor, an organic transistor, and an element for monochromatic or white illumination.

## Patentansprüche

1. Eine Verbindung der nachstehenden Formel 1: 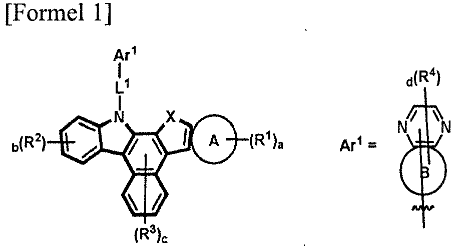 wobei,
1) Ring A eine C₁₀-Arylgruppe ist,
2) Ring B ausgewählt ist aus der Gruppe, bestehend aus den nachstehenden Formeln B-1 bis B-16: 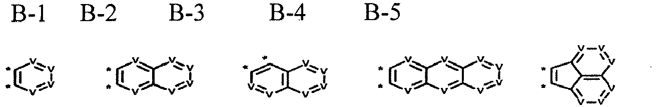 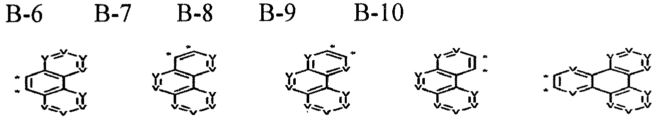 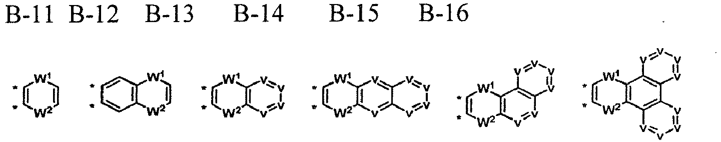 wobei in den Formeln B-1 bis B-16 "*" die Position angibt, die mit Pyrazin, das zwei N umfasst, kondensiert werden soll,
3) W¹ und W² jeweils unabhängig eine Einfachbindung, S oder O sind,
4) V N oder C ist,
5) X O oder S ist,
6) a eine ganze Zahl von 0 bis 6 ist, b und c jeweils eine ganze Zahl von 0 bis 4 sind und d eine ganze Zahl von 0 bis 11 ist,
7) R¹, R² und R³ gleich oder voneinander verschieden sind und jeweils unabhängig ausgewählt sind aus der Gruppe, bestehend aus Wasserstoff, Deuterium, Halogen, einer Cyanogruppe, einer Nitrogruppe, einer C₆-C₆₀-Arylgruppe, einer Fluorenylgruppe, einer heterocyclischen C₂-C₆₀-Gruppe, die mindestens ein Heteroatom enthält, ausgewählt aus der Gruppe bestehend aus O, N, S, Si und P, einer kondensierten Ringgruppe aus einem aliphatischen C₃-C₆₀-Ring und einem aromatischen C₆-C₆₆-Ring, einer C₁-C₅₀-Alkylgruppe, einer C₂-C₂₀-Alkenylgruppe, einer C₂-C₂₀-Alkinylgruppe, einer C₁-C₃₀-Alkoxylgruppe, einer C₆-C₃₀-Aryloxylgruppe und -L'-N(R^{a})(R^{b}),
oder, wenn a, b und c 2 oder mehr sind, R¹, R² und R³ jeweils in der Mehrzahl und gleich oder verschieden sind und eine Mehrzahl von R¹, eine Mehrzahl von R² oder eine Mehrzahl von R³ miteinander verbunden sein kann, um einen Ring zu bilden, und
R⁴ durch eine der nachstehenden Formeln R-1 bis R-10 dargestellt wird: 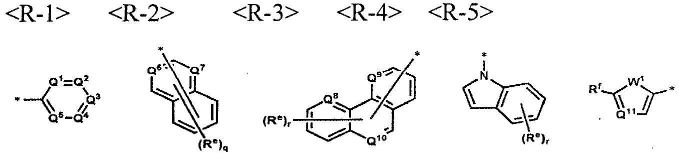 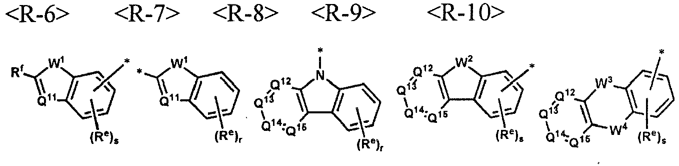 wobei in den Formeln R-1 bis R-10
1) Q¹ bis Q¹⁵ jeweils unabhängig CR^{g} oder N sind,
2) W¹ S, O oder NR^{h} ist,
3) W² bis W⁴ jeweils unabhängig S, O, NR^{h} oder CRⁱR^{j} sind,
4) R^{e} ausgewählt ist aus der Gruppe bestehend aus Wasserstoff, Deuterium, Halogen, einer Silangruppe, substituiert oder unsubstituiert mit einer C₁-C₂₀-Alkylgruppe oder einer C₆-C₂₀-Arylgruppe, einer Siloxangruppe, einer Borgruppe, einer Germaniumgruppe, einer Cyanogruppe, einer Nitrogruppe, einer C₁-C₂₀-Alkylthiogruppe, einer C₁-C₂₀-Alkoxylgruppe, einer C₁-C₂₀-Alkylgruppe, einer C₂-C₂₀-Alkenylgruppe, einer C₂-C₂₀-Alkinylgruppe, einer C₆-C₂₀-Arylgruppe, einer C₆-C₂₀-Arylgruppe, substituiert mit Deuterium, einer Fluorenylgruppe, einer heterocyclischen C₂-C₂₀-Gruppe, die mindestens ein Heteroatom enthält, ausgewählt aus der Gruppe, bestehend aus O, N, S, Si und P, einer C₃-C₂₀-Cycloalkylgruppe, einer C₇-C₂₀-Arylalkylgruppe und einer C₈-C₂₀-Arylalkenylgruppe, und wenn diese Substituenten benachbart sind, können sie miteinander verbunden sein, um einen Ring zu bilden,
5) R^{f} und R^{g} jeweils unabhängig ausgewählt sind aus der Gruppe bestehend aus Wasserstoff, Deuterium, einer C₆-C₂₀-Arylgruppe, einer Fluorenylgruppe, einer kondensierten Ringgruppe eines aliphatischen C3-C2o-Rings und eines aromatischen C₆-C₂₀-Rings, einer heterocyclischen C₂-C₂₀-Gruppe, die mindestens ein Heteroatom enthält, ausgewählt aus O, N, S, Si und P, einer C₁-C₂₀-Alkylgruppe, einer C₂-C₂₀-Alkenylgruppe, einer C₂-C₂₀-Alkinylgruppe und einer C₁-C₃₆-Alkoxylgruppe,
6) R^{h}, Rⁱ und R^{j} jeweils unabhängig ausgewählt sind aus der Gruppe, bestehend aus einer C₆-C₂₀-Arylgruppe, einer heterocyclischen C₂-C₂₀-Gruppe, die mindestens ein Heteroatom enthält, ausgewählt aus O, N, S, Si und P, einer C₁-C₂₀-Alkylgruppe, einer C₂-C₂₀-Alkenylgruppe, einer C₁-C₂₀-Alkoxylgruppe und einer Fluorenylgruppe, wobei Rⁱ und R^{j} miteinander verbunden sein können, um zusammen mit C, an das sie gebunden sind, eine Spiroverbindung zu bilden,
7) q jeweils unabhängig eine ganze Zahl von 0 bis 5 ist,
8) r jeweils unabhängig eine ganze Zahl von 0 bis 4 ist,
9) s jeweils unabhängig eine ganze Zahl von 0 bis 3 ist,
wobei, wenn q, r und s jeweils 2 oder mehr sind, R^{e} jeweils gleich oder verschieden ist und * die zu verbindende Position angibt,
8) L' ausgewählt ist aus der Gruppe bestehend aus einer Einfachbindung, einer C₆-C₆₀-Arylengruppe, einer Fluorenylengruppe, einer kondensierten Ringgruppe aus einem aliphatischen C₃-C₆₀-Ring und einem aromatischen C₆-C₆₀-Ring, und einer heterocyclischen C₂-C₆₀-Gruppe, und R^{a} und R^{b} jeweils unabhängig ausgewählt sind aus der Gruppe, bestehend aus einer C₆-C₆₀-Arylgruppe, einer Fluorenylgruppe, einer kondensierten Ringgruppe aus einem aliphatischen C₃-C₆₀-Ring und einem aromatischen C₆-C₆₀-Ring, und einer heterocyclischen C₂-C₆₀-Gruppe, die mindestens ein Heteroatom enthält, ausgewählt aus der Gruppe bestehend aus O, N, S, Si und P,
9) L¹ jeweils unabhängig ausgewählt ist aus der Gruppe, bestehend aus einer Einfachbindung, einer C₆-C₆₀-Arylengruppe, einer Fluorenylengruppe, einer kondensierten Ringgruppe aus einem aliphatischen C₃-C₆₀-Ring und einem aromatischen C₆-C₆₀-Ring und einer heterocyclischen C₂-C₆₀-Gruppe, und
die Arylgruppe, Fluorenylgruppe, Arylengruppe, heterocyclische Gruppe, kondensierte Ringgruppe, Alkylgruppe, Alkenylgruppe, Alkoxylgruppe und Aryloxygruppe gegebenenfalls jeweils weiter mit einem oder mehreren Substituenten substituiert sein können, ausgewählt aus der Gruppe, bestehend aus Deuterium, Halogen, einer Silangruppe, die mit einer C₁-C₂₀-Alkylgruppe oder einer C₆-C₂₀-Arylgruppe substituiert oder unsubstituiert ist, einer Siloxangruppe, einer Borgruppe, einer Germaniumgruppe, einer Cyanogruppe, einer Nitrogruppe, -L'-N(R^{a})(R^{b}), einer C₁-C₂₀-Alkylthiogruppe, einer C₁-C₂₀-Alkoxylgruppe, einer C₁-C₂₀-Alkylgruppe, einer C₂-C₂₀-Alkenylgruppe, einer C₂-C₂₀-Alkinylgruppe, einer C₆-C₂₀-Arylgruppe, einer C₆-C₂₀-Arylgruppe substituiert mit Deuterium, einer Fluorenylgruppe, einer heterocyclischen C₂-C₂₀-Gruppe, einer C₃-C₂₀-Cycloalkylgruppe, einer C₇-C₂₀-Arylalkylgruppe und einer C₈-C₂₀-Arylalkenylgruppe, und diese Substituenten miteinander verbunden sein können, um einen Ring zu bilden, wobei der "Ring" ein aliphatischer C₃-C₆₀-Ring, ein aromatischer C₆-C₆₀-Ring, eine heterocyclische C₂-C₆₀-Gruppe oder ein durch deren Kombination gebildeter kondensierter Ring ist, der einen gesättigten oder ungesättigten Ring einschließt.

2. Die Verbindung nach Anspruch 1, wobei obenstehende Formel 1 durch eine der nachstehenden Formeln 2 bis 4 dargestellt wird: 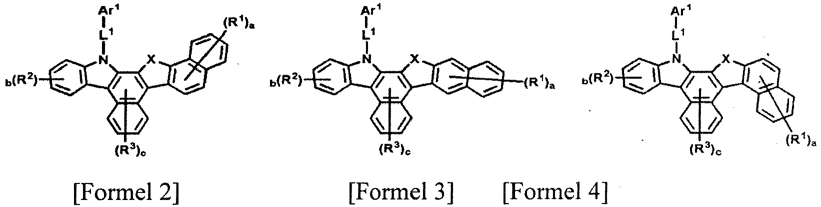 wobei in den Formeln 2 bis 4 X, L¹, Ar¹, R¹, R², R³, a, b und c die gleichen sind, wie in Anspruch 1 definiert.

3. Die Verbindung nach Anspruch 1, wobei obenstehende Formel 1 durch eine der nachstehenden Formeln 5 bis 7 dargestellt wird: 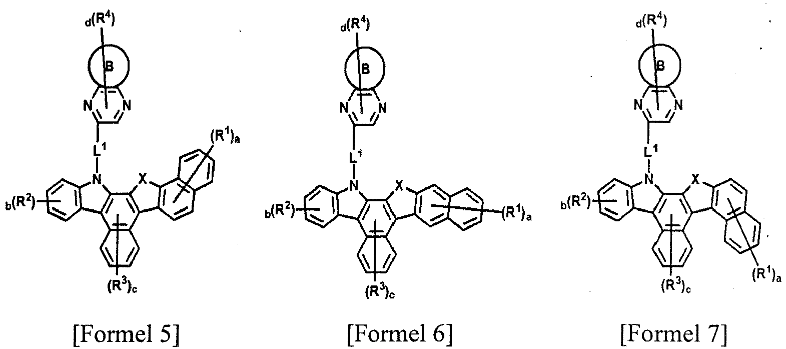 wobei in den Formeln 5 bis 7 X, L¹, Ar¹, R¹, R², R³, R⁴, a, b, c, d und Ring B die gleichen sind, wie in Anspruch 1 definiert.

4. Die Verbindung nach Anspruch 1, wobei die chemische Struktur Ar¹ der Formel 1, umfassend das Pyrazin, durch eine der nachstehenden Formeln C-1 bis C-22 dargestellt wird: 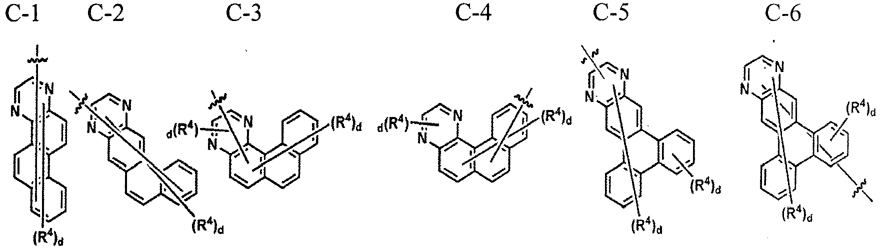 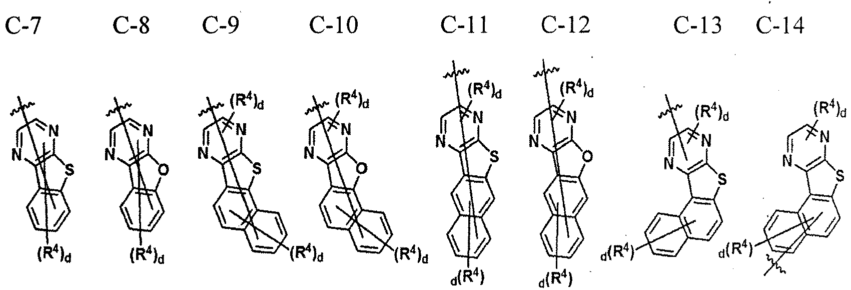 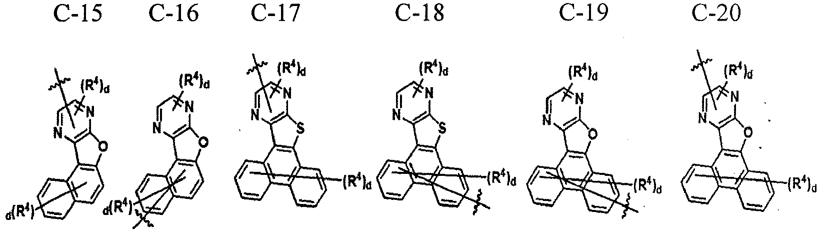 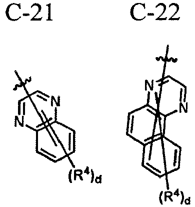 wobei in den Formeln C-1 bis C-22 R⁴ gleich ist wie in Anspruch 1 definiert, und d für eine ganze Zahl von 0 bis 11 steht.

5. Die Verbindung nach Anspruch 1, wobei Formel 1 eine der nachstehenden Verbindungen ist:

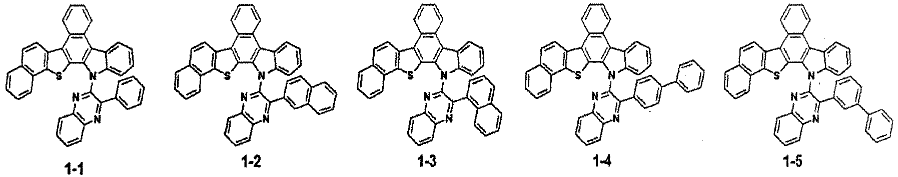

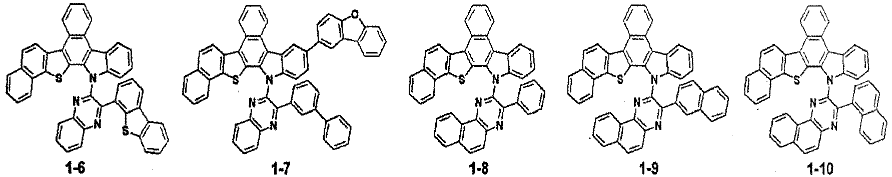

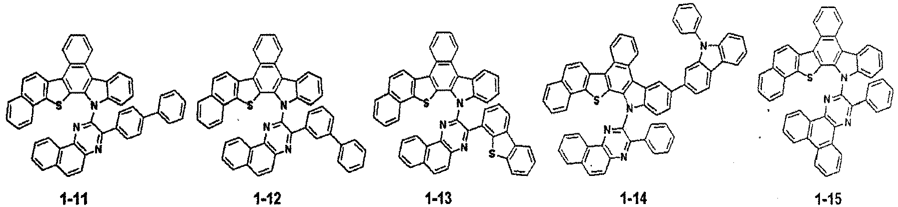

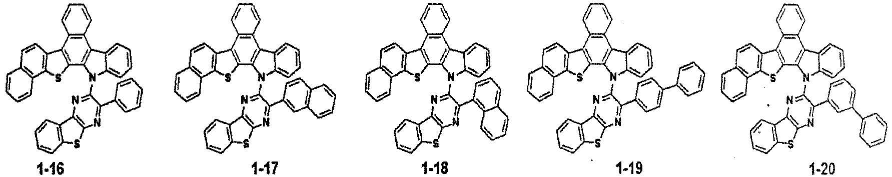

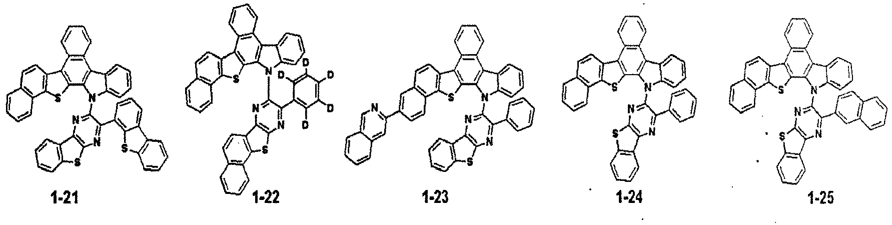

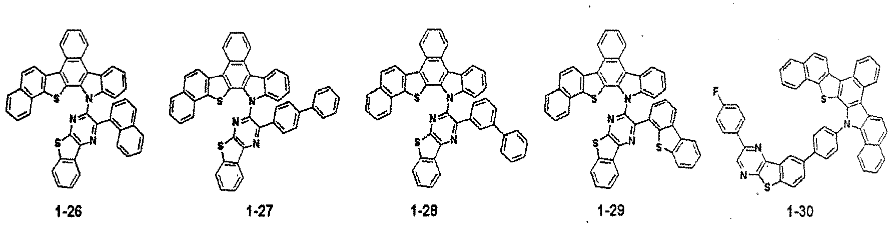

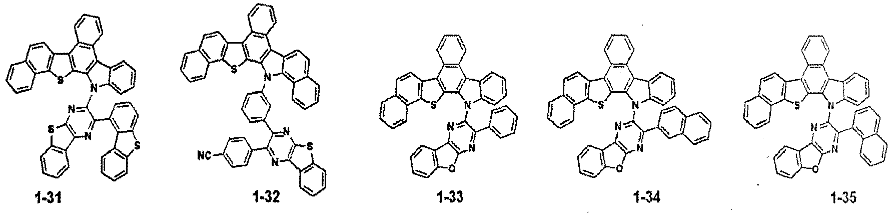

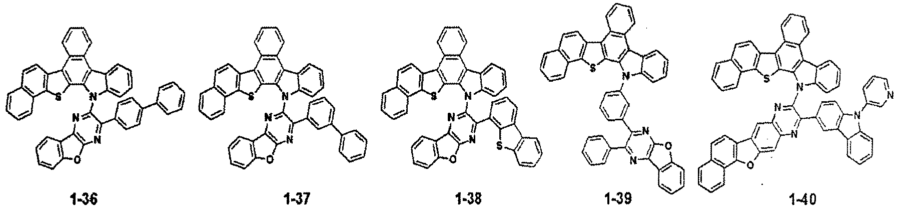

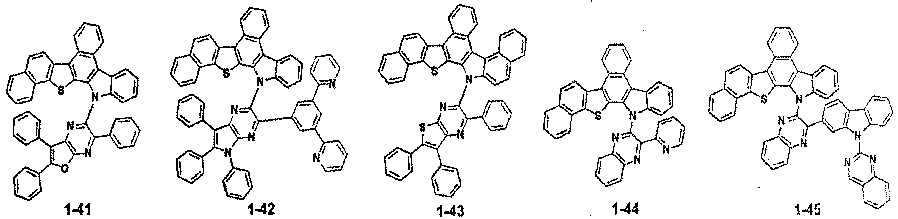

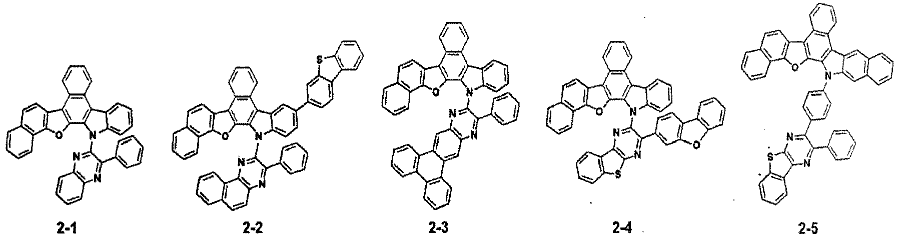

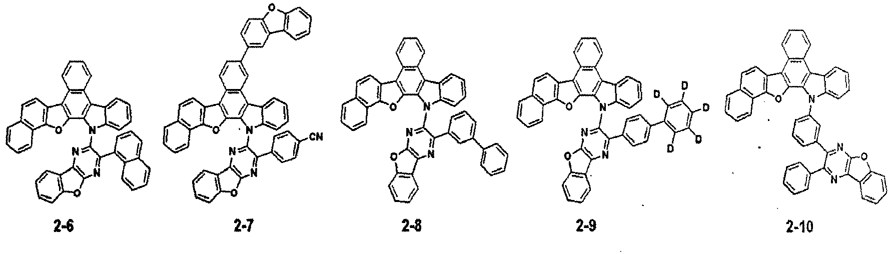

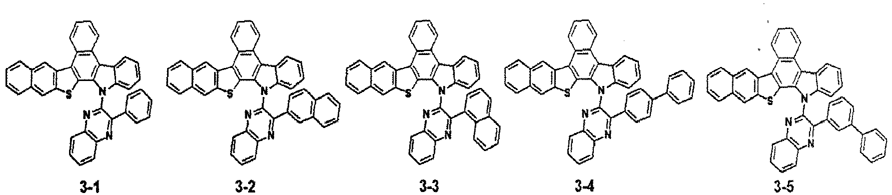

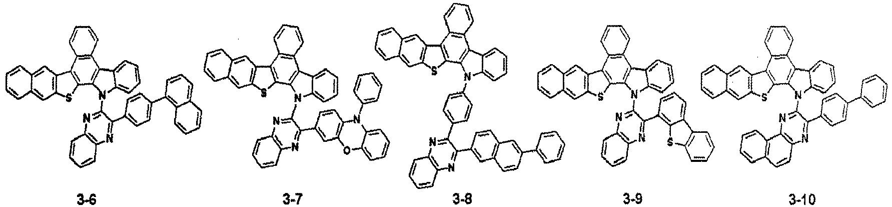

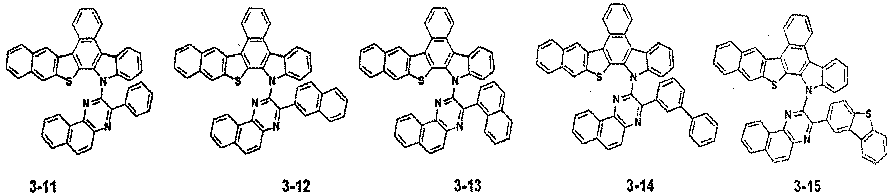

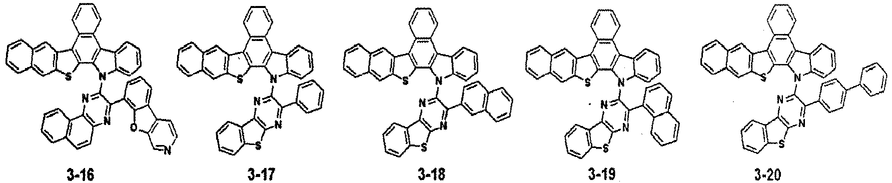

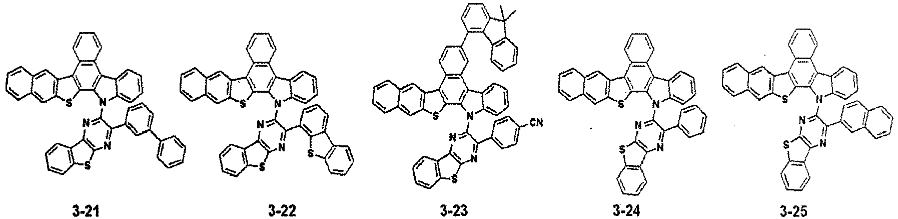

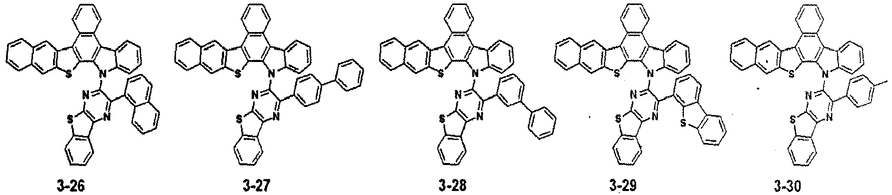

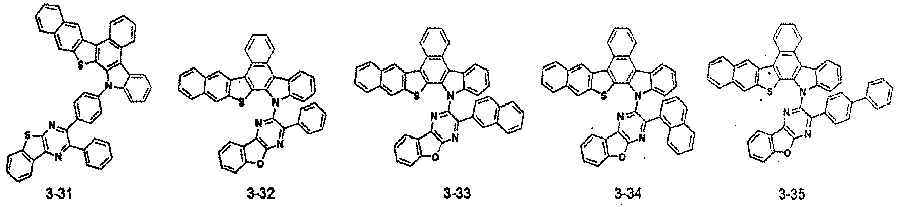

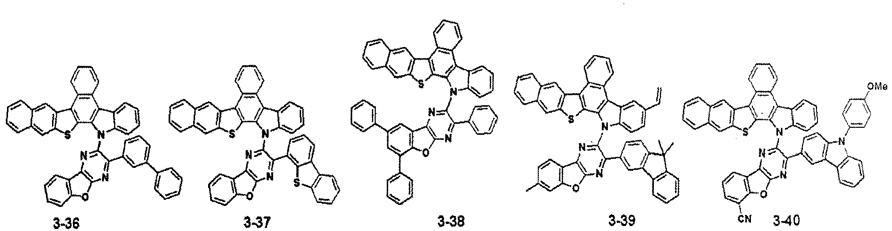

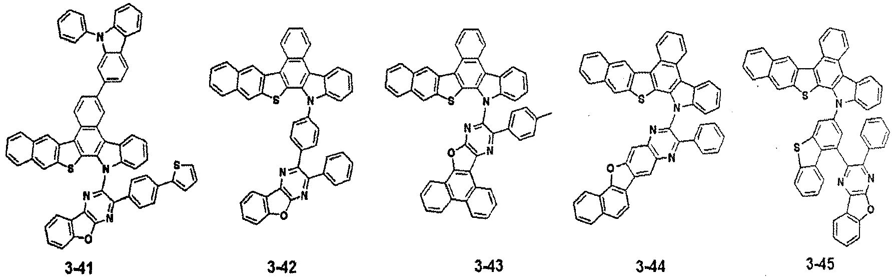

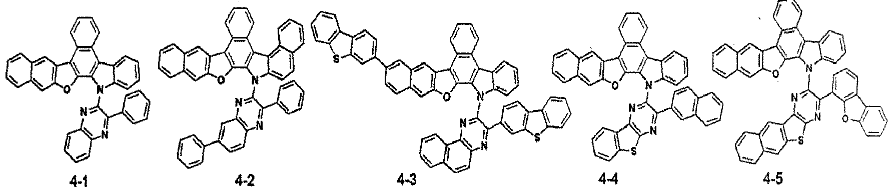

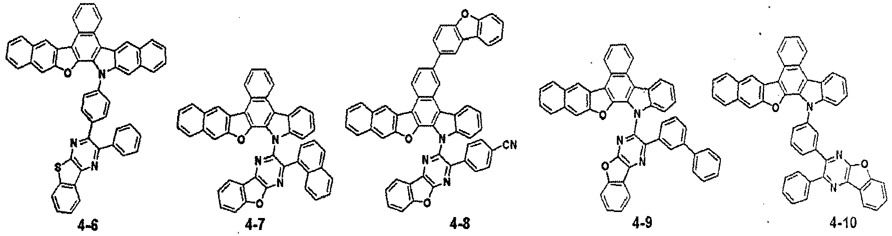

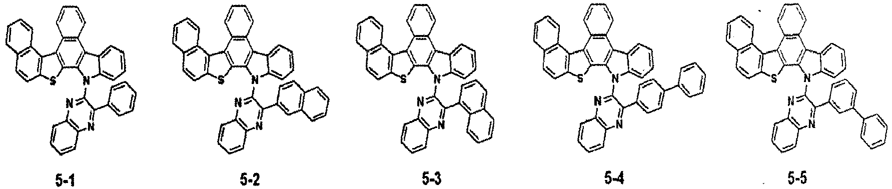

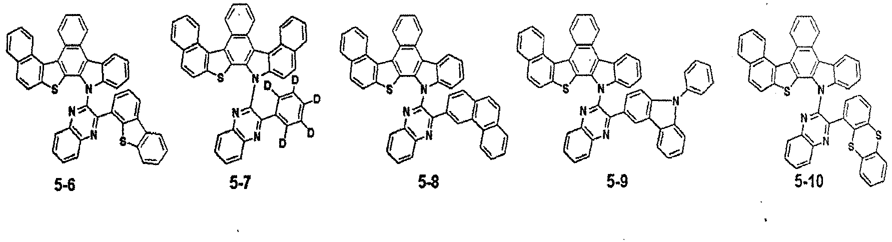

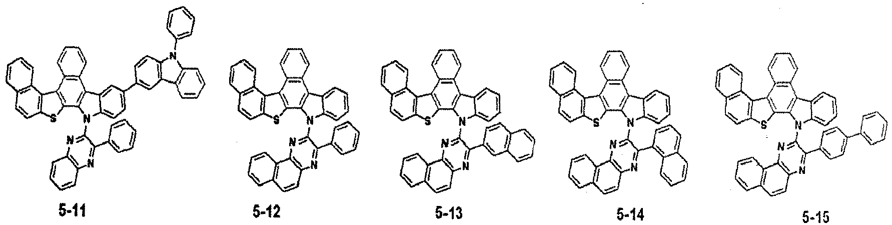

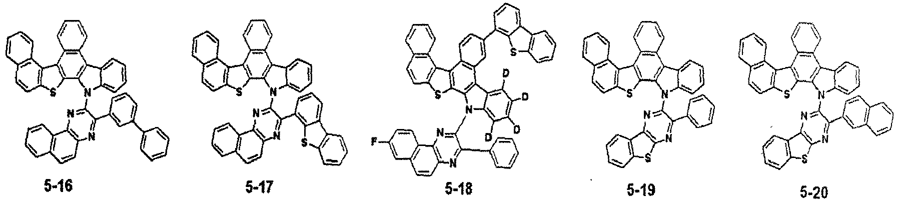

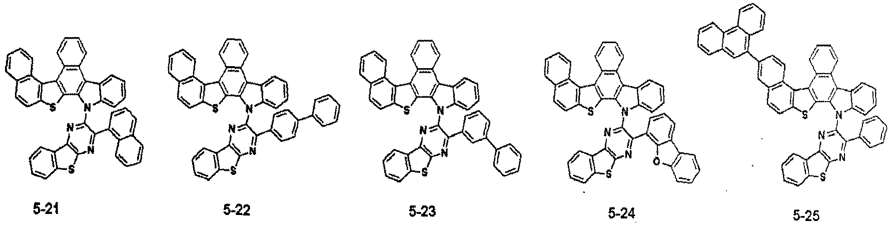

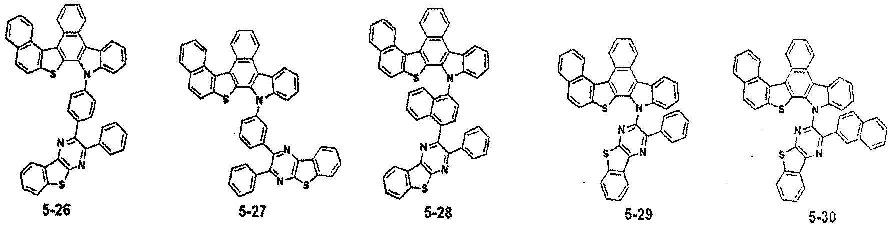

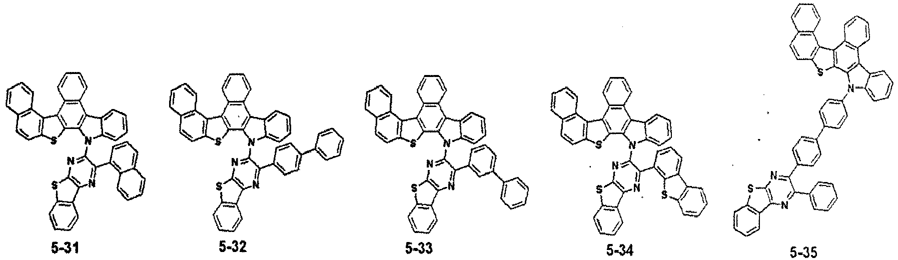

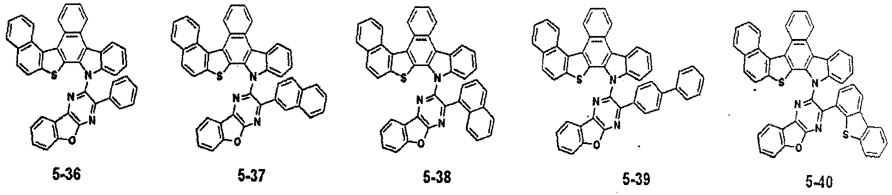

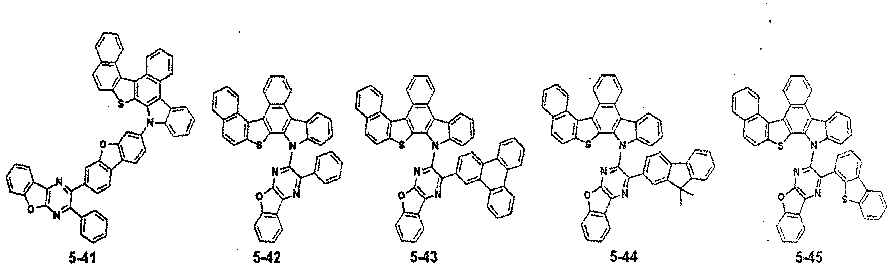

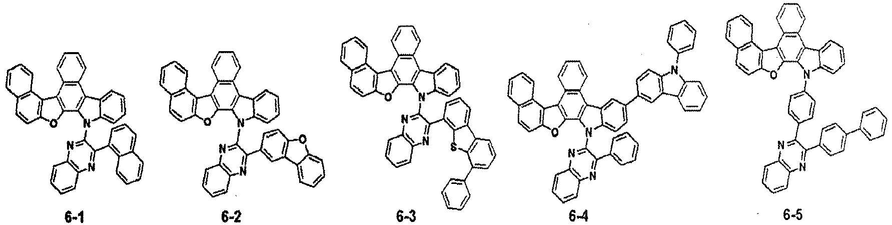

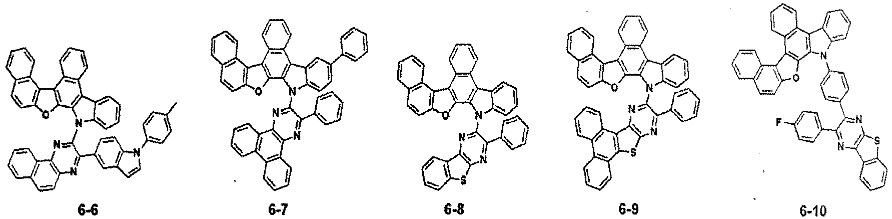

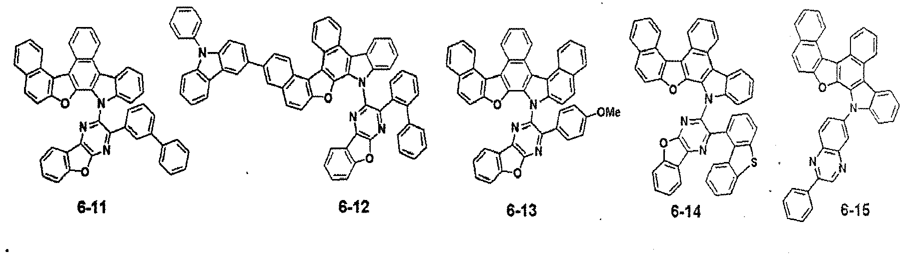

6. Ein organisches elektrisches Element, umfassend eine erste Elektrode, eine zweite Elektrode und eine zwischen der ersten Elektrode und der zweiten Elektrode gebildete Schicht aus organischem Material, wobei die Schicht aus organischem Material die Verbindung nach einem der Ansprüche 1 bis 5 umfasst.

7. Das organische elektrische Element nach Anspruch 6, wobei die Verbindung in mindestens einer Schicht aus einer Lochinjektionsschicht, einer Lochtransportschicht, einer Emissionshilfsschicht, einer Elektronentransporthilfsschicht, einer Elektronentransportschicht und einer lichtemittierenden Schicht enthalten ist, und die Verbindung als eine einzige Verbindung oder ein Gemisch aus zwei oder mehr verschiedenen Arten enthalten ist.

8. Das organische elektrische Element nach Anspruch 6, wobei die Verbindung als phosphoreszierendes Wirtsmaterial der lichtemittierenden Schicht verwendet wird.

9. Das organische elektrische Element nach Anspruch 6, wobei die Schicht aus organischem Material durch eines der Verfahren der Rotationsbeschichtung, des Düsendruckens, Tintenstrahldruckens, der Schlitzbeschichtung, Tauchbeschichtung oder Walze-auf-Walze gebildet wird.

10. Eine elektronische Vorrichtung, umfassend eine Anzeigevorrichtung und eine Steuereinheit zur Ansteuerung der Anzeigevorrichtung, wobei die Anzeigevorrichtung das organische elektrische Element nach Anspruch 6 umfasst.

11. Die elektronische Vorrichtung nach Anspruch 10, wobei das organische elektrische Element ausgewählt ist aus der Gruppe, bestehend aus einer organischen lichtemittierenden Diode, einer organischen Solarzelle, einem organischen Fotoleiter, einem organischen Transistor und einem Element zur monochromatischen oder weißen Beleuchtung ist.

## Revendications

1. Composé de formule 1 ci-dessous : 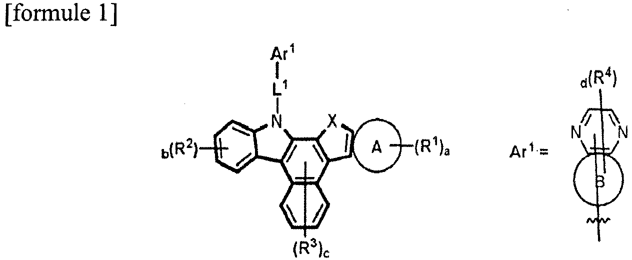 dans laquelle
1) le cycle A est un groupe aryle en C₁₀,
2) le cycle B est choisi dans le groupe constitué par les formules B-1 à B-16 suivantes : 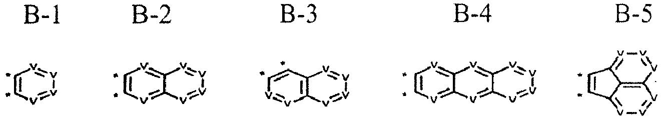 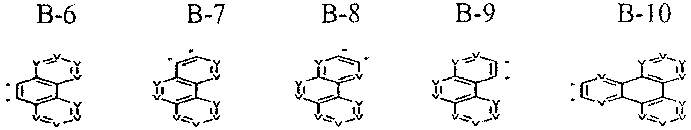 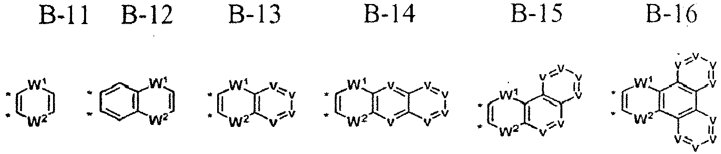 dans les formules B-1 à B-16, "*" indique la position devant être condensée avec la pyrazine comprenant deux N,
3) chacun de W¹ et W² est indépendamment une liaison simple, S ou O,
4) V est N ou C,
5) X est O ou S,
6) a est un entier de 0 à 6, chacun de b et c est un entier de 0 à 4, d est un entier de 0 à 11,
7) chacun de R¹, R² et R³, qui sont identiques ou différents, est indépendamment choisi dans le groupe constitué par l'hydrogène, le deutérium, un halogène, un groupe cyano, un groupe nitro, un groupe aryle en C₆ à C₆₀, un groupe fluorényle, un groupe hétérocyclique en C₂ à C₆₀ contenant au moins un hétéroatome choisi dans le groupe constitué par O, N, S, Si et P, un groupe cyclique condensé d'un cycle aliphatique en C₃ à C₆₀ et d'un cycle aromatique en C₆ à C₆₀, un groupe alkyle en C₁ à C₅₀, un groupe alcényle en C₂ à C₂₀, un groupe alcynyle en C₂ à C₂₀, un groupe alcoxy en C₁ à C₃₀, un groupe aryloxy en C₆ à C₃₀ et -L'-N(R^{a})(R^{b}),
ou bien, quand a, b et c valent 2 ou plus, R¹, R² et R³ sont chacun plusieurs et sont identiques ou différents, et plusieurs R¹, plusieurs R², ou plusieurs R³ peuvent être liés entre eux pour former un cycle, et
R⁴ est représenté par l'une quelconque des formules R-1 à R-10 suivantes : 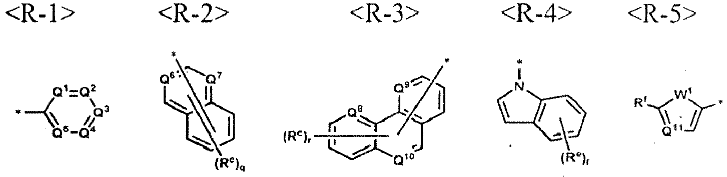 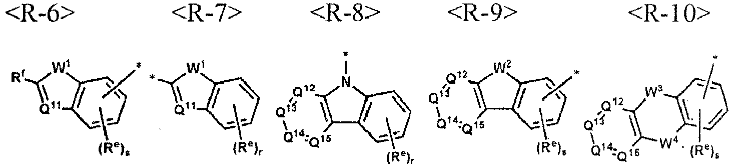 dans les formules R-1 à R-10,
1) chacun de Q¹ à Q¹⁵ est indépendamment CR^{g} ou N,
2) W¹ est S, O ou NR^{h},
3) chacun de W² à W⁴ est indépendamment S, O, NR^{h} ou CRⁱR^{j},
4) R^{e} est choisi dans le groupe constitué par l'hydrogène, le deutérium, un halogène, un groupe silane non substitué ou substitué par un groupe alkyle en C₁ à C₂₀ ou un groupe aryle en C₆ à C₂₀, un groupe siloxane, un groupe bore, un groupe germanium, un groupe cyano, un groupe nitro, un groupe alkylthio en C₁ à C₂₀, un groupe alcoxy en C₁ à C₂₀, un groupe alkyle en C₁ à C₂₀, un groupe alcényle en C₂ à C₂₀, un groupe alcynyle en C₂ à C₂₀, un groupe aryle en C₆ à C₂₀, un groupe aryle en C₆ à C₂₀ substitué par le deutérium, un groupe fluorényle, un groupe hétérocyclique en C₂ à C₂₀ contenant au moins un hétéroatome choisi dans le groupe constitué par O, N, S, Si et P, un groupe cycloalkyle en C₃ à C₂₀, un groupe arylalkyle en C₇ à C₂₀, et un groupe arylalcényle en C₈ à C₂₀ et, quand ces substituants sont adjacents, ils peuvent être liés entre eux pour former un cycle,
5) chacun de R^{f} et R^{g} est indépendamment choisi dans le groupe constitué par l'hydrogène, le deutérium, un groupe aryle en C₆ à C₂₀, un groupe fluorényle, un groupe cyclique condensé d'un cycle aliphatique en C₃ à C₂₀ et d'un cycle aromatique en C₆ à C₂₀, un groupe hétérocyclique en C₂ à C₂₀ contenant au moins un hétéroatome choisi parmi O, N, S, Si et P, un groupe alkyle en C₁ à C₂₀, un groupe alcényle en C₂ à C₂₀, un groupe alcynyle en C₂ à C₂₀, et un groupe alcoxy en C₁ à C₃₀,
6) chacun de R^{h}, Rⁱ et R^{j} est indépendamment choisi dans le groupe constitué par un groupe aryle en C₆ à C₂₀, un groupe hétérocyclique en C₂ à C₂₀ contenant au moins un hétéroatome choisi parmi O, N, S, Si et P, un groupe alkyle en C₁ à C₂₀, un groupe alcényle en C₂ à C₂₀, un groupe alcoxy en C₁ à C₂₀ et un groupe fluorényle, Rⁱ et R^{j} peuvent être liés l'un à l'autre pour former un composé spiro conjointement avec le C auquel ils sont liés,
7) chaque q est indépendamment un entier de 0 à 5,
8) chaque r est indépendamment un entier de 0 à 4,
9) chaque s est indépendamment un entier de 0 à 3,
quand chacun de q, r et s vaut 2 ou plus, chaque R^{e} est identique ou différent, et * indique la position devant être liée,
8) L' est choisi dans le groupe constitué par une liaison simple, un groupe arylène en C₆ à C₆₀, un groupe fluorénylène, un groupe cyclique condensé d'un cycle aliphatique en C₃ à C₆₀ et d'un cycle aromatique en C₆ à C₆₀, et un groupe hétérocyclique en C₂ à C₆₀, et chacun de R^{a} et R^{b} est indépendamment choisi dans le groupe constitué par un groupe aryle en C₆ à C₆₀, un groupe fluorényle, un groupe cyclique condensé d'un cycle aliphatique en C₃ à C₆₀ et d'un cycle aromatique en C₆ à C₆₀, et un groupe hétérocyclique en C₂ à C₆₀ contenant au moins un hétéroatome choisi dans le groupe constitué par O, N, S, Si et P,
9) chaque L¹ est indépendamment choisi dans le groupe constitué par une liaison simple, un groupe arylène en C₆ à C₆₀, un groupe fluorénylène, un groupe cyclique condensé d'un cycle aliphatique en C₃ à C₆₀ et d'un cycle aromatique en C₆ à C₆₀, et un groupe hétérocyclique en C₂ à C₆₀, et
chacun parmi le groupe aryle, le groupe fluorényle, le groupe arylène, le groupe hétérocyclique, le groupe cyclique condensé, le groupe alkyle, le groupe alcényle, le groupe alcoxy et le groupe aryloxy peut être éventuellement en outre substitué par un ou plusieurs substituants choisis dans le groupe constitué par le deutérium, un halogène, un groupe silane non substitué ou substitué par un groupe alkyle en C₁ à C₂₀ ou un groupe aryle en C₆ à C₂₀, un groupe siloxane, un groupe bore, un groupe germanium, un groupe cyano, un groupe nitro, -L'-N(R^{a})(R^{b}), un groupe alkylthio en C₁ à C₂₀, un groupe alcoxy en C₁ à C₂₀, un groupe alkyle en C₁ à C₂₀, un groupe alcényle en C₂ à C₂₀, un groupe alcynyle en C₂ à C₂₀, un groupe aryle en C₆ à C₂₀, un groupe aryle en C₆ à C₂₀ substitué par le deutérium, un groupe fluorényle, un groupe hétérocyclique en C₂ à C₂₀, un groupe cycloalkyle en C₃ à C₂₀, un groupe arylalkyle en C₇ à C₂₀, et un groupe arylalcényle en C₈ à C₂₀, et ces substituants peuvent être liés entre eux pour former un cycle, où le "cycle" est un cycle aliphatique en C₃ à C₆₀, un cycle aromatique en C₆ à C₆₀, un hétérocycle en C₂ à C₆₀ ou un cycle condensé formé par la combinaison de ceux-ci, qui comprend un cycle saturé ou insaturé.

2. Composé selon la revendication 1, dans lequel la formule 1 ci-dessus est représentée par l'une quelconque des formules 2 à 4 ci-dessous : 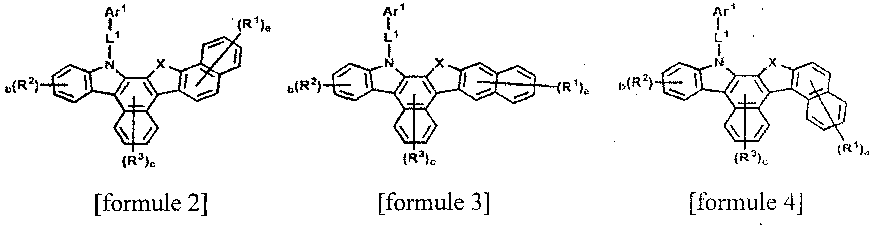 dans les formules 2 à 4, X, L¹, Ar¹, R¹, R², R³, a, b et c sont tels que définis dans la revendication 1.

3. Composé selon la revendication 1, dans lequel la formule 1 ci-dessus est représentée par l'une quelconque des formules 5 à 7 ci-dessous : 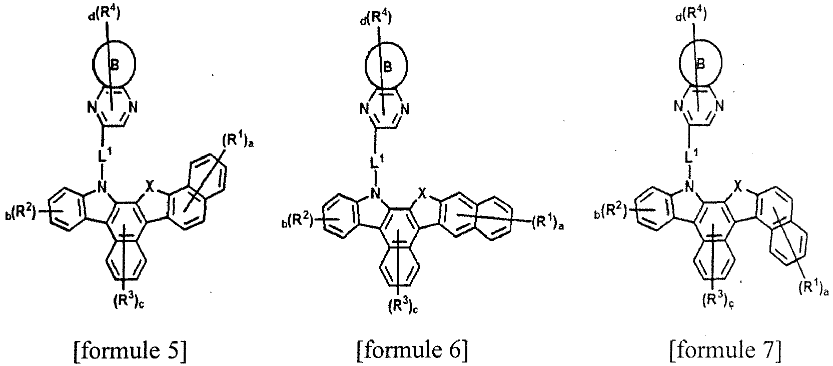 dans les formules 5 à 7, X, L¹, Ar¹, R¹, R², R³, R⁴, a, b, c, d et le cycle B sont tels que définis dans la revendication 1.

4. Composé selon la revendication 1, dans lequel la structure chimique Ar¹ de la formule 1 comprenant la pyrazine est représentée par l'une quelconque des formules C-1 à C-22 suivantes : 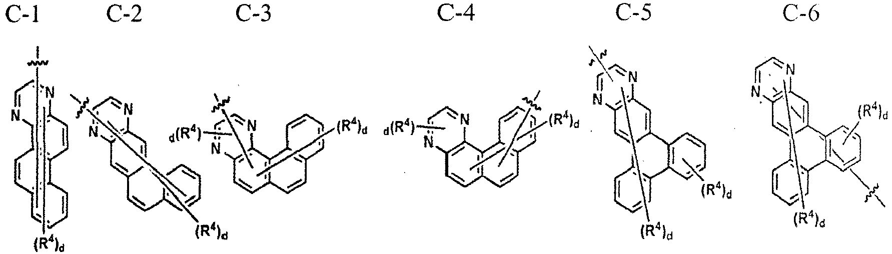 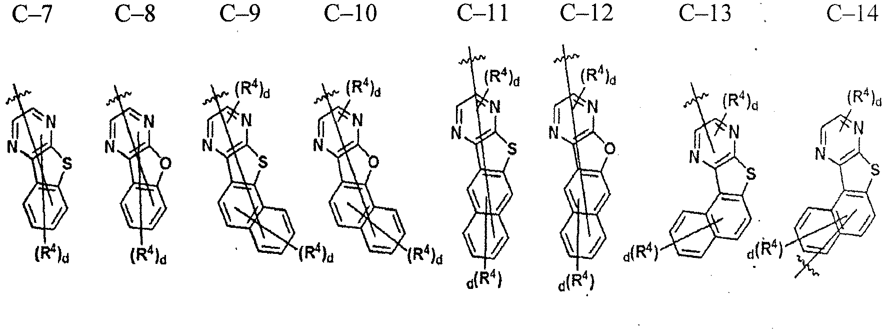 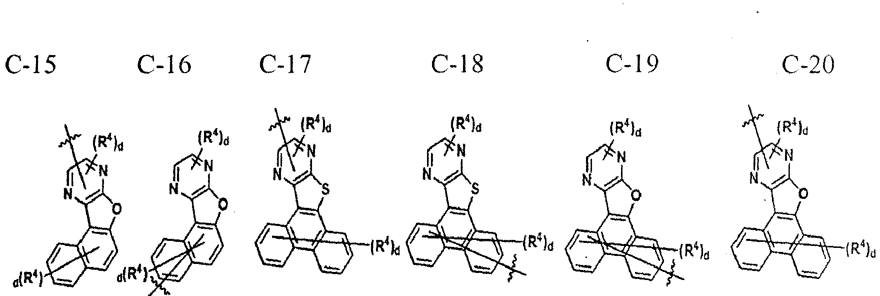 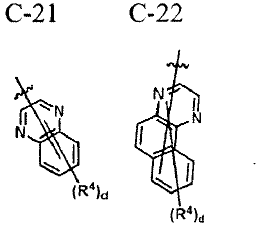 dans les formules C-1 à C-22, R⁴ est tel que défini dans la revendication 1, et d est un entier de 0 à 11.

5. Composé selon la revendication 1, dans lequel la formule 1 est l'un quelconque des composés ci-dessous :

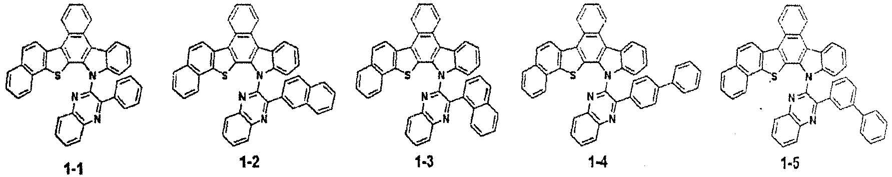

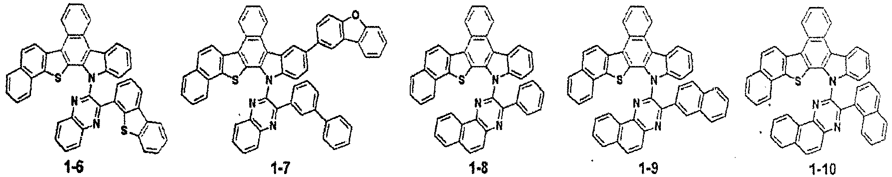

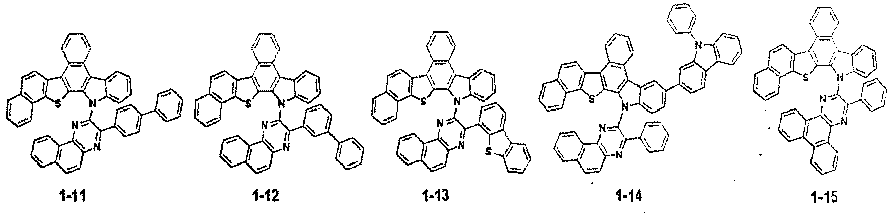

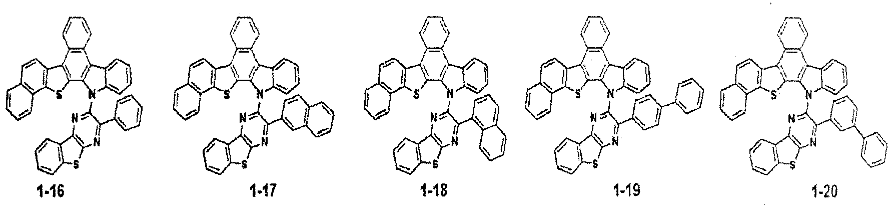

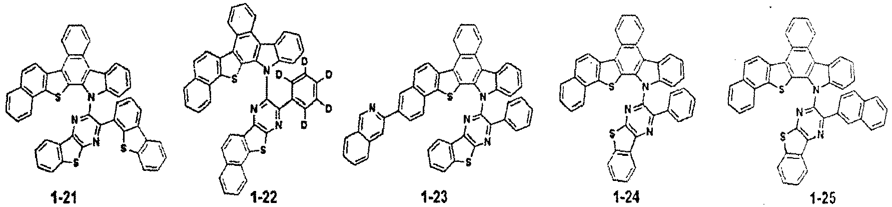

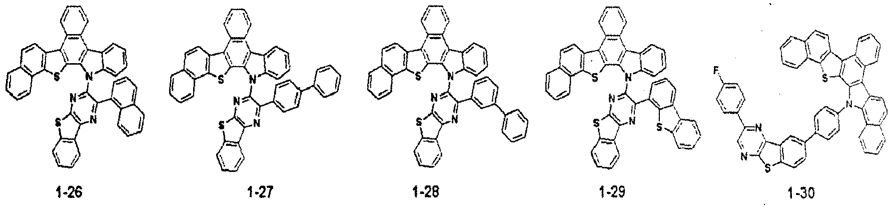

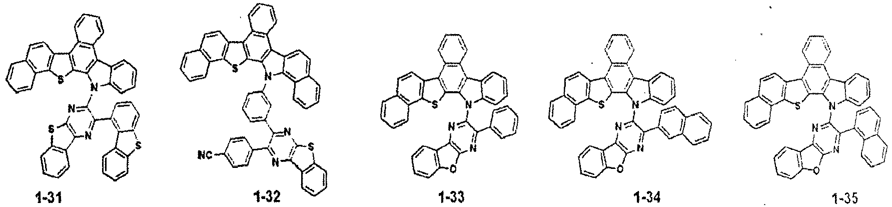

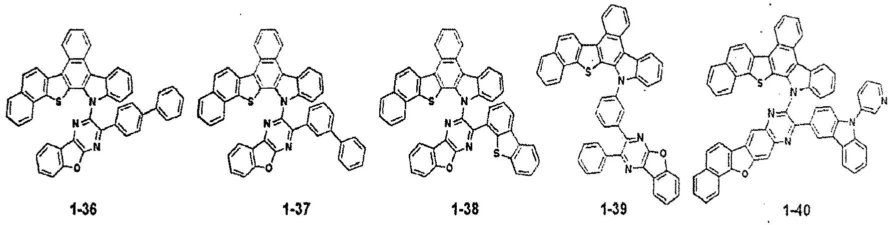

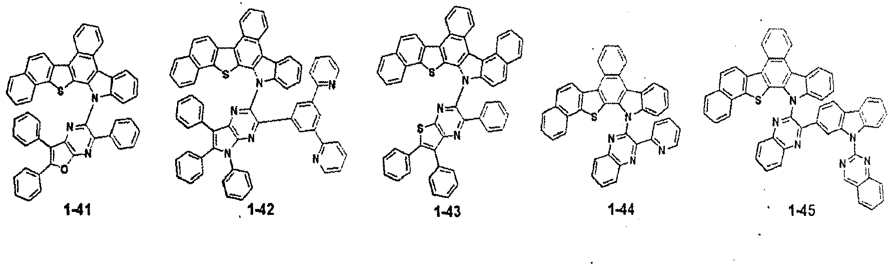

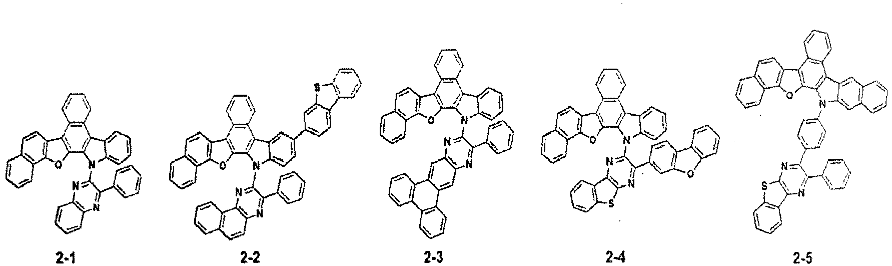

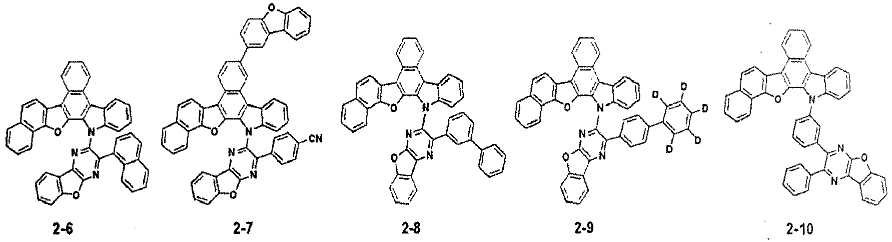

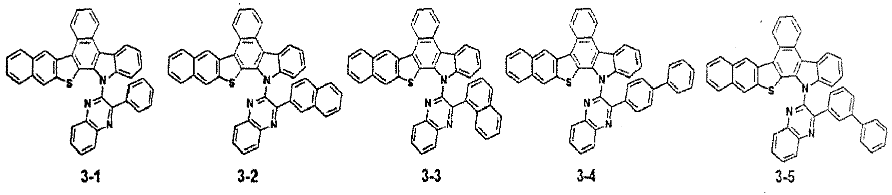

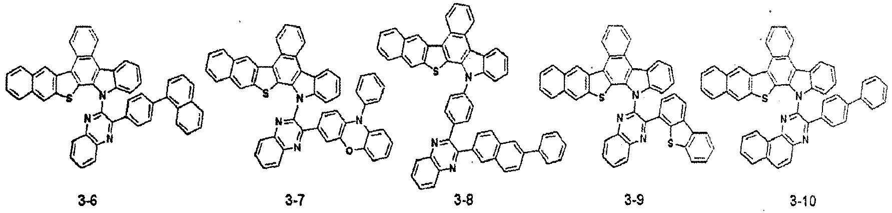

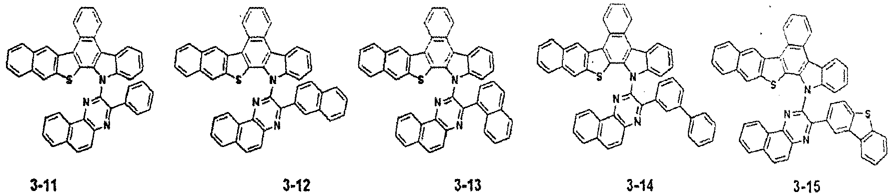

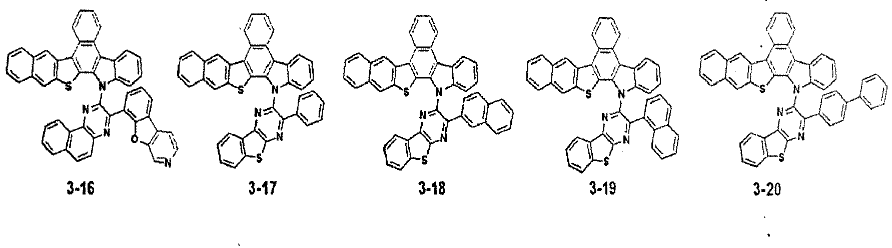

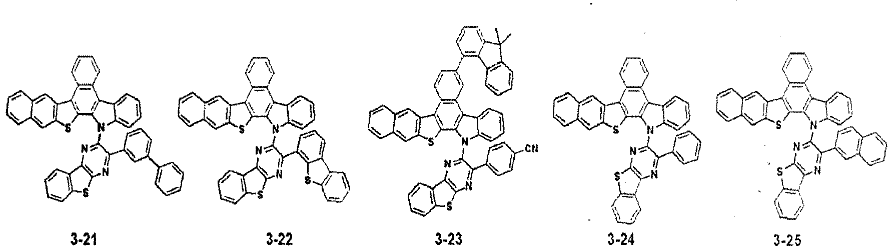

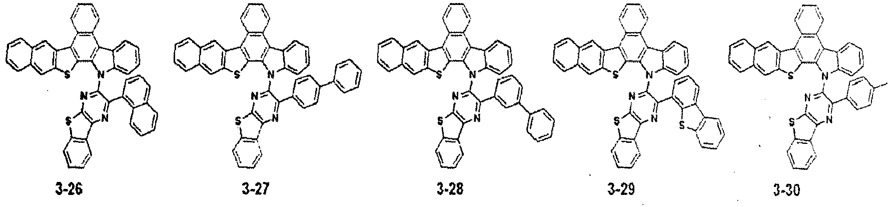

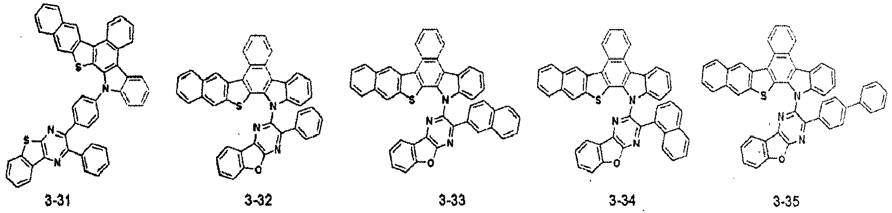

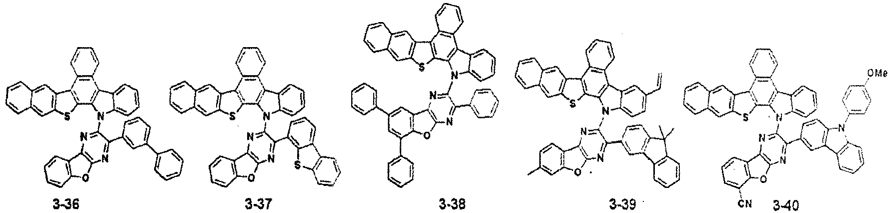

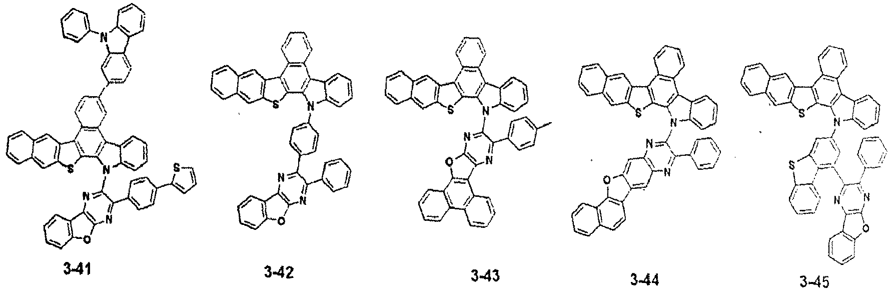

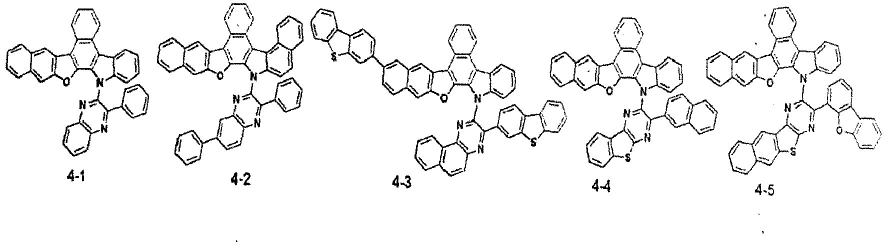

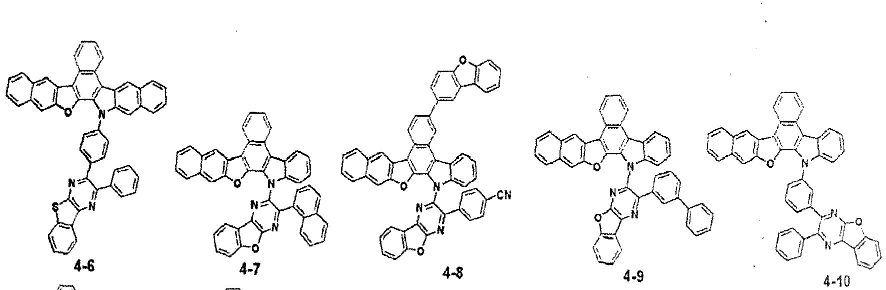

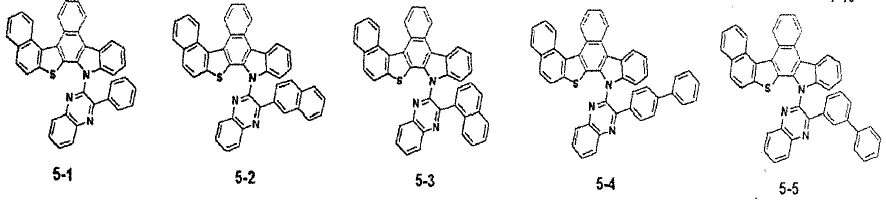

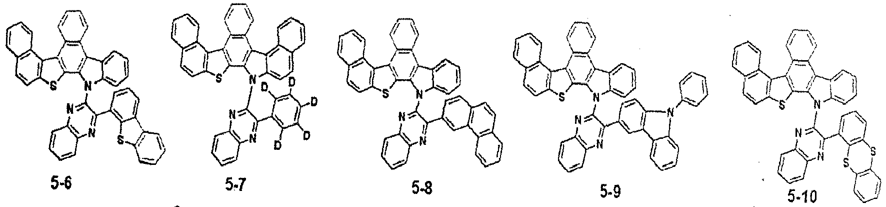

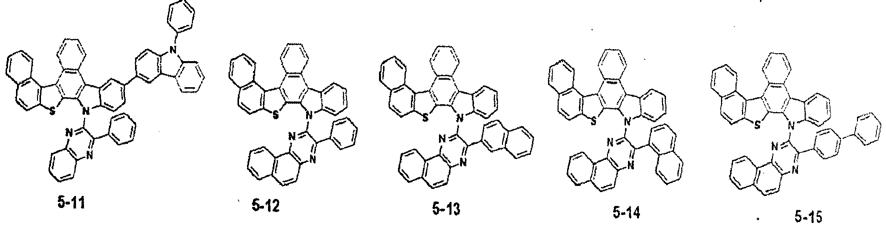

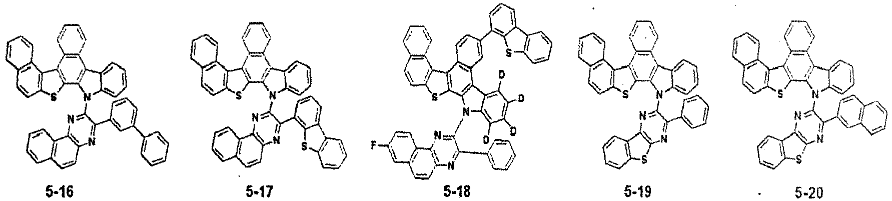

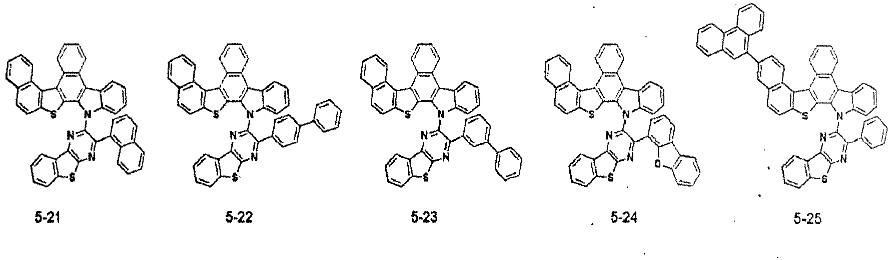

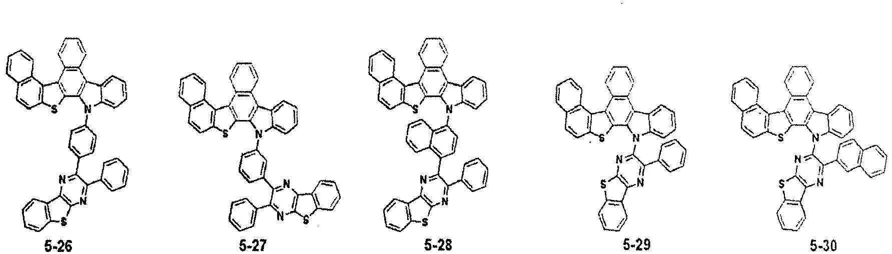

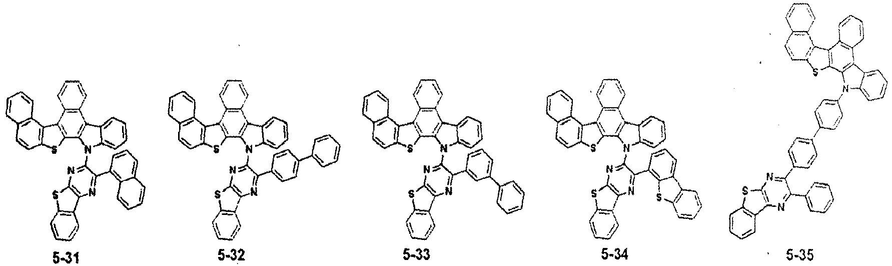

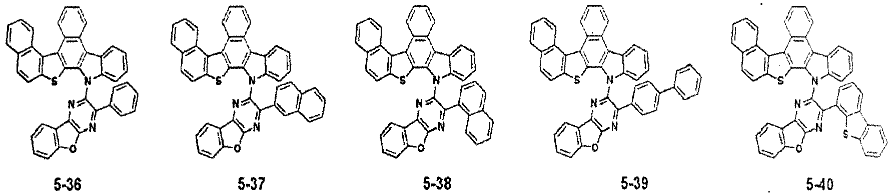

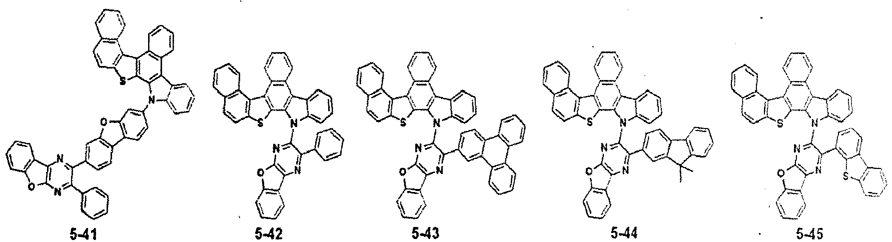

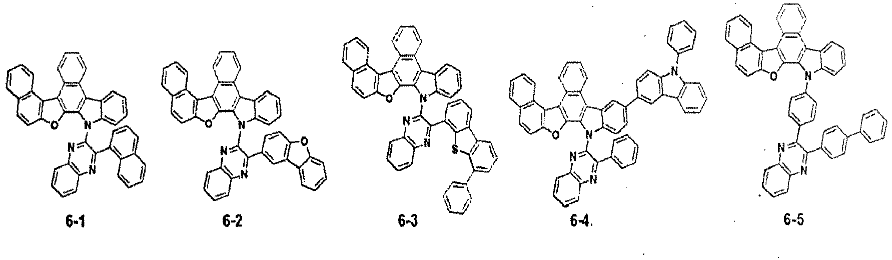

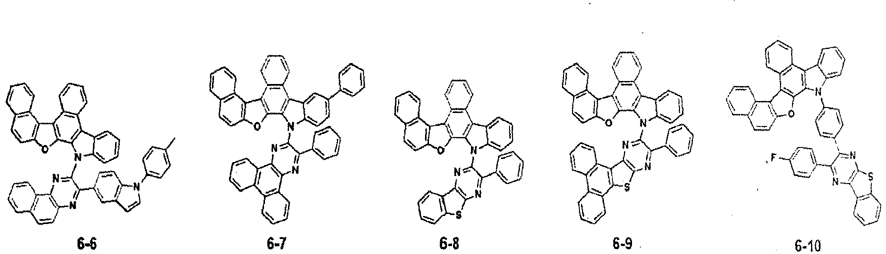

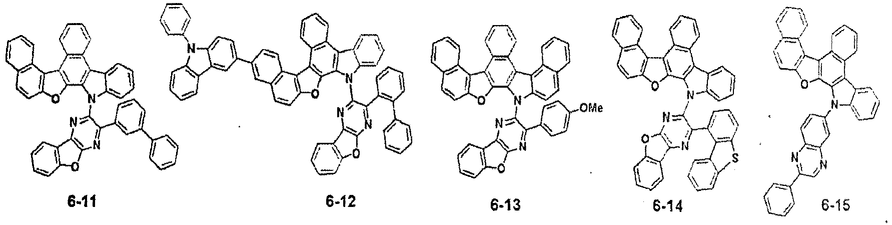

6. Elément électrique organique comprenant une première électrode, une deuxième électrode, et une couche de matériau organique formée entre la première électrode et la deuxième électrode, dans lequel la couche de matériau organique comprend le composé de l'une quelconque des revendications 1 à 5.

7. Elément électrique organique selon la revendication 6, dans lequel le composé est compris dans au moins une couche parmi une couche d'injection de trous, une couche de transport de trous, une couche auxiliaire d'émission, une couche auxiliaire de transport d'électrons, une couche de transport d'électrons et une couche luminescente, et le composé est compris sous la forme d'un seul composé ou d'un mélange de deux types différents ou plus.

8. Elément électrique organique selon la revendication 6, dans lequel le composé est utilisé en tant que matériau hôte phosphorescent de la couche luminescente.

9. Elément électrique organique selon la revendication 6, dans lequel la couche de matériau organique est formée par l'un quelconque des procédés d'enduction centrifuge, impression par buse, impression par jet d'encre, enduction fente, enduction par trempage, ou rouleau à rouleau.

10. Dispositif électronique comprenant un dispositif d'affichage et une unité de commande pour piloter le dispositif d'affichage, dans lequel le dispositif d'affichage comprend l'élément électrique organique de la revendication 6.

11. Dispositif électronique selon la revendication 10, dans lequel l'élément électrique organique est choisi dans le groupe constitué par une diode luminescente organique, une cellule solaire organique, un photoconducteur organique, un transistor organique, et un élément pour éclairage monochromatique ou blanc.
